(19) Europäisches Patentamt
European Patent Office
Office européen des brevets

(11) EP 4 786 593 A1

# (12) EUROPEAN PATENT APPLICATION
published in accordance with Art. 153(4) EPC

(43) Date of publication:
**05.08.2026 Bulletin 2026/32**

(21) Application number: **24870480.1**

(22) Date of filing: **13.09.2024**

(51) International Patent Classification (IPC):
***C12N 15/113*** *(2010.01)* ***A61K 31/713*** *(2006.01)*
***A61P 1/16*** *(2006.01)* ***C07H 21/02*** *(2006.01)*
***A61K 47/54*** *(2017.01)*

(52) Cooperative Patent Classification (CPC):
**A61K 31/713; A61K 47/54; A61P 1/16; C07H 21/02; C12N 15/113**

(86) International application number:
**PCT/CN2024/118774**

(87) International publication number:
**WO 2025/066936 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 CN 202311265471**

(71) Applicant: **Rigerna Therapeutics (Beijing) Co., Ltd.**
**Beijing 102629 (CN)**

(72) Inventors:
- **HUANG, Yuanyu**
  **Beijing 102629 (CN)**
- **LI, Haitao**
  **Beijing 102629 (CN)**
- **HAN, Xiaofeng**
  **Beijing 102629 (CN)**
- **WANG, Rihua**
  **Beijing 102629 (CN)**
- **GAO, Yongxin**
  **Beijing 102629 (CN)**

(74) Representative: **De Clercq & Partners**
**Edgard Gevaertdreef 10a**
**9830 Sint-Martens-Latem (BE)**

Remarks:
The complete document including Reference Table(s) and the Sequence Listing(s) can be downloaded from the EPO website

# (54) NUCLEIC ACID CHIMERA CONTAINING MULTIPLE DOUBLE-STRANDED OLIGONUCLEOTIDE MOLECULES, COMPOSITION, AND USE

(57) The present invention relates to the field of nucleic acid drugs, and provides a nucleic acid chimera containing multiple double-stranded oligonucleotide molecules. The nucleic acid chimera has a chimera structure composed of at least two oligonucleotide molecules, which can simultaneously regulate the expression of the same or different genes through interaction with different target sites.

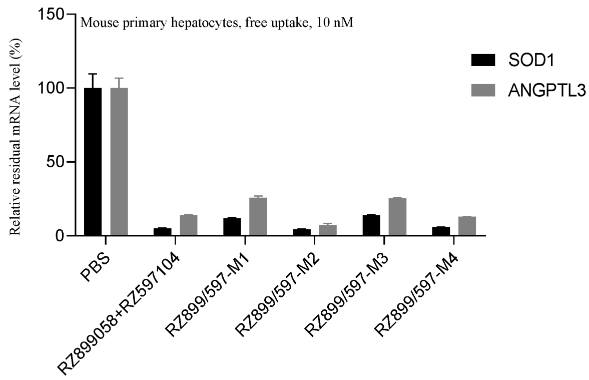


Fig. 1



Processed by Luminess, 75001 PARIS (FR)

## Description

### CROSS-REFERENCE TO RELATED APPLICATIONS

The present application claims priority to Chinese Patent Application No. 202311265471.0, filed with the China National Intellectual Property Administration on September 27, 2023, entitled "NUCLEIC ACID CHIMERA CONTAINING MULTIPLE DOUBLE-STRANDED OLIGONUCLEOTIDE MOLECULES, COMPOSITION, AND USE THEREOF", the entire content of which is incorporated in this application by reference.

### TECHNICAL FIELD

The present invention belongs to the field of nucleic acid pharmaceuticals, and specifically relates to a nucleic acid chimera containing multiple double-stranded oligonucleotide molecules, a composition, and use thereof.

### BACKGROUND

Double-stranded oligonucleotide molecules can regulate gene expression through RNA interference and targeting RNA of a gene. In current techniques, a single type of a double-stranded oligonucleotide molecule is generally adopted to regulate gene expression. ,A chimeric molecule containing multiple double-stranded oligonucleotides may be used for regulation of gene expression through different mechanisms. For example, two double-stranded oligonucleotide molecules may regulate the expression of one or more genes by targeting different genes or different positions of the mRNA of the same target gene, respectively.

Therefore, it is necessary to provide a novel nucleic acid chimera, comprising a chimeric structure composed of two or more oligonucleotide molecules, which can regulate the expression of different genes through interactions with different targets.

### SUMMARY

The present disclosure provides a nucleic acid chimera comprising at least two double-stranded oligonucleotide molecules, a composition, and use thereof.

In a first aspect, the present disclosure provides a nucleic acid chimera, comprising a nucleotide unit I, where the nucleotide unit I has a structure represented by formula (101):

$$H-\left(X_1\right)_{j_1}-Y_1-\left[\left(X_2\right)_{j_2}-Y_2\right]_k-\left(X_3\right)_{j_3}-H$$

(101)

wherein each of $X_1$, $X_2$, and $X_3$ is independently selected from

$$*-\left(M_1\right)_m-\left(M_2\right)_n-*\ ,$$

and each $M_1$ independently comprises a ligand capable of binding to a cellular receptor; each $M_2$ is independently selected from a modified or unmodified nucleotide; and each m is independently selected from 1, 2, or 3, and each n is independently selected from an integer of 0-3;

wherein $X_1$, $X_2$, and $X_3$ may be the same or different; and

wherein each $Y_1$ and $Y_2$ independently represents a sense strand of a double-stranded oligonucleotide, and each of the sense strands may correspond to the same target gene or different target genes; and the corresponding relationship means that the sense strand is identical or substantially identical to a segment of consecutive nucleotides in the mRNA of the target gene; and

$j_1$, $j_2$, and $j_3$ are independently selected from an integer of 0-3, and $j_2 \neq 0$.

In some embodiments, the nucleic acid chimera further comprises a nucleotide unit **II,** wherein the nucleotide unit II comprises an antisense strand complementary or substantially complementary to the sense strand in the double-stranded oligonucleotide, the number of the antisense strands is equal to the number of the sense strands, and each of the antisense strands is at least partially complementary to a corresponding sense strand thereof to form a duplex region;

where the antisense strand is complementary or at least partially complementary to the mRNA of at least one target gene.

In other embodiments of the present disclosure, the present disclosure provides a nucleic acid chimera, comprising a nucleotide unit III, where the nucleotide unit III has a structure represented by formula (301) or an isomer thereof, or a pharmaceutically acceptable salt thereof:

$$HO - (M_1)_{j_4} - Y_3 - (M_1)_{j_5} - Dt - (M_1)_{j_6} - Y_4 - (M_1)_{j_7} - OH$$

(301)

where each $M_1$ independently comprises a ligand capable of binding to a cellular receptor;
wherein, $Y_3$ is selected from a sense strand and $Y_4$ is selected from a sense strand, or $Y_3$ is selected from an antisense strand and $Y_4$ is selected from a sense strand; and
$j_4$, $j_5$, $j_6$, and $j_7$ are each independently selected from integers of 0-3, and at least one of $j_6$ and $j_7$ is not 0; and when $Y_3$ is selected from the sense strand, at least one of $j_4$ and $j_5$ is not 0.

In a second aspect, the present disclosure provides a composition, comprising the nucleic acid chimera described in the present disclosure and a physiologically acceptable excipient.

In a third aspect, the present disclosure provides the nucleic acid chimera for use in treating a disease or condition caused by dysregulation of expression of at least one gene.

In a fourth aspect, the present disclosure provides use of the nucleic acid chimera in preparation of a medicament for treating a disease or condition caused by expression dysregulation of at least one gene.

In a fifth aspect, the present disclosure provides a method for treating a disease or condition, comprising administering the nucleic acid chimera described in the present disclosure to an individual in need thereof.

In some embodiments, the nucleic acid chimera according to the present disclosure is administered to the individual subcutaneously or intravenously.

In some embodiments, after *in vivo* administration, the nucleic acid chimera according to the present disclosure is cleaved to afford at least two independent double-stranded oligonucleotide molecules, each targeting a portion of an mRNA transcribed from one or more target genes, wherein the target genes may be the same or different.

## BRIEF DESCRIPTION OF THE DRAWINGS

Fig. 1 shows relative expression levels of target genes SOD1/ANGPTL3 in mouse primary hepatocytes after administration of the nucleic acid chimera.

Fig. 2 shows $IC_{50}$ curves of target gene SOD1 in mouse primary hepatocytes after administration of the nucleic acid chimera.

Fig. 3 shows $IC_{50}$ curves of target gene ANGPTL3 in primary mouse hepatocytes after administration of the nucleic acid chimera.

Fig. 4 shows relative expression levels of target gene SOD1 in mice after administration of the nucleic acid chimera.

Fig. 5 shows relative expression levels of target gene ANGPTL3 in mice after administration of the nucleic acid chimera.

Fig. 6 shows relative expression levels of target genes SOD1/ANGPTL3 in mouse primary hepatocytes after administration of a nucleic acid chimera.

Fig. 7 shows relative expression levels of target genes SOD1/ANGPTL3 in mouse primary hepatocytes after administration of the nucleic acid chimera.

Fig. 8 shows relative expression levels of target gene SOD1 in mice after administration of the nucleic acid chimera.

Fig. 9 shows relative expression levels of target gene ANGPTL3 in mice after administration of the nucleic acid chimera.

Fig. 10 shows relative expression levels of target gene SOD1 in mice after administration of the nucleic acid chimera.

Fig. 11 shows relative expression levels of target gene ANGPTL3 in mice after administration of the nucleic acid chimera.

## DETAILED DESCRIPTION

The technical solutions in the embodiments of the present invention will be clearly and completely described below. Obviously, the embodiments described are only a part, but not all, of the embodiments of the present invention. Based on the embodiments of the present invention, all other embodiments obtained by those of ordinary skill in the art without creative efforts shall fall within the scope of protection of the present invention.

**[Definition]**

The term "alicyclic ring" refers to a structure having a cyclic carbon skeleton, for example, monocyclic rings, spiro rings, bridged rings, and the like. The term "4- to 8-membered alicyclic ring" refers to a cyclic carbon skeleton structure having 4 to 8 carbon atoms.

The term "saturated alicyclic ring" refers to a cyclic carbon skeleton structure in which all bonds are carbon-carbon single bonds, with no carbon-carbon double bonds or carbon-carbon triple bonds.

The term "alkyl" has a general structural formula of

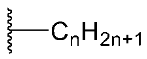
,

and the alkyl may be linear or branched alkyl. The term "$C_1$-$C_6$ alkyl" refers to alkyl having 1 to 6 carbon atoms.

The term "alkylene" has a general structural formula of

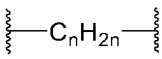
,

and the alkylene may be linear or branched alkylene.

The term "alkyloxy" has a general structural formula of

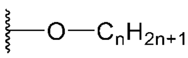
.

The term "NH" refers to imino, with a structural formula of

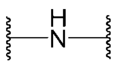
.

The term "CO" refers to carbonyl, with a structural formula of

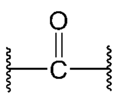
.

The term "CN" refers to cyano, with a structural formula of

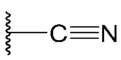
.

The term "trityl" has a structural formula of:

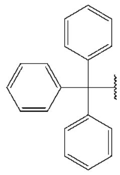
.

The term "4-methoxytrityl" has a structural formula of

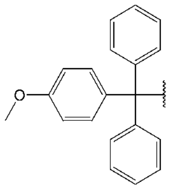
.

The term "4,4'-dimethoxytrityl" has a structural formula of

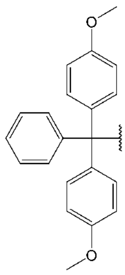
.

The term "4,4',4"-trimethoxytriphenyl" has a structural formula of

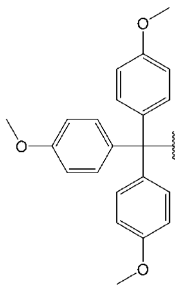
.

The term "" indicates the site where the group is connected via a covalent bond.

In the chemical structure of a ligand or compound, the bond "-" indicates that the configuration is unspecified. If chiral isomerism exists in the chemical structure, the bond "-" can be ""," "," or include both the configurations "" and "". Although all the above structural formulas are depicted as certain isomeric forms for the sake of simplicity, the present disclosure may include all isomers, such as tautomers, rotamers, geometric isomers, diastereomers, racemates, and enantiomers.

In the chemical structure of a ligand or compound, the bond "" indicates that the configuration is unspecified. If cis-trans isomerism exists in the chemical structure, the configuration of the bond "" may be the E-configuration, the Z-configuration, or include both the E- and Z-configurations.

Unless otherwise specified, the expression "selected from ... and ..." in the present disclosure means that at least one thereof can be selected.

Unless otherwise specified, the expression "is ... or ..." in the present disclosure means that any one thereof can be selected.

Unless otherwise specified, the "compound," "ligand," "nucleic acid-ligand conjugate," and "nucleic acid" in the present disclosure may each independently exist in the form of a salt, a mixed salt, or a non-salt (e.g., a free acid or a free base). When present in the form of a salt or a mixed salt, it may be a pharmaceutically acceptable salt.

The term "pharmaceutically acceptable salt" includes pharmaceutically acceptable acid addition salts and pharmaceutically acceptable base addition salts.

The term "pharmaceutically acceptable acid addition salt" refers to a salt formed with an inorganic acid or an organic acid that can retain the biological effectiveness of the free base without other side effects. Inorganic acid salts include, but are not limited to, hydrochloride, hydrobromide, sulfate, nitrate, phosphate, and the like. Organic acid salts include, but are not limited to, formate, acetate, 2,2-dichloroacetate, trifluoroacetate, propionate, hexanoate, octanoate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalenedisulfonate, and the like. These salts may be prepared by methods known in the art.

The term "pharmaceutically acceptable base addition salt" refers to a salt formed with an inorganic base or an organic base that retains the biological effectiveness of the free acid without other side effects. Salts derived from inorganic bases include, but are not limited to, sodium salt, potassium salt, lithium salt, ammonium salt, calcium salt, magnesium salt, iron salt, zinc salt, copper salt, manganese salt, aluminum salt, and the like. Preferred inorganic salts are ammonium salt, sodium salt, potassium salt, calcium salt, and magnesium salt; preferably sodium salt. Salts derived from organic bases include, but are not limited to, salts of the following: primary amines, secondary amines, and tertiary amines; substituted amines, including naturally occurring substituted amines, cyclic amines; and basic ion exchange resin, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, N-ethylpiperidine, polyamine resin, and the like. Preferred organic bases include isopropylamine, diethylamine, ethanolamine, trimethylamine, dicyclohexylamine, choline, and caffeine. These salts may be prepared by methods known in the art.

The term "oligonucleotide" refers to deoxyribonucleic acid (DNA) or ribonucleic acid (RNA), typically consisting of 10 to 50 nucleotides. Oligonucleotides can regulate gene expression through a series of processes such as RNA interference, ribonuclease-mediated target degradation, splicing regulation, non-coding RNA inhibition, gene activation, and programmed gene editing.

The term "antisense oligonucleotide (ASO)" refers to a single-stranded oligonucleotide molecule, typically consisting of 10 to 50 nucleotides. After entering the cell, ASOs bind to their complementary target mRNA via the principle of base pairing under the action of ribonuclease H1, thereby inhibiting the expression of the target gene.

The term "target gene" refers to the gene that encodes the target protein.

The term "target nucleic acid" or "target gene nucleic acid" refers to any nucleic acid molecule whose expression or activity can be regulated by double-stranded oligonucleotide compounds. Target nucleic acids include, but are not limited to, RNA transcribed from DNA encoding a target protein (including, but not limited to, pre-mRNA, mRNA, or portions thereof), and cDNA derived from such RNA and miRNA. For example, the target nucleic acid may be a cellular gene (or mRNA transcribed from the gene) whose expression is associated with a particular condition or disease state.

"Gene suppression" by an RNA interference molecule refers to a reduction in the mRNA level of a target gene in a cell by at least about 50%, at least about 60%, at least about 70%, at least about 80%, at least about 90%, at least about 95%, and any integer therebetween.

The term "regulating gene expression" refers to the upregulation or downregulation of gene expression, or the level of RNA molecules or equivalent RNA molecules encoding one or more proteins or protein subunits, such that the expression, level, or activity is greater than or less than the value observed in the absence of regulation.

The term "complementary" means that a nucleic acid can form hydrogen bonds with another nucleic acid sequence through conventional Watson-Crick or other non-conventional types. The "complementary" or fully complementary means that all consecutive residues of a nucleic acid sequence will form hydrogen bonds with the same number of consecutive residues in a second nucleic acid sequence. Incompletely complementary refers to a situation where some (but not all) of the nucleoside units in two strands can form hydrogen bonds with one another. "Substantially complementary" means that a polynucleotide strand exhibits complementarity with no more than three nucleotide mismatches, excluding any regions intentionally designed to be non-complementary (e.g., overhangs) in the polynucleotide strand.

The terms "chimera," "construct," or "fusion" have the same meaning and refer to a compound including at least two oligonucleotide molecules, which may be the same or different.

The terms "targeting" or "targeting to" refer to the association of the antisense strand of an siRNA with a specific target nucleic acid molecule or a specific nucleotide region within a target nucleic acid molecule.

The terms "2'-modified" or "2'-substituted" refer to a sugar containing a substituent other than H or OH at the 2' position. A 2'-modified monomer includes, but not limited to, BNA and a monomer with 2'-substituent (e.g., a nucleoside and nucleotide).

The term "optionally substituted" means that a group may or may not have a substituent; and the examples of substituents include, but are not limited to, $C_1$-$C_3$ alkyl, $C_1$-$C_3$ alkoxy, amino, hydroxyl, halogen, aryl, and the like. Preferred

substituents are $C_1$-$C_3$ alkyl, $C_1$-$C_3$ alkoxy, and fluoro.

Unless otherwise specified, the terms "multi-target siRNA conjugate," "multi-targeting siRNA conjugate," "siRNA chimera," and "nucleic acid chimera" used in the present disclosure are used interchangeably.

Unless otherwise specified, in the sequences of the present disclosure, the meaning of the base compositions and modification are as follows: uppercase letters A, U, G, C, and T represent the base compositions of nucleotides; the lowercase letter m indicates that the nucleotide represented by the uppercase letter immediately adjacent to its left is a 2'-methoxy modified nucleotide; the lowercase letter f indicates that the nucleotide represented by the uppercase letter immediately adjacent to its left is a 2'-fluoro modified nucleotide; the lowercase letter d indicates that the nucleotide represented by the uppercase letter immediately adjacent to the left of the letter d is a 2'-deoxy modified nucleotide; the composite identifier (moe) indicates that the nucleotide represented by the uppercase letter immediately adjacent to its left is a 2'-O-methoxyethyl modified nucleotide; the structural formula of (CR01008) is

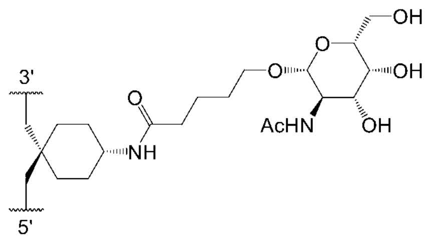

;

the lowercase letter s indicates a phosphorothioate linkage between the nucleotide represented by the uppercase letter or (CR01008) immediately adjacent to its left and the nucleotide represented by the uppercase letter or (CR01008) immediately adjacent to its right. VP indicates that the 5'-phosphate of the nucleotide represented by the letter immediately following it is modified to a nucleotide containing a vinyl phosphonate. The structural formula of VPUm is

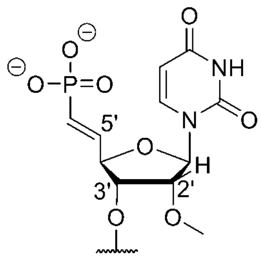

.

## Nucleic Acid Chimera

In the first aspect, the present disclosure provides a nucleic acid chimera, comprising a nucleotide unit I, where the nucleotide unit I has a structure represented by formula (101):

$$H-\left(X_1\right)_{j_1}-Y_1-\left[\left(X_2\right)_{j_2}-Y_2\right]_k-\left(X_3\right)_{j_3}-H$$

(101)

where each of $X_1$, $X_2$, and $X_3$ is independently selected from

$$*-\left(M_1\right)_m-\left(M_2\right)_n-*\ ,$$

and each $M_1$ independently comprises a ligand capable of binding to a cellular receptor; each $M_2$ is independently selected from a modified or unmodified nucleotide; and each m is independently selected from 1, 2, or 3, and each n is independently selected from an integer of 0-3;

where $X_1$, $X_2$, and $X_3$ may be the same or different; and

where each of $Y_1$ and $Y_2$ independently represents a sense strand of a double-stranded oligonucleotide, and each of the sense strands may correspond to the same target gene or a different target gene; and the corresponding

relationship means that the sense strand is identical or substantially identical to a segment of consecutive nucleotides in the mRNA of the target gene;

$j_1$, $j_2$, and $j_3$ are independently selected from an integer of 0-3, and $j_2 \neq 0$; and

k is selected from an integer of 1 to 5.

In some embodiments, n is independently selected from an integer of 0 to 2; for example, each n is independently selected from 0 or 1.

In some embodiments, k is selected from 1;

In some embodiments, $j_1$ is selected from 0, $j_2$ is selected from 3, and $j_3$ is selected from 0;

In some embodiments, $j_1$ is selected from 0, $j_2$ is selected from 3, and $j_3$ is selected from 3;

In some embodiments, $j_1$ is selected from 1, $j_2$ is selected from 1, and $j_3$ is selected from 1;

In some embodiments, $j_1$ is selected from 2, $j_2$ is selected from 2, and $j_3$ is selected from 2;

In some embodiments, $j_1$ is selected from 3, $j_2$ is selected from 3, and $j_3$ is selected from 0;

In some embodiments, $j_1$ is selected from 3, $j_2$ is selected from 0, and $j_3$ is selected from 3;

In some embodiments, m is selected from 1, 2, or 3, and each n is independently selected from 0 or 1;

In some embodiments, m is selected from 1, and n is selected from 0; or, m is selected from 1, and n is selected from 1.

In some embodiments, the nucleic acid chimera further comprises a nucleotide unit **II,** wherein the nucleotide unit II comprises an antisense strand complementary or substantially complementary to the sense strand in the double-stranded oligonucleotide, the number of the antisense strands is equal to the number of the sense strands, and each of the antisense strands is at least partially complementary to a corresponding sense strand thereof to form a duplex region; where the antisense strand is complementary or at least partially complementary to an mRNA of at least one target gene.

In some embodiments, the antisense strand is complementary or at least partially complementary to mRNAs of two or at least two target genes.

In other embodiments, one of the antisense strands is complementary or at least partially complementary to a portion of the mRNA of target gene A; and another of the antisense strands is complementary or at least partially complementary to a portion of the mRNA of target gene B, wherein the target gene A and the target gene B may be the same or different.

In some embodiments, the sense and antisense strands of the double-stranded oligonucleotide each comprise at least partially modified nucleotides; and preferably, all nucleotides in the sense and antisense strands are modified nucleotides.

In some embodiments, the sense strand and/or the antisense strand comprise at least one phosphorothioate linkage; preferably, nucleotides at positions 1 and 2 from the 5' terminus of the sense and antisense strands are linked to adjacent nucleotides via phosphorothioate linkages.

In some embodiments, the sense strand and the antisense strand comprise at least 15 consecutive nucleotides, where the antisense strand has complementarity to the mRNA of the target gene.

In some embodiments, one or more unmodified nucleotides are present in $X_2$. Optionally, unmodified nucleotides may serve as structural units that promote cleavage or hydrolysis of the chimera.

After *in vivo* administration, the chimera described herein may optionally be cleaved at an intermediate position, e.g., at $X_2$, or at a position of the one or more unmodified nucleotides, thereby providing two or more independent double-stranded oligonucleotides.

For example, in some embodiments, the chimera is cleaved at $X_2$ or at a linkage position with $X_2$.

In some embodiments, after *in vivo* administration, the chimera may be cleaved to provide at least two independent double-stranded oligonucleotide portions, where the independent double-stranded oligonucleotides target the same target gene or the different target genes respectively, to allow expression regulation of the same target gene or different target genes.

In other embodiments, after *in vivo* administration, the chimera may be cleaved (including complete cleavage or partial cleavage) to provide two independent portions each comprising a double-stranded oligonucleotide; where a first double-stranded oligonucleotide targets a portion of the mRNA of the target gene A, the second double-stranded oligonucleotide targets a portion of the mRNA of the target gene B, and the first double-stranded oligonucleotide is capable of regulating expression of the target gene A, and the second double-stranded oligonucleotide is capable of regulating expression of the target gene B.

In some embodiments, when the chimera is partially cleaved, an uncleaved chimera can regulate expression of a target gene associated with any of the double-stranded oligonucleotides.

In some embodiments, the nucleic acid chimera comprises a first double-stranded oligonucleotide (siRNA) molecule and a second double-stranded oligonucleotide molecule; optionally, where a sense strand of the first double-stranded oligonucleotide and/or the second double-stranded oligonucleotide comprises a single-stranded overhang at a 3' terminus thereof; and optionally, an antisense strand of the first and/or second double-stranded oligonucleotide

comprises an overhang at a 3' terminus thereof, where a nucleic acid sequence of the single-stranded overhang of the sense strand is complementary or substantially complementary to a nucleotide sequence of the single-stranded overhang of the antisense strand, and the first double-stranded oligonucleotide and the second double-stranded oligonucleotide each are conjugated to at least one ligand.

In some embodiments, the overhang includes 1-3 nucleotides or 1-2 nucleotides. Optionally, the overhang is entirely DNA, entirely RNA, or comprises a mixture of DNA and RNA nucleotides, and the DNA and RNA may be natural or modified.

In some embodiments, the first double-stranded oligonucleotide regulates expression of a first target gene, and the second double-stranded oligonucleotide regulates expression of a second target gene, wherein the first target gene and the second target gene are different.

In some embodiments, the nucleic acid chimera is such that, as compared to when the first and second double-stranded oligonucleotides are not linked together, the first and second double-stranded oligonucleotides independently regulate at least 70%, at least 75%, or at least 80% expression level of their respective target genes.

In some embodiments, the nucleic acid chimera comprises at least partially modified nucleotides. In other embodiments, the double-stranded oligonucleotides in the nucleic acid chimera are all modified nucleotide.

The modified nucleotide is independently selected from an abasic nucleotide, a 2'-halogenated modified nucleotide, a 2'-deoxy modified nucleotide or a 2'-O-$(CH_2)_{n2}$-$R_1$ modified nucleotide, or a nucleotide analog; and the nucleotide analog is selected from one or more of peptide nucleic acid (PNA), morpholino (MNA), bridged nucleic acid (BNA), locked nucleic acid (LNA), glycol nucleic acid (GNA), threose nucleic acid (TNA), and unlocked nucleic acid (UNA); where $n_2$ is selected from 0, 1, or 2; $R_1$ is selected from optionally substituted $C_{1-6}$ alkyl, optionally substituted $C_{1-6}$ alkoxy, or -Si$(R_{1a})_3$, each $R_{1a}$ is independently selected from optionally substituted $C_{1-6}$ alkyl or optionally substituted $C_{1-6}$ alkoxy, and the term "substituted" means one or more hydrogen atoms on the alkyl or the alkoxy being replaced by substituents; and optionally, each substituent is independently selected from $C_1$-$C_3$ alkyl, $C_1$-$C_3$ alkoxy, or halogen.

In some embodiments, 2'-O-$(CH_2)_{n2}$-$R_1$ is selected from 2'-O-$CH_3$, 2'-O-$CH_2$-$CH_3$, 2'-O-TBDMS, 2'-O-TIPS, 2'-O-TOM, 2'-O-$CH_2$-O-$CH_2$-$CH_3$, 2'-O-$CH_2$-O-$CH_2$-$CF_3$ or 2'-O-$CH_2$-$CH_2$-O-$CH_3$.

In some embodiments, the sense strand and the antisense strand of the double-stranded oligonucleotide are complementary or substantially complementary to form a duplexe, wherein the sense strand and the antisense strand are as shown in the following formulas:

SS: 5'- (N)a' - (X)p' - (N)b' - (X)q' - (N)c' - (X)r' - (N) d' -3'

AS: 3' - (N)a - (X)p - (N)b - (X)q - (N)c -5' ,

wherein, SS represents the sense strand, and AS represents the antisense strand; and all nucleotides of the sense and antisense strands are modified nucleotides;

each N independently represents the following modified nucleotide: a 2'-O-methyl modified nucleotide, a 2'-fluoro modified nucleotide, or a 2'-deoxy-modified nucleotide;

each X independently represents a 2'-O-methoxyethyl modified nucleotide, a 2'-O-methyl modified nucleotide, or a nucleotide modified by substitution with 2'-$O(CH_2)_{n3}OR_3$; wherein $n_3$ is 1 or 2, and R is selected from substituted or unsubstituted $C_1$-$C_6$ alkyl, or substituted or unsubstituted $C_1$-$C_6$ alkoxy; and optionally, a substituent is selected from a halogen, $C_1$-$C_3$ alkyl, $C_1$-$C_3$ alkoxy, hydroxyl, or amino;

a, a', p, p', b, b', q, q', c, c', r', and d' each independently represent the number of nucleotides, wherein: a' is selected from an integer of 3-8; p' is selected from an integer of 0-3; b' is selected from an integer of 4-13; q' is selected from an integer of 0-4; c' is selected from an integer of 3-9; r' is selected from an integer of 0-3; d' is selected from an integer of 0-9; a is selected from an integer of 4-7; p is selected from an integer of 0-1; b is selected from an integer of 4-8; q is selected from an integer of 0-4; c is selected from an integer of 6-10; and p', q', r', p, and q are not simultaneously 0, and $0 \leq q' + r' \leq 4$.

In some embodiments, a' is selected from an integer of 3-8; p' is selected from 0 or 1; b' is selected from an integer of 4-13; q' is selected from 0 or 1; c' is selected from an integer of 3-9; r' is selected from 0 or 1; d' is selected from an integer of 1-8; a is selected from an integer of 4-7; p is selected from 1; b is selected from an integer of 4-8; q is selected from 0 or 1; and c is selected from an integer of 6-10.

In some embodiments, the nucleotide N is selected from a 2'-O-methyl modified nucleotide or a 2'-fluoro modified nucleotide, and the nucleotide X is selected from a 2'-O-methoxyethyl modified nucleotide or a 2'-O-methyl modified nucleotide, and further preferably a 2'-O-methoxyethyl modified nucleotide.

In some embodiments, the sense strand is 17-21 nucleotides in length, and the antisense strand is 19-23 nucleotides in length.

In some embodiments, in the direction from the 5' end to the 3' end, in the sense strand ,at least three of the

nucleotides at positions 7-10 of the nucleotide sequence are selected from 2'-fluoro modified nucleotides, and the nucleotides at the remaining positions are selected from 2'-O-methyl modified nucleotides or 2'-O-methoxyethyl modified nucleotides; and in the antisense strand, the nucleotides at positions 2, 6, 14, and 16 of the nucleotide sequence are selected from 2'-fluoro modified nucleotides, any one of the nucleotides at positions 9, 10, 11, and 12 is selected from a 2'-fluoro modified nucleotide, and the nucleotides at the remaining positions are selected from 2'-O-methyl modified nucleotides or 2'-O-methoxyethyl modified nucleotides.

In some embodiments, in the direction from the 5' end to the 3' end, in the sense strand, at least three of the nucleotides at positions 7-10 of the nucleotide sequence are selected from 2'-fluoro modified nucleotides, up to two of the nucleotides at positions 5, 12, and 18 are selected from 2'-O-methoxyethyl modified nucleotides, and the nucleotides at the remaining positions are selected from 2'-O-methyl modified nucleotides; and in the antisense strand, the nucleotides at positions 2, 6, 14, and 16 of the nucleotide sequence are selected from 2'-fluoro modified nucleotides, any one of the nucleotides at positions 9-12 is selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides.

In some embodiments, each nucleotide of the double-stranded oligonucleotide is independently selected from a modified nucleotide, wherein modifications of the sense and antisense strands are selected from any one of the following (1)-(8):

(1) in the direction from the 5' end to the 3' end, in the sense strand, the nucleotides at positions 7-10 of the nucleotide sequence are selected from 2'-fluoro modified nucleotides, and the nucleotides at the remaining positions are selected from 2'-O-methyl modified nucleotides; and in the antisense strand, the nucleotides at positions 2, 6, 9, 14, and 16 of the nucleotide sequence are selected from 2'-fluoro modified nucleotides, and the nucleotides at the remaining positions are selected from 2'-O-methyl modified nucleotides;

(2) in the direction from the 5' end to the 3' end, in the sense strand, the nucleotides at positions 7-10 of the nucleotide sequence are selected from 2'-fluoro modified nucleotides, and the nucleotides at the remaining positions are selected from 2'-O-methyl modified nucleotides; and in the antisense strand, the nucleotides at positions 2, 6, 12, 14, and 16 of the nucleotide sequence are selected from 2'-fluoro modified nucleotides, and the nucleotides at the remaining positions are selected from 2'-O-methyl modified nucleotides;

(3) in the direction from the 5' end to the 3' end, in the sense strand, the nucleotides at positions 7-10 of the nucleotide sequence are selected from 2'-fluoro modified nucleotides, and the nucleotides at the remaining positions are selected from 2'-O-methyl modified nucleotides; and in the antisense strand, the nucleotides at positions 2, 6, 9, 14, and 16 of the nucleotide sequence are selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides;

(4) in the direction from the 5' end to the 3' end, in the sense strand, the nucleotides at positions 7-10 of the nucleotide sequence are selected from 2'-fluoro modified nucleotides, and the nucleotides at the remaining positions are selected from 2'-O-methyl modified nucleotides; and in the antisense strand, the nucleotides at positions 2, 6, 12, 14, and 16 of the nucleotide sequence are selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides;

(5) in the direction from the 5' end to the 3' end, in the sense strand, the nucleotide at position 5, 12, or 18 of the nucleotide sequence is selected from a 2'-O-methoxyethyl modified nucleotide, the nucleotides at positions 7-10 are selected from 2'-fluoro modified nucleotides, and the nucleotides at the remaining positions are selected from 2'-O-methyl modified nucleotides; and in the antisense strand, the nucleotides at positions 2, 6, 9, 14, and 16 of the nucleotide sequence are selected from 2'-fluoro modified nucleotides, and the nucleotides at the remaining positions are selected from 2'-O-methyl modified nucleotides;

(6) in the direction from the 5' end to the 3' end, in the sense strand, the nucleotide at position 5, 12, or 18 of the nucleotide sequence is selected from a 2'-O-methoxyethyl modified nucleotide, the nucleotides at positions 7-10 are selected from 2'-fluoro modified nucleotides, and the nucleotides at the remaining positions are selected from 2'-O-methyl modified nucleotides; and in the antisense strand, the nucleotides at positions 2, 6, 12, 14, and 16 of the nucleotide sequence are selected from 2'-fluoro modified nucleotides, and the nucleotides at the remaining positions are selected from 2'-O-methyl modified nucleotides;

(7) in the direction from the 5' end to the 3' end, in the sense strand, the nucleotide at position 5, 12, or 18 of the nucleotide sequence is selected from a 2'-O-methoxyethyl modified nucleotide, the nucleotides at positions 7-10 are selected from 2'-fluoro modified nucleotides, and the nucleotides at the remaining positions are selected from 2'-O-methyl modified nucleotides; and in the antisense strand, the nucleotides at positions 2, 6, 9, 14 and 16 of the nucleotide sequence are selected from 2'-fluoro modified nucleotides, the nucleotide at position 15 is selected from a 2'-O-methoxyethyl modified nucleotide, and the nucleotides at the remaining positions are selected from 2'-O-methyl

modified nucleotides; and

(8) in the direction from the 5' end to the 3' end, in the sense strand, the nucleotide at position 5, 12 or 18 of the nucleotide sequence is selected from a 2'-O-methoxyethyl modified nucleotide, the nucleotides at positions 7-10 are selected from 2'-fluoro modified nucleotides, and the nucleotides at the remaining positions are selected from 2'-O-methyl modified nucleotides; and in the antisense strand, the nucleotides at positions 2, 6, 12, 14 and 16 of the nucleotide sequence are selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides.

Optionally, in the duplex, in the direction from the 5' end to the 3' end, the sense strand comprises 1 or 2 consecutive phosphorothioate linkages between the terminal nucleotides at the 5' terminus; and/or the antisense strand independently comprises 1 or 2 consecutive phosphorothioate linkages at the 5' and 3' terminus, respectively.

In some embodiments, in the nucleic acid chimera described in the present disclosure, the nucleotide unit I has a structure represented by formula (102):

$$H-\!\left(M_1\right)_{j_1}-Y_1-\!\left[\left(M_1\right)_{j_2}-Y_2\right]_k-\!\left(M_1\right)_{j_3}-H$$

(102) .

In some embodiments, in the nucleic acid chimera described in the present disclosure, the nucleotide unit I has a structure represented by formula (103):

$$H-\!\left(M_1-M_2\right)_{j_1}-Y_1-\!\left[\left(M_1-M_2\right)_{j_2}-Y_2\right]_k-\!\left(M_1-M_2\right)_{j_3}-H$$

(103) .

In some embodiments, in the nucleic acid chimera described in the present disclosure, each $M_1$ is independently selected from a ligand capable of binding to the asialoglycoprotein receptor.

In some embodiments, in the nucleic acid chimera described in the present disclosure, each $M_1$ is independently selected from a structure represented by formula (201a) or formula (201b), or an isomer thereof, or a pharmaceutically acceptable salt thereof:

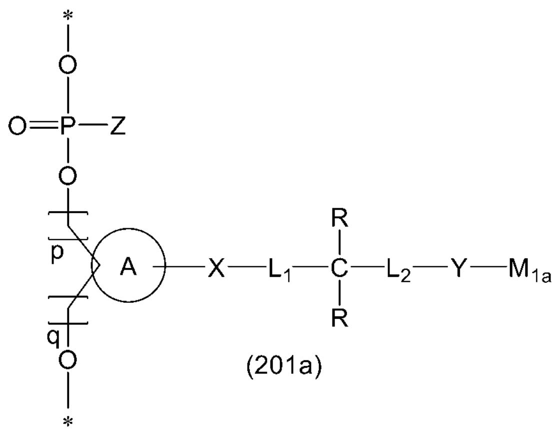

(201a) ,

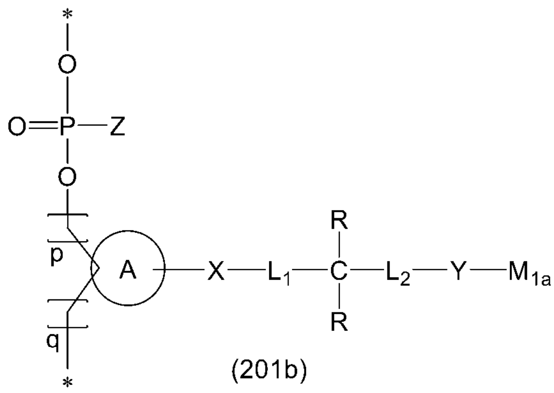

(201b)

wherein, when $M_1$ is located at a terminal position of the nucleotide unit I, $M_1$ is selected from the structure represented by formula (201a), or an isomer thereof, or a pharmaceutically acceptable salt thereof; and

when $M_1$ is not located at a terminal position of the nucleotide unit I, $M_1$ is selected from the structure represented by formula (201b), or an isomer thereof, or a pharmaceutically acceptable salt thereof;

wherein A is selected from an optionally substituted 4- to 10-membered alicyclic ring;

X is selected from NH, O, or S;

$L_1$ is selected from

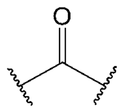, 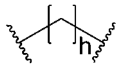, or 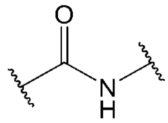;

wherein h is 1, 2, 3, 4, 5, 6, 7, 8, 9, or 10;
each R is independently selected from H, optionally substituted $C_{1-6}$ alkyl, or optionally substituted $C_{1-6}$ alkoxy;
$L_2$ is selected from optionally substituted $C_2$-$C_{20}$ alkylene or

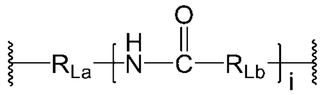;

and $R_{La}$ and $R_{La}$ are independently selected from optionally substituted $C_{1-10}$ alkylene, and i is selected from 1, 2, 3, 4, or 5;
Y is selected from O or S;
$M_{1a}$ is selected from galactose or a derivative thereof, and Z is selected from hydroxyl or sulfhydryl;
p is selected from 1, 2, or 3; and
q is selected from 1, 2, or 3.

In some embodiments, A is selected from

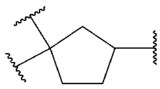, 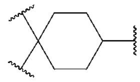, 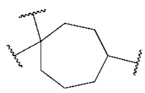, 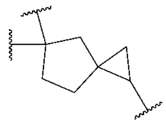,

or

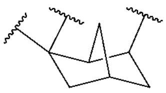.

In some embodiments, A is selected from

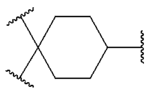.

In some embodiments, X is selected from NH.

In some embodiments, $L_1$ is

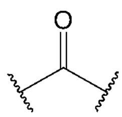.

In some embodiments, each R is independently selected from H.

In some embodiments, $L_2$ is selected from $C_{1-10}$ alkylene or

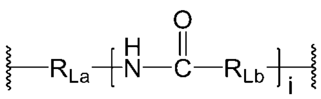;

wherein $R_{La}$ and $R_{Lb}$ are independently selected from $C_{1-5}$ alkylene, and i is 1, 2, or 3.

In some embodiments, i is selected from 1.

In some embodiments, each $L_2$ is independently selected from

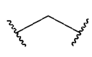, 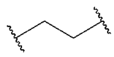, 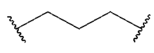,

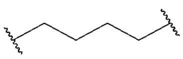, 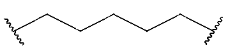, 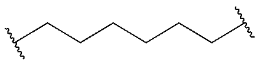, 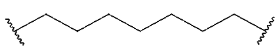,

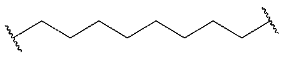, 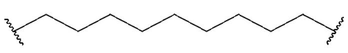, 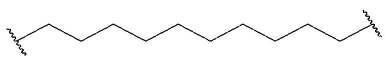,

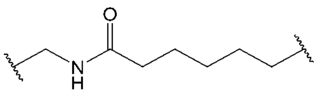, 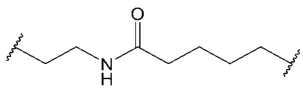, 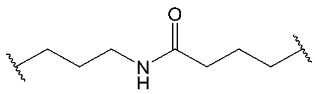,

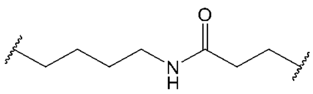, or 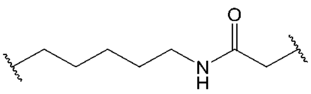.

In some embodiments, $M_{1a}$ is selected from a galactosamine derivative.

In some embodiments, $M_{1a}$ is selected from

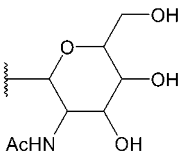.

In some embodiments, Z is selected from hydroxyl.

In some embodiments, Z is selected from sulfhydryl.

In some embodiments, p is selected from 1.

In some embodiments, q is selected from 1.

In some embodiments, in the nucleic acid chimera described in the present disclosure, each $M_1$ is independently selected from a structure represented by formula (202a) or formula (202b), or an isomer thereof or a pharmaceutically acceptable salt thereof:

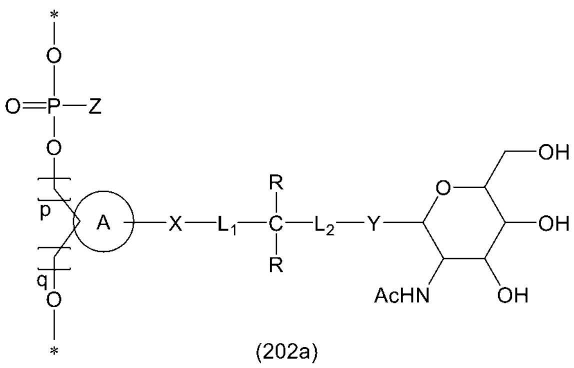

(202a),

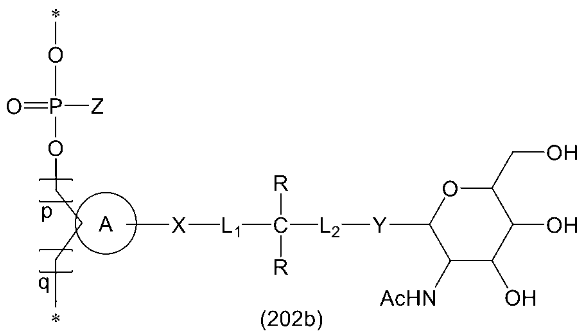

(202b);

wherein when $M_1$ is located at a terminal position of the nucleotide unit I, $M_1$ is selected from the structure represented by formula (202a), or an isomer thereof, or a pharmaceutically acceptable salt thereof; and

when $M_1$ is not located at a terminal position of the nucleotide unit I, $M_1$ is selected from the structure represented by formula (202b), or an isomer thereof, or a pharmaceutically acceptable salt thereof.

In some embodiments, in the nucleic acid chimera described in the present disclosure,

each $M_1$ is independently selected from a structure represented by formula (203a) or formula (203b), or an isomer thereof, or a pharmaceutically acceptable salt thereof:

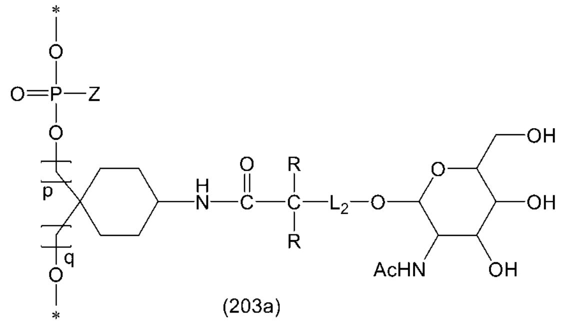
(203a)
,
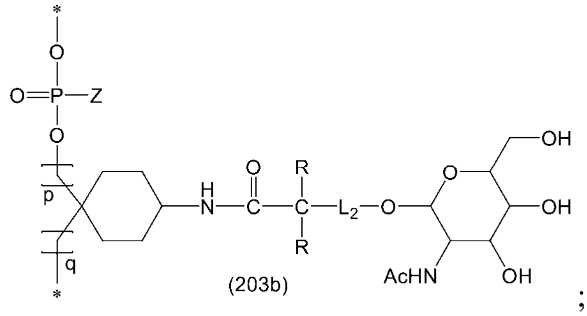
(203b)
;

wherein when $M_1$ is located at a terminal position of the nucleotide unit I, $M_1$ is selected from the structure represented by formula (203a), or an isomer thereof, or apharmaceutically acceptable salt thereof; and
when $M_1$ is not located at a terminal position of the nucleotide unit I, $M_1$ is selected from the structure represented by formula (203b), or an isomer thereof, or a pharmaceutically acceptable salt thereof.

In some embodiments, in the nucleic acid chimera described in the present disclosure, each $M_1$ is independently selected from any one of the following structures or an isomer thereof or a pharmaceutically acceptable salt thereof:

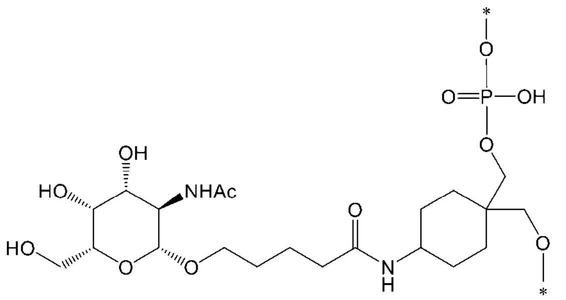
,
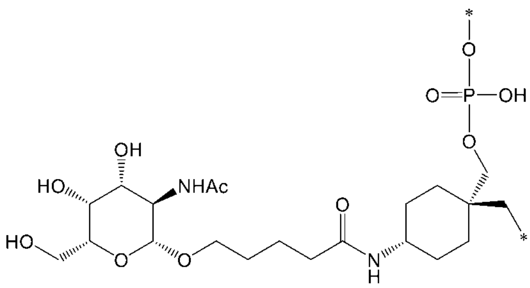
,
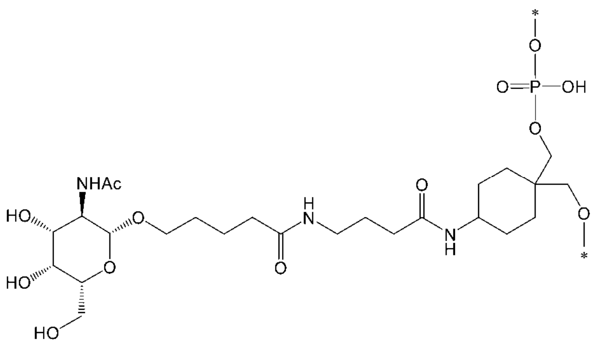
or
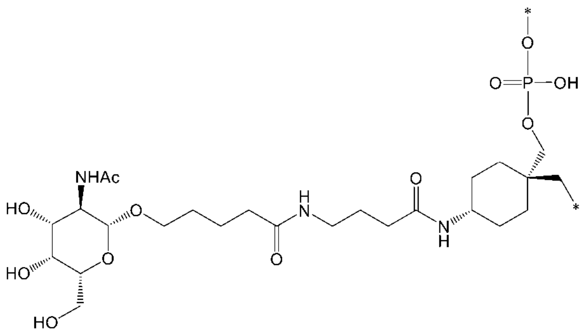
;

wherein when $M_1$ is located at a terminal position of the nucleotide unit I, $M_1$ is selected from

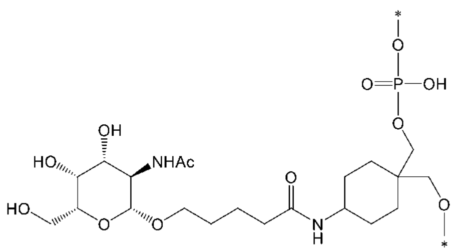

or an isomer thereof or a pharmaceutically acceptable salt thereof, or

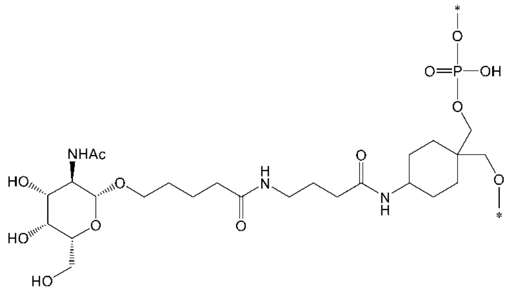

or an isomer thereof or a pharmaceutically acceptable salt thereof;

when $M_1$ is not located at a terminal position of the nucleotide unit I, $M_1$ is selected from

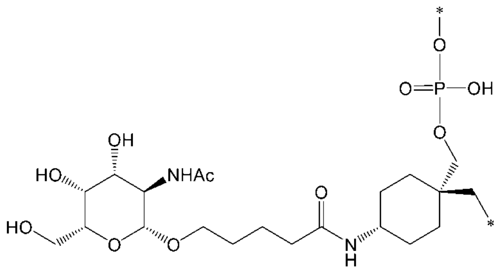

or an isomer thereof or a pharmaceutically acceptable salt thereof, or

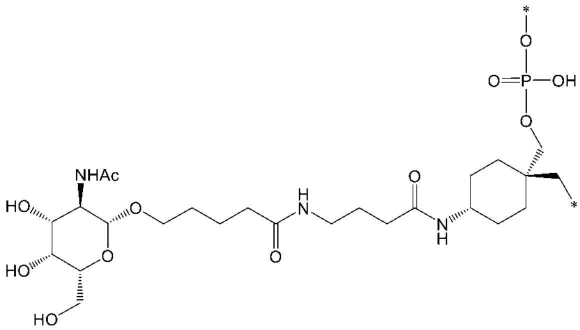

or an isomer thereof or a pharmaceutically acceptable salt thereof.

In some embodiments, in the nucleic acid chimera described in the present disclosure, the nucleotide unit I has a structure represented by formula (104) or an isomer thereof or a pharmaceutically acceptable salt thereof:

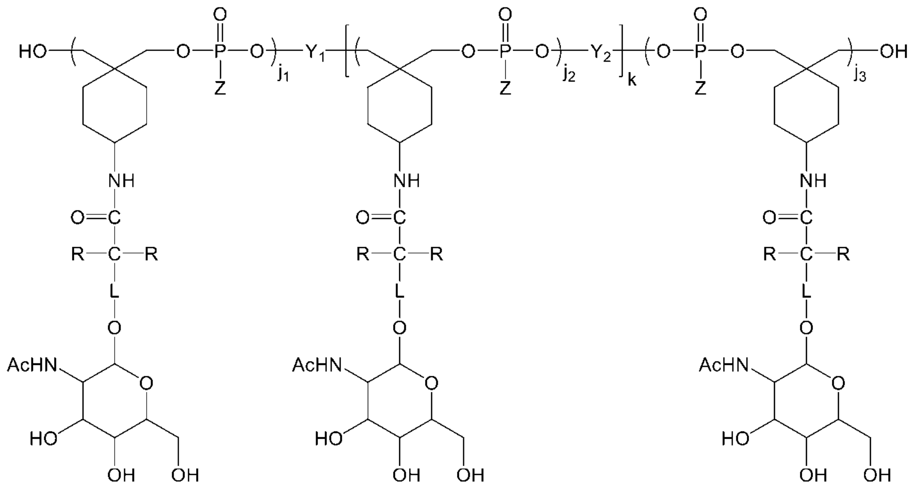

(104)

.

In some embodiments, the nucleic acid chimera according to the present disclosure has a structure represented by formula (105) or an isomer thereof or a pharmaceutically acceptable salt thereof:

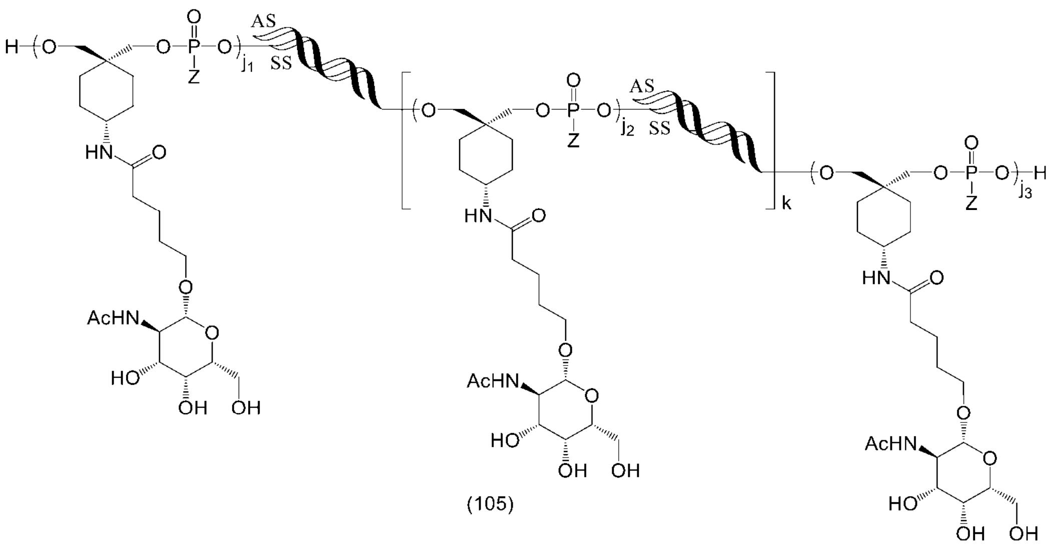

(105)

wherein Z is OH; and

AS represents the antisense strand of the double-stranded oligonucleotide molecule; and SS represents the sense strand of the double-stranded oligonucleotide molecule.

In some embodiments, the nucleic acid chimera according to the present disclosure has a structure represented by formula (106) or an isomer thereof or a pharmaceutically acceptable salt thereof:

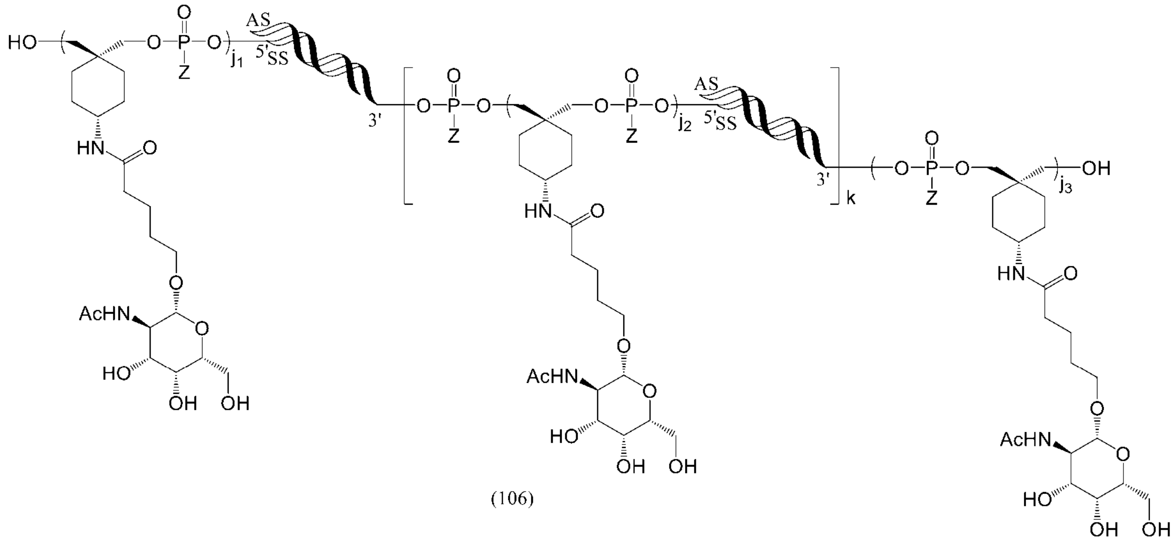

(106)

wherein AS represents the antisense strand of the double-stranded oligonucleotide molecule; and SS represents the sense strand of the double-stranded oligonucleotide molecule;

Z is OH; and

optionally, $j_1$ is selected from 0 or 1, $j_2$ is selected from an integer of 1-3, and k is selected from 1.

In some embodiments, the nucleic acid chimera according to the present disclosure comprises a nucleotide unit III, wherein the nucleotide unit III has a structure represented by formula (301) or an isomer thereof or a pharmaceutically acceptable salt thereof:

$$HO\text{—}(M_1)_{j_4}\text{—}Y_3\text{—}(M_1)_{j_5}\text{—}Dt\text{—}(M_1)_{j_6}\text{—}Y_4\text{—}(M_1)_{j_7}\text{—}OH$$

(301)

wherein, each $M_1$ independently comprises a ligand capable of binding to a cellular receptor; and specifically, each $M_1$ is independently selected from a structure represented by formula (201b) or an isomer thereof or a pharmaceutically acceptable salt thereof:

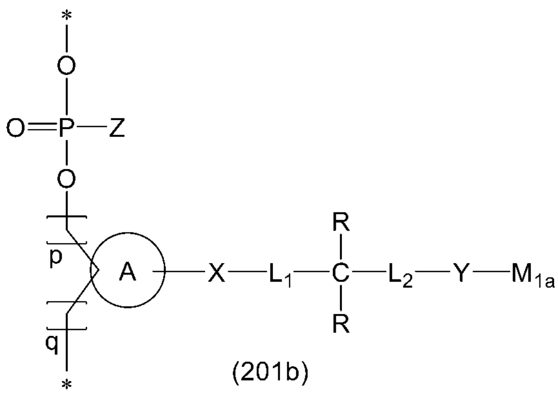

(201b)

A, X, $L_1$, R, $L_2$, Y, $M_{1a}$, p and q are as defined above;

wherein, $Y_3$ is selected from a sense strand and $Y_4$ is selected from a sense strand, or $Y_3$ is selected from an antisense strand and $Y_4$ is selected from a sense strand; $Y_3$ and $Y_4$ in the nucleotide unit III correspond to the same target gene or different target genes; and the corresponding relationship means that the sense strand is identical or substantially identical to a segment of consecutive nucleotides in the mRNA of the target gene, or the antisense strand is complementary or substantially complementary to a segment of consecutive nucleotides in the mRNA of the target gene; and when $Y_3$ is selected from the antisense strand and $Y_4$ is selected from the sense strand, the antisense strand $Y_3$ and the sense strand $Y_4$ in the nucleotide unit III do not form base pairing;

$j_4$, $j_5$, $j_6$, and $j_7$ are each independently selected from integers of 0-3, and at least one of $j_6$ and $j_7$ is not 0; and when $Y_3$ is selected from the sense strand, at least one of $j_4$ and $j_5$ is not 0.

In some embodiments, when $Y_3$ is selected from the sense strand and $Y_4$ is selected from the sense strand, the 5' terminus of $Y_3$ is conjugated to the 5' terminus of $Y_4$, or the 3' terminus of $Y_3$ is conjugated to the 3' terminus of $Y_4$; and Dt has a structure represented by formula (401) or a stereoisomer thereof or a tautomer thereof:

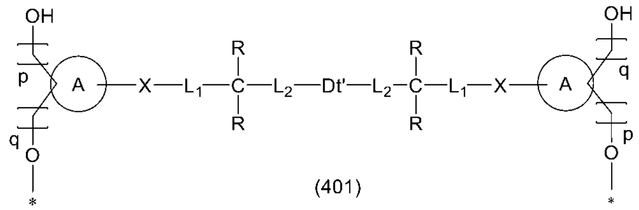

(401)

wherein A, X, $L_1$, R, $L_2$, p, and q are as defined above;

Dt' is selected from

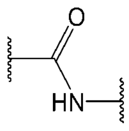, 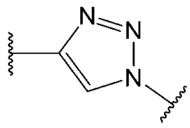, 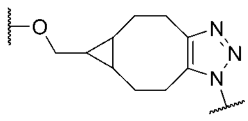, or 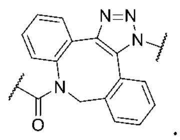.

In some embodiments, when $Y_3$ is selected from the antisense strand and $Y_4$ is selected from the sense strand, the 5' terminus of $Y_3$ is conjugated to the 3' terminus of $Y_4$, or the 3' terminus of $Y_3$ is conjugated to the 5' terminus of $Y_4$; and Dt has a structure represented by formula (501) or a stereoisomer thereof or a tautomer thereof:

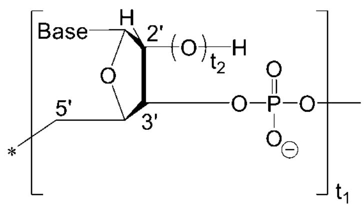

(501)

wherein $t_1$ is selected from an integer of 0-5, $t_2$ is independently selected from 0 or 1, and Base is selected from a nucleobase A, U, G, C, or T; and when $t_1$ is 0, $j_6$ is not 0.

In some embodiments, when $Y_3$ is selected from the sense strand, and $Y_4$ is selected from the sense strand, Dt has a structure represented by formula (402) or a stereoisomer thereof or a tautomer thereof:

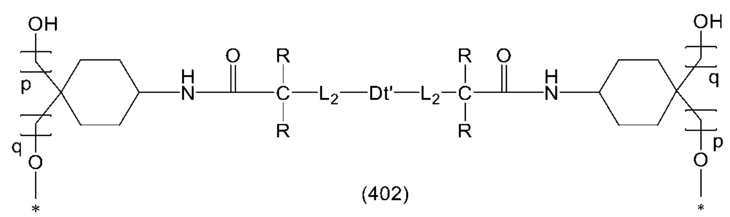

(402)

.

In some embodiments, Dt' is selected from

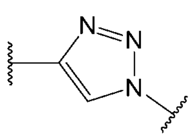

.

In some embodiments, when $Y_3$ is selected from the sense strand and $Y_4$ is selected from the sense strand, Dt has a structure represented by formula (403) or a stereoisomer thereof or a tautomer thereof:

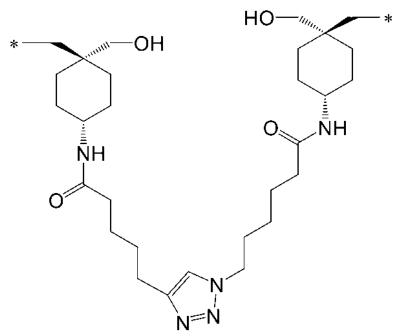

(403)

.

In some embodiments, when $Y_3$ is selected from the sense strand and $Y_4$ is selected from the sense strand, the nucleotide unit III has a structure represented by formula (301a) or an isomer thereof or a pharmaceutically acceptable salt thereof:

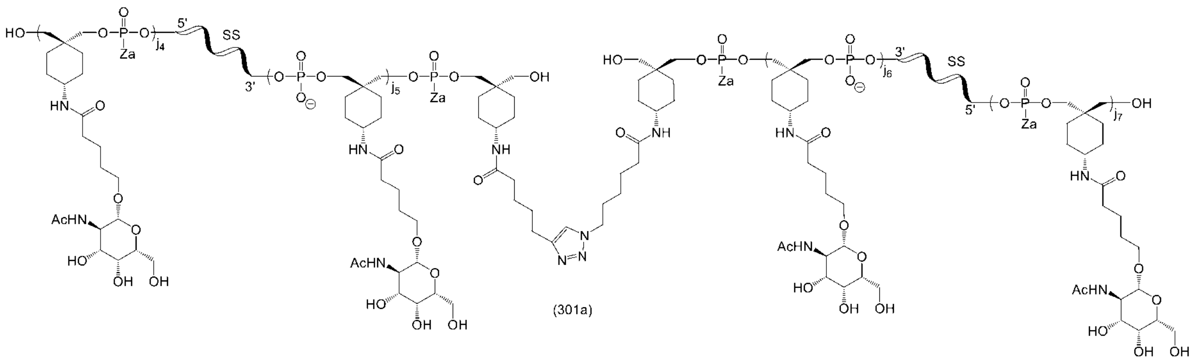

(301a)

wherein each Za is independently selected from hydroxyl or sulfhydryl.

In some embodiments, when $Y_3$ is selected from the sense strand and $Y_4$ is selected from the sense strand, the nucleic acid chimera further comprises two antisense strands, the nucleotide sequence of one antisense strand is at least partially reverse-complementary to the nucleotide sequence of the sense strand $Y_3$ in the nucleotide unit III, and the nucleotide sequence of another antisense strand is at least partially reverse-complementary to the nucleotide sequence of the sense strand $Y_4$ in the nucleotide unit III.

In some embodiments, when $Y_3$ is selected from the antisense strand and $Y_4$ is selected from the sense strand, the nucleotide unit III has a structure represented by formula (301b) or an isomer thereof or a pharmaceutically acceptable salt thereof:

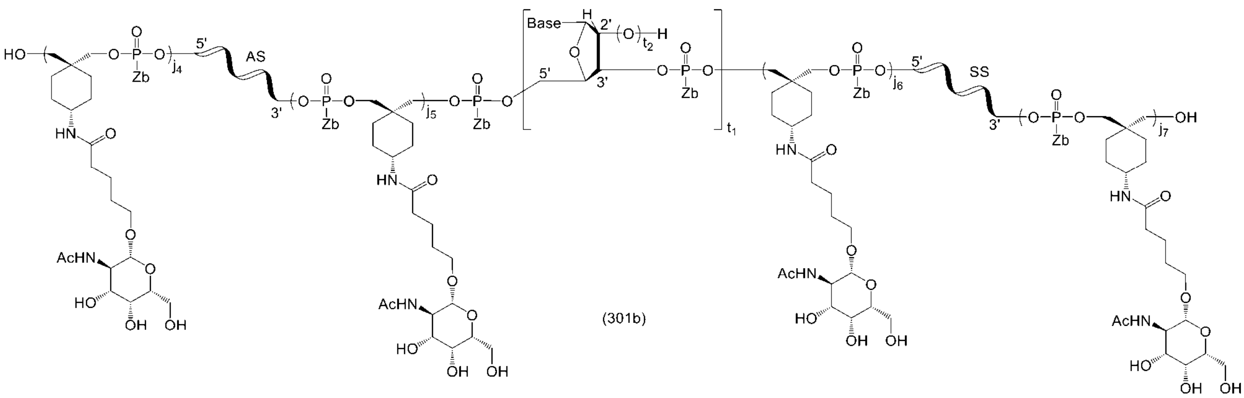

(301b)

wherein each Zb is independently selected from hydroxyl or sulfhydryl.

In some embodiments, when $Y_3$ is selected from the antisense strand and $Y_4$ is selected from the sense strand,

the nucleotide unit III has a structure represented by formula (302b) or an isomer thereof or a pharmaceutically acceptable salt thereof:

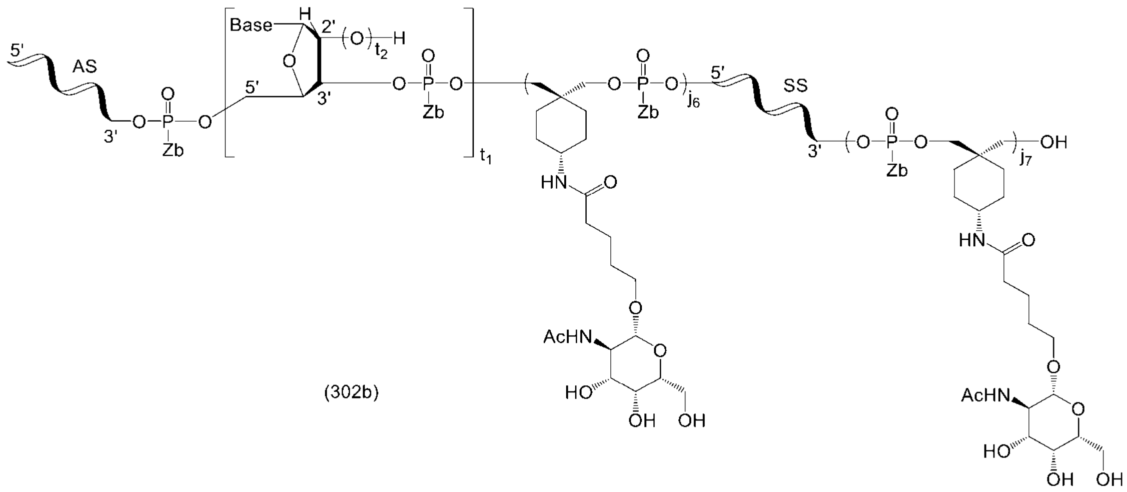

(302b)

wherein each Zb is independently selected from hydroxyl or sulfhydryl.

In some embodiments, when $Y_3$ is selected from the antisense strand and $Y_4$ is selected from the sense strand, the nucleic acid chimera comprises two nucleotide units III, and the antisense strand $Y_3$ and the sense strand $Y_4$ in the same nucleotide unit III correspond to the same target gene or different target genes, and the antisense strand $Y_3$ and the sense strand $Y_4$ in the same nucleotide unit III do not form base complementary pairing;

the nucleotide sequence of the antisense strand $Y_3$ in a first nucleotide unit III is at least partially reverse-complementary to the nucleotide sequence of the sense strand $Y_4$ in a second nucleotide unit III; and

the nucleotide sequence of the sense strand $Y_4$ in the first nucleotide unit III is at least partially reverse-complementary to the nucleotide sequence of the antisense strand $Y_3$ in the second nucleotide unit III.

In some embodiments, when $Y_3$ is selected from the antisense strand and $Y_4$ is selected from the sense strand, the nucleic acid chimera further comprises one sense strand and one antisense strand, wherein the nucleotide sequence of the sense strand is at least partially reverse-complementary to the nucleotide sequence of the antisense strand $Y_3$ in the nucleotide unit III; and the nucleotide sequence of the antisense strand is at least partially reverse-complementary to the nucleotide sequence of the sense strand $Y_4$ in the nucleotide unit III.

In some embodiments, the nucleic acid chimera consists of two nucleotide units III, wherein in the first nucleotide unit III, $Y_3$ is the antisense strand of an siRNA corresponding to the target gene A and $Y_4$ is the sense strand of an siRNA corresponding to the target gene B, and in the second nucleotide unit III, $Y_4$ is the sense strand of the siRNA corresponding to the target gene A and $Y_3$ is the antisense strand of the siRNA corresponding to the target gene B; and the antisense strand $Y_3$ of the siRNA corresponding to the target gene A in the first nucleotide unit III is at least partially reverse complementary to the sense strand $Y_4$ of the siRNA corresponding to the target gene A in the second nucleotide unit III; and the sense strand $Y_4$ of the siRNA corresponding to the target gene B in the first nucleotide unit III is at least partially reverse complementary to the antisense strand $Y_3$ of the siRNA corresponding to the target gene B in the second nucleotide unit III; wherein the target gene A and the target gene B may be the same or different; and the antisense strand $Y_3$ of the siRNA corresponding to the target gene A and the sense strand $Y_4$ of the siRNA corresponding to the target gene B in the first nucleotide unit III do not form base pairing, and the sense strand $Y_4$ of the siRNA corresponding to the target gene A and the antisense strand $Y_3$ of the siRNA corresponding to the target gene B in the second nucleotide unit III do not form base pairing.

In some embodiments, Y3 and Y4 in formula (301) independently represent the sense strand or the antisense strand of an siRNA; and the siRNAs may correspond to the same or different target genes.

In some embodiments, the nucleic acid chimera according to the present disclosure may comprises two nucleotide units III, wherein in the first nucleotide unit III, Y3 is the antisense strand of the siRNA corresponding to the target gene A and Y4 is the sense strand of the siRNA corresponding to the target gene B, and the target gene A and the target gene B are the same or different; and the sequence of the antisense strand of the siRNA corresponding to the target gene A in the first nucleotide unit III is complementary or at least partially reverse complementary to the sequence of the sense strand of the siRNA corresponding to the target gene A in the second nucleotide unit III. Optionally, the target gene A and the target gene B are different target genes.

**Composition**

In the second aspect, the present disclosure provides a composition, comprising the nucleic acid chimera according to the present disclosure and a physiologically acceptable excipient.
In the third aspect, the present disclosure provides the nucleic acid chimera for use in treating a disease or condition caused by expression dysregulation of at least one gene.
Preferably, the present disclosure provides the nucleic acid chimera for use in simultaneously treating a disease or condition caused by expression dysregulation of two genes.

Use

In the fourth aspect, the present disclosure provides use of the nucleic acid chimera in preparation of a medicament for treating a disease or condition caused by expression dysregulation of at least one gene.
Preferably, the present disclosure provides use of the nucleic acid chimera in the preparation of a medicament for treating a disease or condition caused by expression dysregulation of two genes.

**Method**

In the fifth aspect, the present disclosure provides a method for treating a disease or condition, comprising administering the nucleic acid chimera according to the present disclosure to an individual in need thereof.
In some embodiments, the nucleic acid chimera according to the present disclosure is administered to the individual subcutaneously or intravenously.
In some embodiments, after *in vivo* administration, the nucleic acid chimera according to the present disclosure is cleaved to yield at least two independent double-stranded oligonucleotide molecules, each of which targets a portion of mRNA transcribed from one or more target genes, wherein the target genes may be the same or different.
Unless otherwise specified, all siRNA sequences used in the Examples of the present disclosure were synthesized by Suzhou Biosyntech Co., Ltd.; all PCR primers were synthesized by Tsingke Biotechnology (Beijing) Co., Ltd.; and experimental animals, C57BL/6J mice, were purchased from SPF (Beijing) Biotechnology Co., Ltd.
Unless otherwise specified, the real-time PCR detection data from *in vitro* and *in vivo* siRNA activity experiments in each example of the present disclosure were caculated using the ΔΔCt method for relative quantification of target gene mRNA in each test group. The calculation method is summarized as follows:

$$\Delta Ct\ (\text{test group}) = Ct\ (\text{target gene in test group}) - Ct\ (\text{reference gene in test group})$$

$$\Delta Ct\ (\text{control group}) = Ct\ (\text{target gene in control group}) - Ct\ (\text{reference gene in control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

The mRNA expression level of the target gene in the test group was normalized based on the control group as a reference, and the remaining mRNA expression level of the target gene in the control group was defined as 100%.

$$\text{Relative remaining mRNA expression level of target gene in test group} = 2^{-\Delta\Delta Ct}\ (\text{test group}) \times 100\%$$

Inhibition rate of target gene mRNA in test group = 100% - relative mRNA expression level of target gene in test group

Unless otherwise specified, the reagent ratios described in each example of the present disclosure were calculated by volume (v/v); and experimental activity data were expressed as $\overline{X} \pm$STDEV, and all experimental data were graphed and analyzed using GraphPad Prism 8.0 software.
Unless otherwise specified, all reagents used in each example of the present disclosure were purchased from Beijing Ouhe Technology Co., Ltd., and the information of the main reagents is shown in Table 1.

Table 1 Reagent information

| Reagent Name | Abbreviation | CAS No. |
|---|---|---|
| Lithium aluminum hydride ($LiAlH_4$) | - | 16853-85-3 |
| Wet palladium on carbon (10 wt% loading) | Pd/C | - |
| Palladium hydroxide on carbon (10 wt% loading) | $Pd(OH)_2/C$ | - |
| O-Benzotriazol-1-yl-N,N,N',N'-tetramethyluronium hexafluorophosphate | HBTU | 94790-37-1 |
| 2-(7-Azabenzotriazol-1-yl)-N,N,N',N'-tetramethyluronium hexafluorophosphate | HATU | 148893-10-1 |
| 4,4'-Dimethoxytrityl chloride/4,4'-dimethoxytriphenylmethyl chloride | DMTrCl | 40615-36-9 |
| Bis(diisopropylamino)(2-cyanoethoxy)phosphine | - | 102691-36-1 |
| 4,5-Dicyanoimidazole | DCI | 1122-28-7 |
| 4-Dimethylaminopyridine | DMAP | 1122-58-3 |
| Amino CPG (Aminoalkyl-CPG, model No.: C3006-1000) | 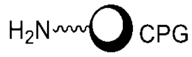 | |
| 4 M Hydrochloric acid in 1,4-dioxane | - | - |
| trans-4-(Boc-amino)cyclohexanecarbaldehyde | - | 181308-57-6 |
| N-Benzyloxycarbonyl-4-aminobutyric acid | - | 5105-78-2 |
| 5-[[(2R,3R,4R,5R,6R)-3-Acetamido-4,5-diacetyloxy-6- | Compound 4 | 1159408-54- |
| (acetoxymethyl)-2-tetrahydropyranyl]oxy]pentanoic acid | | 4 |
| CPG represents a controlled pore glass carrier. | | |

Preparation Example 1 Preparation of Compound CR01008

The synthetic route of Compound CR01008 is as follows:

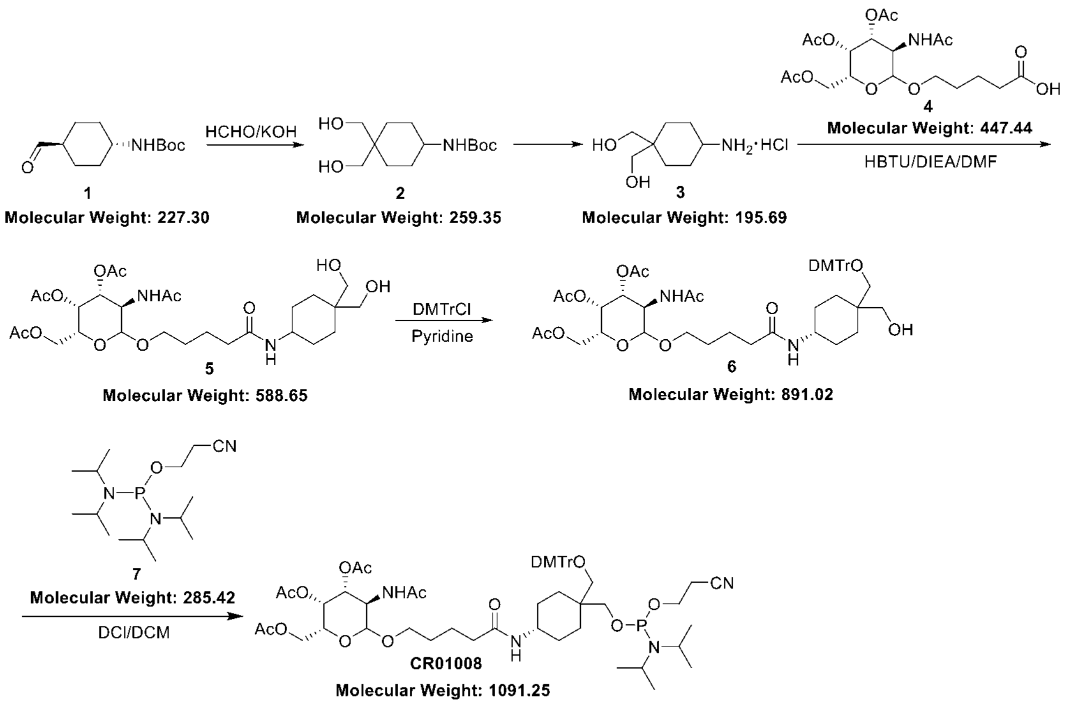


1 Molecular Weight: 227.30
2 Molecular Weight: 259.35
3 Molecular Weight: 195.69
4 Molecular Weight: 447.44
5 Molecular Weight: 588.65
6 Molecular Weight: 891.02
7 Molecular Weight: 285.42
CR01008 Molecular Weight: 1091.25

(1-1) Synthesis of Compound 2

Compound 1 (trans-4-(Boc-amino)cyclohexanecarbaldehyde, 10.0 g, 1.0 eq) and aqueous formaldehyde solution (8.9 g, 37 wt%, 2.4 eq) were dissolved in 33 mL of methanol, and added dropwise with 13 mL of 45.3 wt% aqueous KOH solution; after the completion of dropwise addition, the reaction mixture was stirred at 25°C for 30 minutes, then heated up to 60°C and refluxed at 60°C for 2 hours. After the reaction was completed, the reaction solution was cooled to room temperature and then evaporated to dryness under reduced pressure to afford a crude product as a white solid. A small amount of water was added to the crude product for trituration, followed by filtration to give Compound 2 as a white solid (9 g, yield: 78.9%). MS-ESI *(m/z)* = 260 $[M + H]^+$

(1-2) Synthesis of Compound 3

Compound 2 (9 g, 1 eq) prepared in step (1-1) was dissolved in 70 mL of 1,4-dioxane, a solution of 4 M hydrochloric acid in 1,4-dioxane (45 mL) was added, and the reaction mixture was stirred at 25°C for 1 hour. After the reaction was completed, the reaction solution was evaporated to dryness under reduced pressure to give Compound 3 as a white solid (6.8 g, yield: 100%).

(1-3) Synthesis of Compound 5

Compound 3 (1.8 g, 2.0 eq) prepared according to step (1-2), Compound 4 (5-[[(2R,3R,4R,5R,6R)-3-acetamido-4,5-diacetyloxy-6-(acetoxymethyl)-2-tetrahydropyranyl]oxy]pentanoic acid, 2.1 g, 1.0 eq) and N,N-diisopropylethylamine (3.5 g, 6.0 eq, abbreviated as DIEA) were dissolved in N,N-dimethylformamide (15 mL, abbreviated as DMF, CAS No.: 68-12-2), HBTU (1.9 g, 1.1 eq) was added, and the reaction mixture was stirred at 25°C for 3 hours under a $N_2$ atmosphere. After completion of the reaction, the reaction solution was evaporated to dryness under reduced pressure, and purified by reverse-phase purification (22 vol% acetonitrile in water) to afford Compound 5 as a white solid (1.78 g, yield: 64.4%). MS-ESI (m/z) = 589$[M + H]^+$.

(1-4) Synthesis of Compound 6

Compound 5 (1.54 g, 1.0 eq) was dissolved in 15 mL of pyridine; the reaction system was cooled to 0°C in an ice-water bath, and 4,4'-dimethoxytrityl chloride (1.32 g, 1.5 eq, DMTrCl, CAS No.: 40615-36-9) was added at 0°C. The reaction was carried out at 25°C for 3 hours, and 15 mL of methanol was added to the reaction solution to quench the reaction. After the reaction was completed, the reaction solution was evaporated to dryness under reduced pressure and purified by reverse-phase purification (60 vol% acetonitrile in water) to afford Compound 6 as a yellow solid (1 g, yield: 42.7%). MS-ESI (m/z) = 891 $[M + H]^+$.

(1-5) Synthesis of Compound CR01008

Compound 6 (1.08 g, 1.0 eq) was dissolved in 20 mL of anhydrous dichloromethane, and DCI (115 mg, 0.8 eq) and Compound 7 (bis(diisopropylamino)(2-cyanoethoxy)phosphine, 732 mg, 2.1 eq) were added, respectively. The reaction system was purged with nitrogen three times, and the reaction mixture was stirred at 25°C for 2 hours. After the reaction was completed, 20 mL of saturated aqueous sodium bicarbonate solution was added to the reaction solution. The mixture was extracted with 20 mL of dichloromethane three times (3 × 20 mL). The organic phases were combined, evaporated to dryness under reduced pressure, subjected to reverse-phase purification (72 vol% acetonitrile in water), and then vacuum-dried for 12 hours to give Compound CR01008 as a white powder (1 g, yield: 76.0%). MS-ESI (m/z) = 1091 $[M + Na]^+$.

$^1$H NMR (400 MHz, DMSO-$d_6$) δ 1.05 (d, *J* = 6.7 Hz, 6H).1.14 (d, *J* = 6.7 Hz, 6H), 1.37 - 1.17 (m, 5H), 1.60 - 1.40 (m, 6H), 1.68 - 1.62 (m, 1H), 1.80 (s, 3H), 1.80 (s, 3H), 1.92 (s, 3H), 2.02 (s, 5H), 2.13 (s, 3H), 2.71 (t, *J* = 5.9 Hz, 2H), 2.79 (d, *J* = 8.4 Hz, 1H), 2.87 (d, *J* = 8.4 Hz, 1H), 3.36 (s, 1H), 3.58 - 3.39 (m, 3H), 3.69 - 3.60 (m, 2H), 3.75 (s, 7H), 3.90 (dt, *J* = 11.2, 8.8 Hz, 1H), 4.05 (s, 3H),4.51 (d, *J* = 8.4 Hz, 1H), 4.99 (dd, *J* = 11.3, 3.4 Hz, 1H), 5.24 (d, *J* = 3.4 Hz, 1H), 5.78 (s, 1H), 6.93 - 6.87 (m, 4H),7.35 - 7.21 (m, 7H), 7.44 - 7.37 (m, 2H), 7.66 (*d*, *J* = 7.8 Hz, 1H), 7.84 (d, *J* = 9.2 Hz, 1H).

**Preparation Example 2 Preparation of Compound CR01008Z**

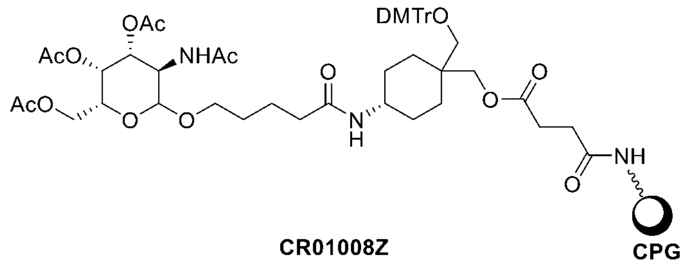

CR01008Z

The synthetic route of Compound CR01008Z is as follows:

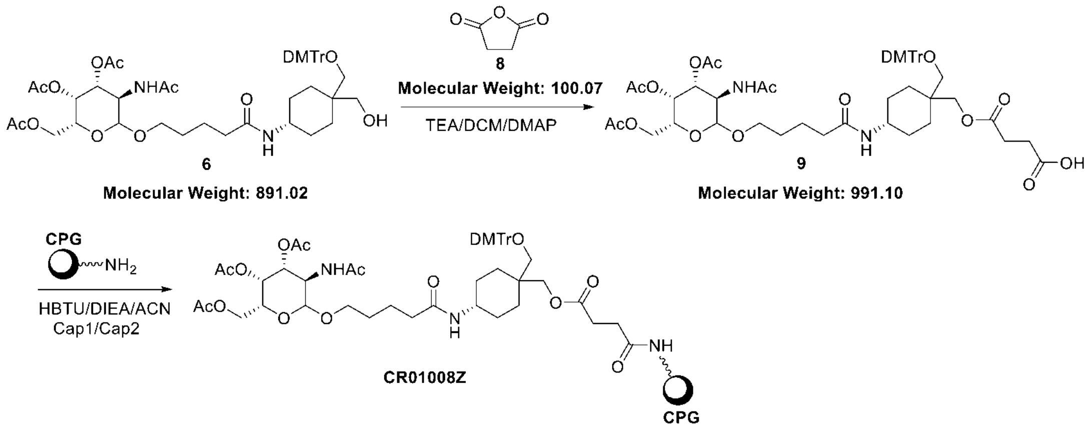


(2-1) Synthesis of Compound 9

Compound 6 (500 mg) prepared according to step (1-4) was dissolved in 10 mL of dichloromethane.

Compound 8 (succinic anhydride, 112 mg), DMAP (6.8 mg), and triethylamine (226.2 mg, abbreviated as TEA or $Et_3N$) were added. The reaction system was purged with nitrogen three times, and the reaction mixture was stirred at 25°C for 16 hours, followed by flash purification to give Compound 9 (300 mg, yield: 53.6%). MS-ESI (m/z) = 1013 $[M + Na]^+$.

(2-2) Synthesis of Compound CR01008Z

Compound 9 (50 mg) prepared according to step (1.6), amino CPG (1.25 g, 80 μmol/g, 0.1 mmol), HBTU (27 mg), and DIEA (12 mg) were added to a 20 mL sample vial, and the reaction was carried out on a shaker for 16 hours. After the reaction was completed, the reaction solution was filtered to give a filter cake. The filter cake was first washed with 10 mL of acetonitrile once (1 × 10 mL), followed by vacuum drying. The dried filter cake, DMAP (3 mg), Cap1 (10 mL, 200 V), and Cap2 (1 mL, 20 V) were added to a 20 mL sample vial, and the reaction was carried out on a shaker for 6 hours. After the reaction was completed, the reaction solution was filtered to give a filter cake. The filter cake was first washed with 10 mL of acetonitrile once (1 × 10 mL), followed by vacuum drying to give Compound CR01008Z (1.03 g, with a loading of 20-30 μmol/g).

Cap1 and Cap2 are capping reagents, wherein Cap1 is a 20 vol% N-methylimidazole solution in a pyridine/acetonitrile mixture (volume ratio of pyridine to acetonitrile: 3:5); and Cap2 is a 20 vol% acetic anhydride solution in acetonitrile.

**Preparation Example 3 Preparation of Compound NM090**

The synthetic route of Compound NM090 is shown as follows:

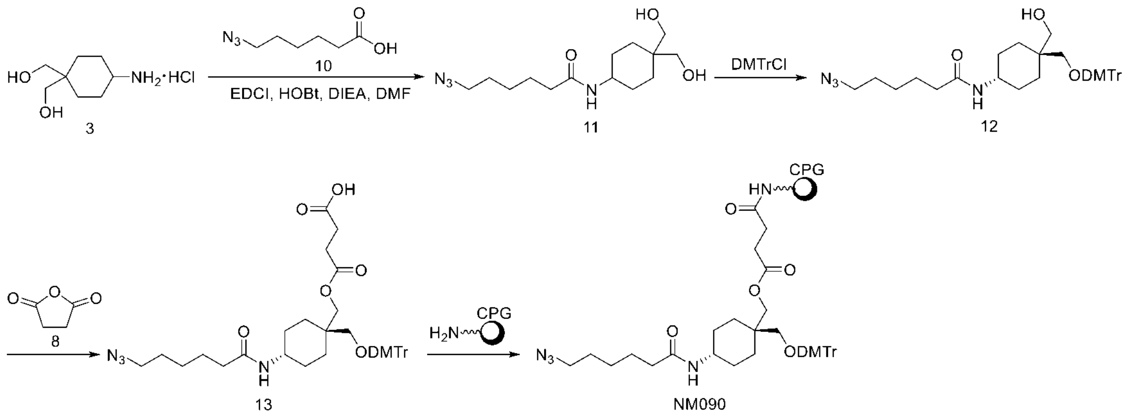


(3-1) Synthesis of Compound 11

Compound 3 (3 g, 15.3 mmol, 1.0 eq) and Compound 10 (2.4 g, 15.2 mmol, 1.0 eq, 6-azidohexanoic acid, CAS No.: 79598-53-1), 1-ethyl-3-(3-dimethylaminopropyl)carbodiimide hydrochloride (4.38 g, 22.95 mmol, 1.5 eq, abbreviated as EDCI, CAS No.: 25952-53-8), and 1-hydroxybenzotriazole (3.15 g, 22.95 mmol, 1.5 eq, abbreviated as HOBt, CAS No.: 2592-95-2) were dissolved in DMF (150 mL). DIEA (6 g, 3.0 eq) was added in an ice bath, the mixture was stirred at room temperature for 2-3 hours, and the reaction was completed. The reaction solution was added with water (100 mL), and extracted with ethyl acetate twice (100 mL each time). The organic phase was separated and combined, dried, concentrated, and then subjected to reverse-phase purification (eluent: acetonitrile/water = 30/70, v/v) to give Compound 11 as a white solid (3 g, yield: 75%). MS ESI (m/z) = 299 $[M + H]^+$.

(3-2) Synthesis of Compound 12

Compound 11 (3 g, 11.19 mmol, 1.0 eq) was dissolved in pyridine (30 mL). DMTrCl (3.7 g, 11.19 mmol, 1.0 eq) was added under an ice bath, and the reaction system was purged with nitrogen 3 times. The mixture was stirred at room temperature for 3 hours and quenched with methanol (50 mL). After the reaction was completed, the reaction solution was concentrated, added with water (50 mL), and extracted with ethyl acetate 3 times (50 mL each time). The organic phase was separated and combined, dried, concentrated, and then subjected to reverse-phase purification (eluent: acetonitrile/-water = 60/40, v/v) to give Compound 12 as a pale yellow solid (1.9 g, yield: 26%). MS ESI (m/z) = 601 $[M + H]^+$.

(3-3) Synthesis of Compound 13

Compound 12 (200 mg, 0.33 mmol, 1 eq) was dissolved in anhydrous dichloromethane (2 mL). Succinic anhydride (67 mg, 0.66 mmol, 2 eq) and TEA (100 mg, 1.0 mmol, 3 eq) were added, respectively. The reaction system was purged with nitrogen 3 times, and the mixture was stirred at room temperature for 12 hours. After the reaction was completed, the reaction solution was first diluted with dichloromethane (10 mL), and then added with water (10 mL) for extraction. The organic phase was separated, dried, concentrated, and then subjected to reverse-phase purification (eluent: acetonitrile/water = 31/69, v/v) to give Compound 13 as a white powder (160 mg, yield: 69.5%). MS ESI (m/z) = 701 $[M + H]^+$.

(3-4) Synthesis of Compound NM090

Compound 13 (39 mg, 0.056 mmol, 1 eq) was dissolved in anhydrous acetonitrile (2 mL). HBTU (32 mg, 1.5 eq), DIEA (15 mg, 2 eq), and amino CPG (1.43 g, 2 eq) were added, respectively. The reaction system was purged with nitrogen 3 times. The mixture was reacted on a shaker at room temperature for 12 hours, and filtered. The filter cake was rinsed with acetonitrile 3 times (10 mL each time), and dried. Acetonitrile (10 mL) was first added, followed by Cap1 (8 mL) and Cap2 (1 mL). The mixture was reacted on a shaker for 5 hours, and filtered. The filter cake was rinsed with acetonitrile 3 times (10 mL each time) and vacuum-dried for 12 hours to give Compound NM090 as a white solid (1 g, determined loading: 40-60 μmol/g).

**Preparation Example 4 Preparation of Compound NM091**

The synthetic route of Compound NM091 is shown as follows:

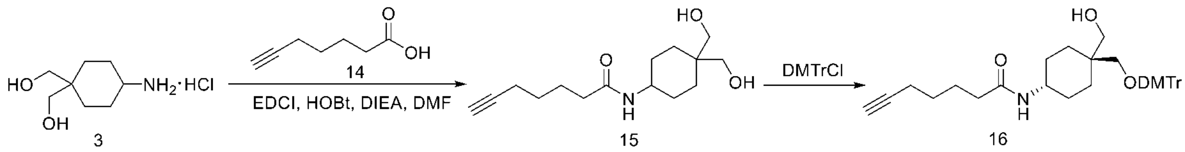


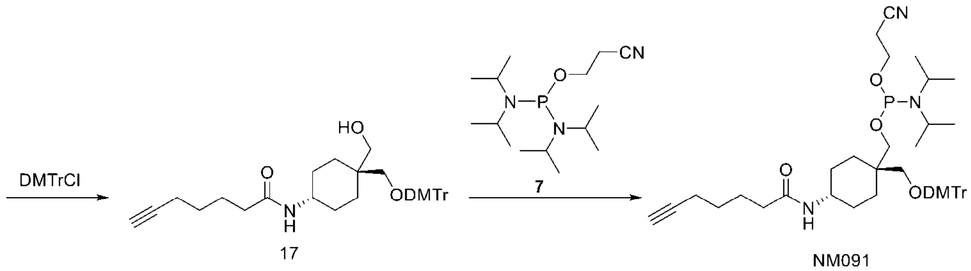


(4-1) Synthesis of Compound 15

Compound 3 (3 g, 15.3 mmol, 1.0 eq), Compound 14 (1.92 g, 15.2 mmol, 1.0 eq, CAS No.: 30964-00-2), EDCI (4.38 g, 22.95 mmol, 1.5 eq), and HOBt (3.15 g, 22.95 mmol, 1.5 eq) were dissolved in DMF (150 mL). DIEA (6 g, 3.0 eq) was added in an ice bath, and the mixture was stirred at room temperature for 2-3 hours. After the reaction was completed, the reaction solution was added with water (100 mL), and extracted with ethyl acetate twice (100 mL each time). The organic phase was dried, concentrated, and then subjected to reverse-phase purification (eluent: acetonitrile/water = 30/70, v/v) to give Compound 15 as a white solid (3 g, yield: 75%). MS ESI (m/z) = 268 $[M + H]^+$.

(4-2) Synthesis of Compound 16

Compound 15 (3 g, 11.19 mmol, 1.0 eq) was dissolved in pyridine (30 mL). DMTrCl (3.7 g, 11.19 mmol, 1.0 eq) was added in an ice bath. The reaction system was purged with nitrogen 3 times, and the mixture was stirred at room temperature for 3 hours and quenched with methanol (50 mL). After the reaction was completed, the reaction solution was concentrated, added with water (50 mL), and then extracted with ethyl acetate 3 times (50 mL each time). The organic phase was separated and combined, dried, concentrated, and then subjected to reverse-phase purification (eluent: acetonitrile/water = 60/40, v/v) to give Compound 16 as a pale yellow solid (1.6 g, yield: 26%). MS ESI (m/z) = 569 $[M + H]^+$.

(4-3) Synthesis of Compound 17

Compound 16 (1.6 g, 2.8 mmol, 1.5 eq) was dissolved in anhydrous dichloromethane (20 mL). DCI (250 mg, 2.24 mmol, 0.8 eq) and Compound 7 (875 mg, 3.1 mmol, 1.1 eq) were added respectively. The reaction system was purged with nitrogen 3 times, and the mixture was stirred at room temperature for 2 hours. After the reaction was completed, the reaction solution was added with a saturated aqueous sodium bicarbonate solution (20 mL), and extracted with dichloromethane 3 times (20 mL each time). The organic phase was separated and combined, dried, concentrated, and then subjected to reverse-phase purification (eluent: acetonitrile/water = 91/9, v/v) to give Compound NM091 as a white powder (1 g, yield: 46.3%). MS ESI (m/z) = 770 $[M + H]^+$.

$^1H$ NMR (400 MHz, DMSO-d6) δ 7.67 (d, J = 7.8 Hz, 1H), 7.43 - 7.36 (m, 2H), 7.35 - 7.17 (m, 8H), 6.88 (dd, J = 15.5, 8.5 Hz, 4H), 3.75 (s, 6H), 3.72 (s, 1H), 3.70 - 3.61 (m, 2H), 3.51 (dq, J = 16.9, 6.8 Hz, 2H), 2.90 - 2.67 (m, 6H), 2.16 (td, J = 7.1, 2.7 Hz, 2H), 2.05 (t, J = 7.3 Hz, 2H), 1.58 (tt, J = 21.1, 11.4 Hz, 6H), 1.46 - 1.37 (m, 2H), 1.35 - 1.19 (m, 4H), 1.13 (d, *J* = 6.7 Hz, 6H), 1.05 (d, J = 6.7 Hz, 6H).

**Preparation Example 5 Preparation of siRNAs and siRNA Chimeras**

(5-1) Synthesis of Sense Strand (SS)

Through the phosphoramidite solid-phase nucleic acid synthesis method, Compound CR01008Z was used for the initial cycle, and nucleoside monomers were sequentially coupled cycle by cycle according to the nucleotide sequence in the 3'-to-5' direction. During the synthesis, Compound CR01008 was treated as one nucleoside monomer.

Each incoporation of the nucleoside monomers comprises four reaction steps: deprotection, coupling, capping, and oxidation or sulfuration. The synthesis conditions are specified as follows:
The nucleoside monomer was prepared as a 0.1 M solution in acetonitrile.

The deprotection reaction conditions were the same for each cycle. The deprotection reaction conditions were as

follows: the temperature was 25°C, the reaction time was 70 seconds, and the deprotection reagent was a solution of dichloroacetic acid in dichloromethane (3 vol%), wherein the molar ratio of dichloroacetic acid to the 4,4'-dimethoxytrityl protecting group on the solid-phase carrier was 5:1.

The coupling reaction conditions were the same for each cycle. The coupling reaction conditions were as follows: the temperature was 25°C, the molar ratio of the nucleic acid sequence attached to the solid-phase carrier to the nucleoside monomer was 1:10, the molar ratio of the nucleic acid sequence attached to the solid-phase carrier to the coupling reagent was 1:65, and the reaction time was 600 seconds; the coupling reagent was a 0.5 M solution of 5-ethylthio-1H-tetrazole in acetonitrile, and the sulfurizing reagent was a 0.2 M solution of xanthane hydride in an acetonitrile/pyridine mixture (the volume ratio of acetonitrile to pyridine was 1:1).

The capping reaction conditions were the same for each cycle. The capping reaction conditions were as follows: the temperature was 25°C; the reaction time was 2 minutes; the capping reagent solution was a mixed solution of Cap1 and Cap2 with a molar ratio of 1:1, wherein Cap1 was a solution of N-methylimidazole in a pyridine/acetonitrile mixture, with a concentration of 20 vol%, the volume ratio of pyridine to acetonitrile was 3:5; and Cap2 was a solution of acetic anhydride in acetonitrile with a concentration of 20 vol%; and the molar ratio of N-methylimidazole in the capping reagent Cap1, acetic anhydride in the capping reagent Cap2, and the nucleic acid sequence attached to the solid-phase carrier was 1:1:1.

The oxidation reaction conditions were the same for each cycle. The oxidation reaction conditions were as follows: the temperature was 25°C; the reaction time was 3 seconds; the oxidizing reagent was a 0.05 M aqueous iodine solution, and the molar ratio of iodine to the nucleic acid sequence attached to the solid-phase carrier in the incoporation reaction was 30:1; and the oxidation reaction was carried out in a water/pyridine mixed solvent (the volume ratio of water to pyridine was 1:9). The sulfuration reaction conditions are as follows: the temperature was 25°C; the reaction time was 360 seconds; the sulfurizing reagent was a 0.2 M solution of xanthane hydride in pyridine, and the molar ratio of the sulfurizing reagent to the nucleic acid sequence attached to the solid-phase carrier in the incoporation reaction was 4:1; and the sulfuration reaction was carried out in a water/pyridine mixed solvent (the volume ratio of water to pyridine was 1:9).

After the incoporation of the last nucleoside monomer was completed, the nucleic acid sequence attached to the solid-phase carrier was sequentially subjected to cleavage, deprotection, purification, and desalting, followed by lyophilization to give the sense strand, in which:

Cleavage and deprotection conditions: The synthesized nucleotide sequence attached to the solid-phase carrier was added to aqueous ammonia with a concentration of 25 wt%, and the amount of aqueous ammonia was 0.5 mL/μmol; the reaction was conducted at 55°C for 16 hours, then the solvent was removed, and the mixture was concentrated to dryness under vacuum. Following the ammonia treatment, the product was dissolved using 0.4 mL/μmol N-methylpyrrolidone relative to the amount of the single-stranded nucleic acid. Subsequently, 0.3 mL/μmol triethylamine and 0.6 mL/μmol triethylamine trihydrofluoride were added to remove the 2'-O-TBDMS protecting group on the ribose.

Purification and desalting conditions: Purification of the nucleic acid was performed using a preparative ion chromatography column (Source 15Q) with NaCl gradient elution. Specifically, Eluent 1 was 20 mM sodium phosphate (pH=8.1) in a water/acetonitrile mixed solvent (the volume ratio of water to acetonitrile was 9:1); Eluent 2 was 1.5 M sodium chloride and 20 mM sodium phosphate (pH = 8.1) in a water/acetonitrile mixed solvent (the volume ratio of water to acetonitrile was 9:1); and the eluent was Eluent 1:Eluent 2 = (100:0)-(50:50). The product eluates were collected and combined. Desalting was performed using a reverse-phase chromatography column. The desalting conditions included the following: desalting was performed using a dextran gel column with the packing material of dextran gel G25, and elution was performed with deionized water.

Detection: Purity was detected using ion exchange chromatography (IEX-HPLC); and molecular weight analysis was performed using a liquid chromatography-mass spectrometry instrument (LC-MS, purchased from Waters Corporation, model No.: LCT Premier). The measured value of the molecular weight was compared with the theoretical value. If the measured value was consistent with the theoretical value, it indicates that the sense strands as shown in Table 2 were obtained.

Table 2 Sequence information and detection results of the sense strands

| Sense strand No. | Sequence and carrier information of the sense strand (5'-3') | Theoretical molecular weight | Actual molecular weight | Purity |
|---|---|---|---|---|
| RZ899058-SS SEQ ID NO.12 | UmsUmsUmUmAmAmUfCfCfUfCmAmCmUmCmUmAmAmAm(CR01008)(CR01008)(CR01008) | 7711.7 | 7713.7 | 94.6% |

(continued)

| Sense strand No. | Sequence and carrier information of the sense strand (5'-3') | Theoretical molecular weight | Actual molecular weight | Purity |
|---|---|---|---|---|
| RZ597104-SS SEQ ID NO.13 | CmsCmsAmAmGmAmGfCfAfCfCmAmAmGmAmAmCmUmAm(CR01008)(CR01008)(CR01008) | 7896.9 | 7898.8 | 92.9% |
| RZ597104-SS2 SEQ ID NO.14 | (CR01008)s(CR01008)s(CR01008)sCmsCmsAmAmGmAmGfCfAfCfCmAmAmGmAmAmCmsUmsAm | 7978.1 | 7977.6 | 98.2% |

Table 3 Unmodified nucleotide sequence information corresponding to the sense strands

| siRNA No. | Naked sequence information of the sense strand (5'-3') | No. |
|---|---|---|
| RZ899058-SS | UUUUAAUCCUCACUCUAAA | SEQ ID NO.1 |
| RZ597104-SS | CCAAGAGCACCAAGAACUA | SEQ ID NO.2 |
| RZ597104-SS2 | CCAAGAGCACCAAGAACUA | SEQ ID NO.2 |

(5-2) Synthesis of Antisense Strand AS

The antisense strand was synthesized through the phosphoramidite solid-phase nucleic acid synthesis method. Using a solid-phase carrier (CPG carrier or PS carrier) for the initial cycle, nucleoside monomers were sequentially coupled cycle by cycle according to the nucleotide sequence in the 3'-to-5' direction. Each incoporation of the nucleoside monomers comprises four reaction steps: deprotection, coupling, capping, and oxidation or sulfuration; the reaction conditions were the same as those for the synthesis of the sense strands in step (5-1).

Detection: Purity was detected using ion exchange chromatography (IEX-HPLC); and molecular weight analysis was performed using a liquid chromatography-mass spectrometry instrument (LC-MS). The measured value of the molecular weight was compared with the theoretical value. If the measured value was consistent with the theoretical value, it indicates that the antisense strands as shown in Table 4 were obtained.

Table 4 Sequence information and detection results of the antisense strands

| Antisense strand No. | Sequence and carrier information of the antisense strands (5'-3') | Theoretical molecular weight | Actual molecular weight | Purity |
|---|---|---|---|---|
| RZ899058-AS SEQ ID NO.15 | VPUmsUfsUmAmGmAfGmUmGfAmGmGmAmUfU(moe)AfAmAmAmsUmsGm | 7204.8 | 7205.2 | 91.5% |
| RZ597104-AS SEQ ID NO.16 | VPUmsAfsGmUmUmCfUmUmGfGmUmGmCmUfC(moe)UfUmGmGmsUmsUm | 7040.4 | 7041.3 | 91.9% |
| RZ899058-AS2 SEQ ID NO.17 | VPUmsUfsUmAmGmAfGmUmGfAmGmGmAmUfU(moe)AfAmAmsAmsUms(CR01008)s(CR01008)s(CR01008) | 8467.3 | 8466.6 | 98.9% |

Table 5 Unmodified nucleotide sequence information corresponding to the antisense strands

| Antisense strand No. | Naked sequence information of the antisense strand (5'-3') | No. |
|---|---|---|
| RZ899058-AS | UUUAGAGUGAGGAUUAAAAUG | SEQ ID NO.3 |

(continued)

| Antisense strand No. | Naked sequence information of the antisense strand (5'-3') | No. |
|---|---|---|
| RZ597104-AS | UAGUUCUUGGUGCUCUUGGUU | SEQ ID NO.4 |
| RZ899058-AS2 | UUUAGAGUGAGGAUUAAAAU | SEQ ID NO.5 |

(5-3) Synthesis of Head-to-Tail Single Strands

The head-to-tail single strand comprises two subsequences, which are respectively named as Target 1 and Target 2, wherein Target 1 and Target 2 are each independently selected from a sense strand and an antisense strand, and the 3' terminus of Target 1 is conjugated to the 5' terminus of Target 2. For example, both Target 1 and Target 2 are selected from the sense strands, or Target 1 is selected from the antisense strand and Target 2 is selected from the sense strand, or Target 1 is selected from the sense strand and Target 2 is selected from the antisense strand.

The head-to-tail single strand was synthesized through the phosphoramidite solid-phase nucleic acid synthesis method. Using a solid-phase carrier (CPG carrier, PS carrier, or compound CR01008Z) for the initial cycle, nucleoside monomers were sequentially coupled cycle by cycle according to the nucleotide sequence in the 3'-to-5'direction. During the synthesis, Compound CR01008 was treated as one nucleoside monomer.

Each incoporation of nucleoside monomers comprises four reaction steps: deprotection, coupling, capping, and oxidation or sulfuration; the reaction conditions were the same as those for the synthesis of the sense strand in step (5-1).

Detection: Purity was detected using ion exchange chromatography (IEX-HPLC); and molecular weight analysis was performed using a liquid chromatography-mass spectrometry instrument (LC-MS). The measured value of the molecular weight was compared with the theoretical value. If the measured value was consistent with the theoretical value, it indicates that the head-to-tail single strand formed by conjugation of the 3' terminus of the sense strand and the 5' terminus of the sense strand as shown in Table 6, and the head-to-tail single strand formed by conjugation of the 3' terminus of the antisense strand and the 5' terminus of the sense strand as shown in Table 8, were obtained.

Table 6 Sequence information and analysis results of the head-to-tail single strands formed by conjugation of the 3' terminus of the sense strand and the 5' terminus of the sense strand

| Head-to-tail single strand No. | Sequence and carrier information (5'-3') | Theoretical molecular weight | Actual molecular weight | Purity |
|---|---|---|---|---|
| RZ899/597-M1-SS SEQ ID NO.18 | UmsUmsUmUmAmAmUfCfCfUfCmAmCmUmCmUmAmAmAm(CR01008)(CR01008)(CR01008)CmCmAmAmGmAmGfCfAfCfCmAmAmGmAmAmCmUmAm | 14065.9 | 14068.0 | 91.3% |
| RZ899/597-M2-SS SEQ ID NO.19 | UmsUmsUmUmAmAmUfCfCfUfCmAmCmUmCmUmAmAmAm(CR01008)(CR01008)(CR01008)Cm | 15638.5 | 15641.3 | 91.0% |
| | CmAmAmGmAmGfCfAfCfCmAmAmGmAmAmCmUmAm(CR01008)(CR01008)(CR01008) | | | |
| RZ899/597-M3-SS SEQ ID NO.20 | (CR01008)UmsUmsUmUmAmAmUfCfCfUfCmAmCmUmCmUmAmAmAm(CR01008)CmCmAmAmGmAmGfCfAfCfCmAmAmGmAmAmCmUmAm(CR01008) | 14065.9 | 14068.0 | 91.8% |

(continued)

| Head-to-tail single strand No. | Sequence and carrier information (5'-3') | Theoretical molecular weight | Actual molecular weight | Purity |
|---|---|---|---|---|
| RZ899/597-M4-SS SEQ ID NO.21 | (CR01008)(CR01008)UmsUmsUmUmAmAmUfCfCfUfCmAmCmUmCmUmAmAmAm(CR01008)(CR01008)CmCmAmAmGmAmGfCfAfCfCmAmAmGmAmAmCmUmAm(CR01008)(CR01008) | 15638.5 | 15642.1 | 93.7% |

Wherein, the structural formula of the head-to-tail single strand formed by conjugation of the 3' terminus of the sense strand and the 5' terminus of the sense strand is as follows:

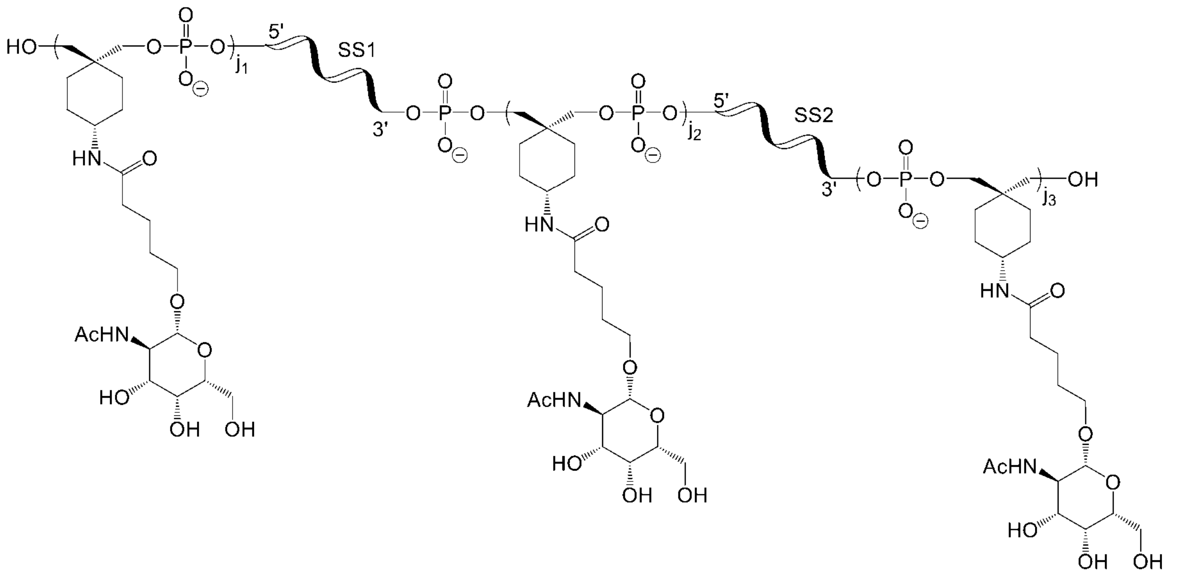

;

wherein,

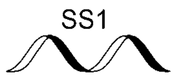

represents the sense strand (Target 1), and

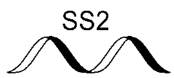

represents the sense strand (Target 2).

In RZ899/597-M1-SS, $j_1$=0, $j_2$=3, and $j_3$=0.

In RZ899/597-M2-SS, $j_1$=0, $j_2$=3, and $j_3$=3.

In RZ899/597-M3-SS, $j_1$=1, $j_2$=1, and $j_3$=1.

In RZ899/597-M4-SS, $j_1$=2, $j_2$=2, and $j_3$=2.

The naked sequence information corresponding to the head-to-tail sense strand formed by conjugation of the 3' terminus of the sense strand and the 5' terminus of the sense strand in Table 6 is shown in Table 7.

Table 7 Unmodified nucleotide sequence information of the head-to-tail single strands formed by conjugation of the 3' terminus of the sense strand and the 5' terminus of the sense strand

| Head-to-tail single strand No. | Naked sequence information of the sense strand (Target 1) (5'-3') | | Naked sequence information of the sense strand (Target 2) (5'-3') | |
|---|---|---|---|---|
| RZ899/597-M1-SS | UUUUAAUCCUCACUCUAAA | SEQ ID NO.1 | CCAAGAGCACCAAGAACUA | SEQ ID NO.2 |
| RZ899/597-M2-SS | UUUUAAUCCUCACUCUAAA | SEQ ID NO.1 | CCAAGAGCACCAAGAACUA | SEQ ID NO.2 |
| RZ899/597-M3-SS | UUUUAAUCCUCACUCUAAA | SEQ ID NO.1 | CCAAGAGCACCAAGAACUA | SEQ ID NO.2 |
| RZ899/597-M4-SS | UUUUAAUCCUCACUCUAAA | SEQ ID NO.1 | CCAAGAGCACCAAGAACUA | SEQ ID NO.2 |

Table 8 Sequence information and analysis results of the head-to-tail single strands formed by conjugation of the 3' terminus of the antisense strand and the 5' terminus of the sense strand

| Head-to-tail single strand No. | Sequence and carrier information (5'-3') | Theoretical molecular weight | Actual molecular weight | Purity |
|---|---|---|---|---|
| RZ597AS/899SS-M16<br>SEQ ID NO.22 | VPUmsAfsGmUmUmCfUmUmGfGmUmGmCmUfC(moe)UfUmGmGmsUmsUm(CR01008)(CR01008)(CR01008)UmsUmsUmUmAmAmUfCfCfUfCmAmCmUmCmUmAmAmAm | 14814.1 | 14816.7 | 95.96 |
| RZ597AS/899SS-M16.2<br>SEQ ID NO.23 | VPUmsAfsGmUmUmCfUmUmGfGmUmGmCmUfC(moe)UfUmGmGmsUmsUmTdTdTd(CR01008)(CR01008)(CR01008)UmsUmsUmUmAmAmUfCfCfUfCmAmCmUmCmUmAmAmAm | 15727 | 15728.7 | 94.99 |
| RZ597AS/899SS-M16.3<br>SEQ ID NO.24 | VPUmsAfsGmUmUmCfUmUmGfGmUmGmCmUfC(moe)UfUmGmGmsUmsUmUUU(CR01008)s(CR01008)s(CR01008)sUmsUmsUmUmAmAmUfCfCfUfCmAmCmUmCmUmAmsAmsAm | 15813.8 | 15813.1 | 93.0 |
| RZ597AS/899SS-M21<br>SEQ ID NO.25 | VPUmsAfsGmUmUmCfUmUmGfGmUmGmCmUfC(moe)UfUmGmGmsUmsUmTdTdTdUmsUmsUm | 15733 | 15735.8 | 95.23 |
| | UmAmAmUfCfCfUfCmAmCmUmCmUmAmAmAm(CR01008)(CR01008)(CR01008) | | | |
| RZ899AS/597SS-M21<br>SEQ ID NO.26 | VPUmsUfsUmAmGmAfGmUmGfAmGmGmAmUfU(moe)AfAmAmAmsUmsGmTdTdTdCmsCmsAmAmGmAmGfCfAfCfCmAmAmGmAmAmCmUmAm(CR01008)(CR01008)(CR01008) | 16082 | 16084.8 | 97.31 |

The structural formula of the head-to-tail single strand formed by conjugation of the 3' terminus of the antisense strand and the 5' terminus of the sense strand is as follows:

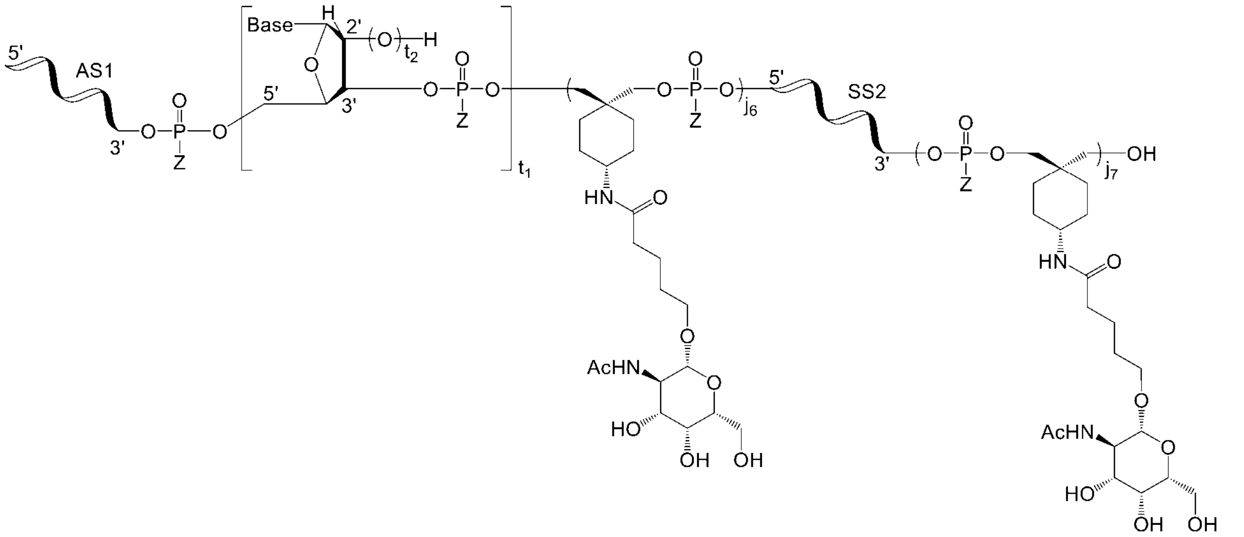

;,

wherein,

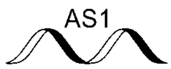

represents the antisense strand (Target 1), and

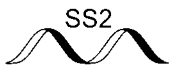

represents the sense strand (Target 2).

In RZ597AS/899SS-M16, $t_1$=0, $j_6$=3, and $j_7$=0.

In RZ597AS/899SS-M16.2, $t_1$=3, $t_2$=0, Base represents the nucleobase T, $j_6$=3, and $j_7$=0.

In RZ597AS/899SS-M16.3, $t_1$=3, $t_2$=1, Base represents the nucleobase U, $j_6$=3, and $j_7$=0.

In RZ597AS/899SS-M21, $t_1$=3, $t_2$=0, Base represents the nucleobase T, $j_6$=0, and $j_7$=3.

In RZ899AS/597SS-M21, $t_1$=3, $t_2$=0, Base represents the nucleobase T, $j_6$=0, and $j_7$=3.

The naked sequence information corresponding to the head-to-tail sense strand formed by conjugation of the 3' terminus of the antisense strand and the 5' terminus of the sense strand in Table 8 is shown in Table 9.

Table 9 Unmodified nucleotide sequence information of the head-to-tail single strands formed by conjugation of the 3' terminus of the antisense strand and the 5' terminus of the sense strand

| Head-to-tail single strand No. | Naked sequence information of the antisense strand (Target 1) (5'-3') | | Naked sequence information of the sense strand (Target 2) (5'-3') | |
|---|---|---|---|---|
| RZ597AS/899SS -M16 | UAGUUCUUGGUGCUCUUGG UU | SEQ ID NO.4 | UUUUAAUCCUCACUCUA AA | SEQ ID NO.1 |
| RZ597AS/899SS -M16.2 | UAGUUCUUGGUGCUCUUGG UU | SEQ ID NO.4 | UUUUAAUCCUCACUCUA AA | SEQ ID NO.1 |
| RZ597AS/899SS -M16.3 | UAGUUCUUGGUGCUCUUGG UU | SEQ ID NO.4 | UUUUAAUCCUCACUCUA AA | SEQ ID NO.1 |

(continued)

| Head-to-tail single strand No. | Naked sequence information of the antisense strand (Target 1) (5'-3') | | Naked sequence information of the sense strand (Target 2) (5'-3') | |
|---|---|---|---|---|
| RZ597AS/899SS -M21 | UAGUUCUUGGUGCUCUUGG UU | SEQ ID NO.4 | UUUUAAUCCUCACUCUA AA | SEQ ID NO.1 |
| RZ899AS/597SS -M21 | UUUAGAGUGAGGAUUAAAA UG | SEQ ID NO.3 | CCAAGAGCACCAAGAAC UA | SEQ ID NO.2 |

(5-4) Synthesis of Head-to-Head Single Strands

The head-to-head single strand comprises two subsequences, which were respectively named as Subsequence (the first target) and Subsequence (the second target), wherein Subsequence (the first target) and Subsequence (the second target) are each independently selected from the sense strand and the antisense strand, and the 3' terminus of Subsequence (the first target) is conjugated to the 3' terminus of Subsequence (the second target) via a click chemistry reaction.

Specifically, both Subsequence (the first target) and Subsequence (the second target) are selected from the sense strands, and the 3' terminus of the sense strand is conjugated to the 3' terminus of the sense strand via a click chemistry reaction.

(5-4-1) Synthesis of Subsequences

The subsequences were synthesized through the phosphoramidite solid-phase nucleic acid synthesis method. Using a solid-phase carrier (CPG carrier, PS carrier, or Compound CR01008Z) for the initial cycle, nucleoside monomers were sequentially coupled cycle by cycle according to the nucleotide sequence in the 3'-to-5' direction. In the synthesis process, Compound CR01008, Compound NM090, and Compound NM091 were each treated as one nucleoside monomer.

Each incoporation of nucleoside monomers comprises four reaction steps: deprotection, coupling, capping, and oxidation or sulfuration; the reaction conditions were the same as the conditions for the synthesis of the sense strand in step (5-1).

Detection: Purity was detected using ion exchange chromatography (IEX-HPLC); and molecular weight analysis was performed using a liquid chromatography-mass spectrometry instrument (LC-MS). The measured value of the molecular weight was compared with the theoretical value. If the measured value was consistent with the theoretical value, it indicates that the subsequences shown in Table 12 were obtained.

(5-4-2) Click Chemistry Reaction

The sense strand (the first target) was dissolved in 40 μL of $H_2O$ and 40 μL of carbonate buffer (pH=9.2, 0.2 M) to prepare a sense strand (the first target) solution at a concentration of 0.08 μmol; the sense strand (the second target) was dissolved in 80 μL of $H_2O$ to prepare a sense strand (the second target) solution at a concentration of 0.08 μmol; and, the sense strand (the first target) solution and the sense strand (the second target) solution were mixed and vortexed to obtain the first mixture.

Tris(3-hydroxypropyltriazolylmethyl)amine (100 μL, abbreviated as THPTA, CAS No.: 760952-88-3) and $CuSO_4 \cdot 5H_2O$ (20 μL) were mixed and shaken at 40°C for 5 min to obtain the second mixture.

9.6 μL of the second mixture was added to the first mixture, followed by vortexing; 3.2 μL of an aqueous solution of sodium ascorbate (CAS No.: 134-03-2) at a concentration of 0.1 mol/L was added, and the mixture was vortexed again. The reaction was carried out at 40°C for 0.5 hours to give the head-to-head single strands formed by conjugation of the 3' terminus of the sense strand and the 3' terminus of the sense strand, as specifically shown in Table 12.

Table 10 Sequence information of the head-to-head single strands formed by conjugation of the 3' terminus of the sense strand and the 3' terminus of the sense strand

| Head-to-head single strand No. | Sense strand (first target) | | Sense strand (second target) | |
|---|---|---|---|---|
| | No. | Sequence and carrier information (5'-3') | No. | Sequence and carrier information (5'-3') |
| RZ899SS/597SS-M26 | RZ899SS-M26<br>SEQ ID NO.27 | (CR01008)(CR01008)(CR01008)UmsUmsUmUmAmAmUfCfCfUfCmAmCmUmCmUmAmAmAm(NM090) | RZ597SS-M26<br>SEQ ID NO.28 | CmsCmsAmAmGmAmGfCfAfCfCmAmAmGmAmAmCmUmAm(CR01008)(CR01008)(CR01008)(NM091) |
| RZ899SS/597SS-M26.2 | RZ899SS-M26.2<br>SEQ ID NO.29 | UmsUmsUmUmAmAmUfCfCfUfCmAmCmUmCmUmAmAmAms(CR01008)s(CR01008)(NM090) | RZ597SS-M26.2<br>SEQ ID NO.30 | CmsCmsAmAmGmAmGfCfAfCfCmAmAmGmAmAmCmUmAms(CR01008)s(CR01008)(NM091) |
| RZ899SS/597SS-M26.3 | RZ899SS-M26.3<br>SEQ ID NO.31 | UmsUmsUmUmAmAmUfCfCfUfCmAmCmUmCmUmAmAmAms(CR01008)s(CR01008)s(CR01008)(NM090) | RZ597SS-M26.3<br>SEQ ID NO.32 | CmsCmsAmAmGmAmGfCfAfCfCmAmAmGmAmAmCmUmAms(CR01008)s(CR01008)s(CR01008)(NM091) |
| RZ899SS/597SS-M28 | RZ899SS-M28<br>SEQ ID NO.33 | (CR01008)(CR01008)UmsUmsUmUmAmAmUfCfCfUfCmAmCmUmCmUmAmAmAm(NM090) | RZ597SS-M28<br>SEQ ID NO.34 | (CR01008)(CR01008)CmsCmsAmAmGmAmGfCfAfCfCmAmAmGmAmAmCmUmAm(CR01008)(CR01008)(NM091) |
| RZ899SS/597SS-M28.2 | RZ899SS-M28.2<br>SEQ ID NO.35 | (CR01008)s(CR01008)sUmsUmsUmUmAmAmUfCfCfUfCmAmCmUmCmUmAmAmAms(CR01008)s(CR01008)(NM090) | RZ597SS-M28.2<br>SEQ ID NO.36 | (CR01008)s(CR01008)sCmsCmsAmAmGmAmGfCfAfCfCmAmAmGmAmAmCmUmAms(CR01008)s(CR01008)(NM091) |

Wherein, the structural formula of the head-to-head single strand formed by conjugation of the 3' terminus of the sense strand and the 3' terminus of the sense strand is as follows:

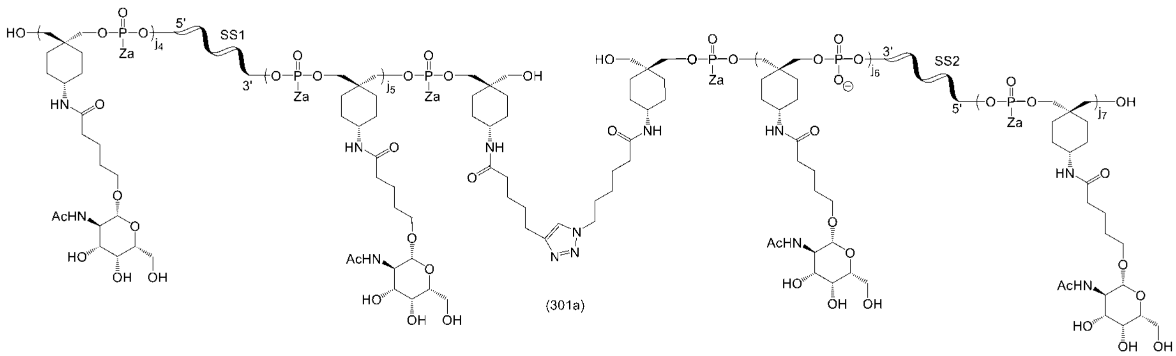

(301a)

;

wherein,

SS1

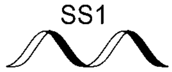

represents the sense strand (first target), and

SS2

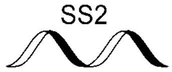

represents the sense strand (second target).

In RZ899SS/597SS-M26, j4=3, j5=0, j6=3, j7=0, and Za is

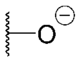

.

In RZ899SS/597SS-M26.2, j4=0, j5=2, j6=2, j7=0, and Za is

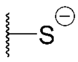

.

In RZ899SS/597SS-M26.3, j4=0, j5=3, j6=3, j7=0, and Za is

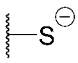

.

In RZ899SS/597SS-M28, j4=2, j5=0, j6=2, j7=2, and Za is

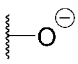

.

In RZ899SS/597SS-M28.2, j4=2, j5=2, j6=2, j7=2, and Za is

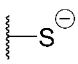

.

The naked sequences corresponding to the head-to-head single strands in Table 10 are shown in Table 11.

Table 11 Unmodified nucleotide sequence information of the head-to-head single strands formed by the conjugation of the 3' terminus of the sense strand and the 3' terminus of the sense strand

| Head-to-head single strand No. | Sense strand (first target) | | | Sense strand (second target) | | |
|---|---|---|---|---|---|---|
| | No. | Naked sequence information (5'-3') | | No. | Naked sequence information (5'-3') | |
| RZ899SS/597 SS-M26 | RZ899SS-M26 | UUUUAAUCCUCACUCU AAA | SEQ ID NO.1 | RZ597SS-M26 | CCAAGAGCACCAAGAA CUA | SEQ ID NO.2 |
| RZ899SS/597 SS-M26.2 | RZ899SS-M26.2 | UUUUAAUCCUCACUCU AAA | SEQ ID NO.1 | RZ597SS-M26.2 | CCAAGAGCACCAAGAA CUA | SEQ ID NO.2 |
| RZ899SS/597 SS-M26.3 | RZ899SS-M26.3 | UUUUAAUCCUCACUCU AAA | SEQ ID NO.1 | RZ597SS-M26.3 | CCAAGAGCACCAAGAA CUA | SEQ ID NO.2 |
| RZ899SS/597 SS-M28 | RZ899SS-M28 | UUUUAAUCCUCACUCU AAA | SEQ ID NO.1 | RZ597SS-M28 | CCAAGAGCACCAAGAA CUA | SEQ ID NO.2 |
| RZ899SS/597 SS-M28.2 | RZ899SS-M28.2 | UUUUAAUCCUCACUCU AAA | SEQ ID NO.1 | RZ597SS-M28.2 | CCAAGAGCACCAAGAA CUA | SEQ ID NO.2 |

(5-5) Synthesis of siRNAs

According to the sequence Nos. for the siRNAs in Table 12, each sequence was mixed at an equimolar ratio, dissolved in deionized water, heated to 95°C, slowly cooled to room temperature, and maintained at room temperature for 10 minutes, to allow the sense strand and the antisense strand to form a double-stranded structure via hydrogen bonding, thereby obtaining the siRNAs shown in Table 12.

Table 12 Sequence information of siRNAs

| siRNA No. | Sense strand | Antisense strand | Molar ratio of sense strand to antisense strand |
|---|---|---|---|
| RZ899058 | RZ899058-SS | RZ899058-AS | 1:1 |
| RZ597104 | RZ597104-SS | RZ597104-AS | 1:1 |

(5-6) Synthesis of siRNA Chimeras

According to the sequence No. for the siRNA chimeras in Table 13, each sequence was mixed at an equimolar ratio, dissolved in deionized water, heated to 95°C, slowly cooled to room temperature, and maintained at room temperature for 10 minutes, to allow the sense strand and the antisense strand to form a double-stranded structure via hydrogen bonding, thereby obtaining the siRNA chimeras shown in Table 13.

Table 13 Sequence information of siRNA chimeras

| **Head-to-tail siRNA chimeras** | | | | |
|---|---|---|---|---|
| siRNA chimera No. | Head-to-tail single strand formed by conjugation of the 3' terminus of the sense strand and the 5' terminus of the sense strand | Antisense strand | Antisense strand | Molar ratio of three components |
| RZ899/597-M1 | RZ899/597-M1-SS | RZ899058-AS | RZ597104-AS | 1:1:1 |
| RZ899/597-M2 | RZ899/597-M2-SS | RZ899058-AS | RZ597104-AS | 1:1:1 |
| RZ899/597-M3 | RZ899/597-M3-SS | RZ899058-AS | RZ597104-AS | 1:1:1 |
| RZ899/597-M4 | RZ899/597-M4-SS | RZ899058-AS | RZ597104-AS | 1:1:1 |
| Head-to-tail siRNA chimeras | | | | |
| siRNA chimera No. | Head-to-tail single strand formed by conjugation of the 3' terminus of the antisense strand and the 5' terminus of the sense strand | Sense strand | Antisense strand | Molar ratio of three components |
| RZ899/597-M16 | RZ597AS/899SS-M16 | RZ597104-SS | RZ899058-AS | 1:1:1 |
| RZ899/597-M16.2 | RZ597AS/899SS-M16.2 | RZ597104-SS | RZ899058-AS | 1:1:1 |
| RZ899/597-M16.3 | RZ597AS/899SS-M16.3 | RZ597104-SS2 | RZ899058-AS | 1:1:1 |
| RZ899/597-M21.2 | RZ597AS/899SS-M21 | RZ597104-SS | RZ899058-AS | 1:1:1 |
| siRNA chimera No. | Head-to-tail single strand formed by conjugation of the 3' terminus of the antisense strand and the 5' terminus of the sense strand | Head-to-tail single strand formed by conjugation of the 3' terminus of the antisense strand and the 5 terminus of the sense strand | | ratio of of two components |
| RZ899/597-M21 | RZ597AS/899SS-M21 | RZ899AS/597SS-M21 | | 1:1 |

(continued)

| Head-to-head siRNA chimeras | | | | |
|---|---|---|---|---|
| siRNA chimera No. | Head-to-head single strand formed by conjugation of the 3' terminus of the sense strand and the 3' terminus of the sense strand | Antisense strand | Antisense strand | Molar ratio of three components |
| RZ899/597-M26 | RZ899SS/597SS-M26 | RZ597104-AS | RZ899058-AS | 1:1:1 |
| RZ899/597-M26.2 | RZ899SS/597SS-M26.2 | RZ597104-AS | RZ899058-AS | 1:1:1 |
| RZ899/597-M26.3 | RZ899SS/597SS-M26.3 | RZ597104-AS | RZ899058-AS | 1:1:1 |
| RZ899/597-M28 | RZ899SS/597SS-M28 | RZ597104-AS | RZ899058-AS | 1:1:1 |
| RZ899/597-M28.2 | RZ899SS/597SS-M28.2 | RZ597104-AS | RZ899058-AS | 1:1:1 |

The synthesized compounds were subjected to molecular weight detection via liquid chromatography-mass spectrometry (LC-MS). The actual molecular weight was consistent with the theoretical molecular weight, indicating that the theoretically required nucleic acid chimeras were obtained.

## Biological Assays

Unless otherwise stated, the reagents, consumables, and instruments/equipment used in the biological assays of the present disclosure were all commercially available products. The main reagents, consumables, and their sources are shown in Table 14, and the main instruments, equipment, and their sources are shown in Table 15.

Table 14 Main reagents and consumables

| Name | Manufacturer |
|---|---|
| 1×PBS | MACGENE (Beijing) Biotechnology Ltd. |
| DMEM medium | MACGENE |
| Serum | Merck |
| Deoxyribonuclease I from bovine pancreas | SIGMA |
| Collagenase from Clostridium histolyticum | SIGMA |
| HBSS | Gibco |
| EDTA | Sinopharm Chemical Reagent Co., Ltd. |
| HBSS (without $Ca^{2+}$ $Mg^{2+}$) | MACGENE |
| Rat tail collagen type I | solarbio |
| Hanwei RNA extraction kit | Zhejiang Hanwei Technology Co., Ltd. |
| Reverse Transcription System | Promega Corporation |
| SYBR Select Master Mix | ABI |
| RNALater | Thermo Fisher Scientific |
| Chloral hydrate | Macklin |

Table 15 Main instruments and equipment

| Name | Manufacturer |
|---|---|
| Metal bath | Eppendorf |

(continued)

| Name | Manufacturer |
|---|---|
| Cell incubator | Thermo Fisher Scientific |
| Biosafety cabinet | ESCO |
| Automatic nucleic acid extraction system | Zhejiang Hanwei Technology Co., Ltd. |
| High-speed refrigerated centrifuge | Eppendorf |
| NANODROP OneC | Thermo Fisher Scientific |
| Gradient PCR amplifier | Eppendorf |
| Fluorescent quantitative PCR instrument | ABI StepOne Plus |
| Gel imaging system | Shanghai Tanon Life Science Co., Ltd. |
| Electrophoresis apparatus | Beijing Liuyi Biotechnology Co., Ltd. |
| Tissuelyser II automatic tissue homogenizer | Shanghai Jingxin Industrial Development Co., Ltd. |
| R540IE small animal anesthesia machine | RWD Life Science Co., Ltd. |

**Example 1**

Evaluation of Inhibitory Activity of Nucleic Acid Chimeras Against Superoxide Dismutase 1 (SOD1) and Angiopoietin-like 3 (ANGPTL3) in Mouse Primary Hepatocytes.

This example evaluated the inhibitory activity of four multi-targeting nucleic acid chimeras with different structures, RZ899/597-M1, RZ899/597-M2, RZ899/597-M3, and RZ899/597-M4, and the RZ899058 + RZ597104 mixture against the target genes, SOD1 and ANGPTL3, in primary hepatocytes from C56BL/6j mice.

(1-1) Isolation of Mouse Primary Hepatocytes

Mouse primary hepatocytes were isolated from fresh liver tissues of C56BL/6j mice. The specific operation steps were as follows: the mice were anesthetized by intraperitoneal injection of a 10% chloral hydrate solution and fixed, and their abdomens and chests were disinfected with 75% ethanol. Surgical instruments were sterilized. The abdominal cavity was opened to expose the hepatic portal vein and inferior vena cava. An indwelling needle equipped with a heparin cap was connected to a scalp needle attached to an infusion pump bottle (0.5 mM EDTA in HBSS as a perfusion solution). The needle was inserted through the inferior vena cava, and perfusion was performed at a rate of 120 drops/min. The hepatic portal vein was cut to allow the perfusion solution to flow out, and perfusion was continued for 4 minutes. Subsequently, the perfusion solution was replaced with a 0.8 mg/mL collagenase type IV solution in HBSS (Sigma, C5138) (comprising 0.08% DNase 1 (Sigma, DN25)), and perfusion was continued for another 8 minutes. The perfused liver was removed from the animal and washed with HBSS (comprising $Ca^{2+}$ and $Mg^{2+}$, MACGENE, CC016). The liver was placed in a sterile Petri dish, and DMEM complete medium (DMEM medium + 10% serum) was added to mince the liver. The cell suspension was filtered through a cell strainer to remove undigested tissues and connective tissues. The suspension was centrifuged at 800 rpm for 3 min, the supernatant was discarded, and DMEM complete medium was added again for resuspension and centrifugation to obtain mouse primary hepatocytes.

(1-2) Cell Culture and Transfection

DMEM complete medium was added to adjust the cell density to $2 \times 10^5$ cells/mL to provide a mouse primary hepatocyte suspension. Subsequently, the cells were seeded into 12-well culture plates pre-coated with rat tail collagen type I (coating procedure was performed at a concentration of 2 $\mu g/cm^2$ according to the instructions provided by Solarbio (C8062)). The volume of the cell suspension added was 1000 $\mu$L/well, corresponding to a cell count of $2 \times 10^5$ cells/well.

Each group of siRNA chimeras was diluted with PBS to a 5 $\mu$M siRNA chimera working solution (based on the siRNA chimera); RZ899058 and RZ597104 were separately diluted with PBS buffer to a 5 $\mu$M RZ899058 working solution (based on siRNA) and a 5 $\mu$M RZ597104 working solution (based on siRNA). 2 $\mu$L/well of the siRNA chimera working solution was added to the above 12-well culture plates, wherein for the RZ899058 + RZ597104 group, 2 $\mu$L/well of the RZ899058 working solution and 2 $\mu$L/well of the RZ597104 working solution were added to the same culture well. This corresponded to a final transfection concentration of 10 nM for each siRNA per well (based on siRNA), and each siRNA was tested in duplicate wells. Additionally, 2 $\mu$L/well of the PBS buffer was added to another 2 culture wells as blank control

wells. The culture plate was shaken to achieve uniform mixing.

The culture plate was placed in a cell incubator at 37°C with 5% $CO_2$ for further incubation of 24 h.

(1-3) Detection

RNA Extraction: Total RNA was extracted from each group of primary hepatocyte samples using an automatic nucleic acid extraction instrument and a nucleic acid extraction kit from Zhejiang Hanwei Technology Co., Ltd., following the method described in the instructions.

Reverse transcription reaction: 1000 ng of total RNA from each extracted primary hepatocyte sample was taken. A 20 μL reverse transcription system was prepared with Reverse Transcription System (A3500) from Promega Corporation using Oligo $(dT)_{15}$ reverse transcription primers, and the reverse transcription reaction was performed according to the method described in the kit instructions. After completion of the reaction, 80 μL of RNase-Free water was added to the reverse transcription system to obtain a cDNA solution for real-time PCR detection.

Real-time PCR detection: A 20 μL real-time PCR reaction system was prepared for each PCR well with the SYBR™ Select Master Mix reagent (Catalog number: 4472908) from ABI in accordance with the method described in the kit instructions. Each reaction system contained 5 μL of the cDNA template obtained from the above reverse transcription reaction, 10 μL of SYBR™ Select Master Mix, 0.5 μL of 10 μM forward primer, 0.5 μL of 10 μM reverse primer (primer sequences are shown in Table 16), and 4 μL of RNase-Free $H_2O$. The prepared reaction systems were placed on an ABI StepOnePlus PCR instrument, and real-time PCR amplification was performed using the three-step method. The amplification program was as follows: pre-denaturation at 95°C for 10 min, followed by 40 cycles comprising denaturation at 95°C for 30 s, annealing at 60°C for 30 s, and extension at 72°C for 30 s. After completion of the program, the difference in gene expression was calculated using the ΔΔCt method described above.

Table 16 Primer sequence list

| Targets | Primer type | Primer sequence (5'-3') | SEQ ID |
|---|---|---|---|
| GAPDH | Forward primer | TGCACCACCAACTGCTTAG | NO.6 |
| | Reverse primer | GGATGCAGGGATGATGTTC | NO.7 |
| ANGPTL3 | Forward primer | GAGGAGCAGCTAACCAACTTAAT | NO.8 |
| | Reverse primer | TCTGCATGTGCTGTTGACTTAAT | NO.9 |
| SOD1 | Forward primer | GGGTTCCACGTCCATCAGTA | NO.10 |
| | Reverse primer | ACACCGTCCTTTCCAGCAGT | NO.11 |

(3-4) Analysis

The detection results are shown in Table 17 and Fig. 1.

Table 17 Inhibitory effects of target genes SOD1/ANGPTL3 in mouse primary hepatocytes

| Groups | SOD1 | | ANGPTL3 | |
|---|---|---|---|---|
| | Mean inhibition rate (%) | STDEV | Mean inhibition rate (%) | STDEV |
| PBS | 0.00 | 9.52 | 0.00 | 6.65 |
| RZ899058+RZ597104 | 95.16 | 0.30 | 86.07 | 0.43 |
| RZ899/597-M1 | 88.27 | 0.48 | 74.20 | 1.13 |
| RZ899/597-M2 | 95.77 | 0.18 | 92.81 | 1.09 |
| RZ899/597-M3 | 86.33 | 0.59 | 74.74 | 0.45 |
| RZ899/597-M4 | 94.19 | 0.17 | 87.21 | 0.19 |

The results showthat the nucleic acid chimeras RZ899/597-M1, RZ899/597-M2, RZ899/597-M3, and RZ899/597-M4 are all capable of effectively reducing the mRNA expression levels of the target genes SOD1 and ANGPTL3. Among them, the inhibitory activities of RZ899/597-M2 and RZ899/597-M4 against the target genes were comparable to, or even higher than, those of the RZ899058 + RZ597104 mixture group.

**Example 2**

Evaluation of $IC_{50}$ Inhibitory Activity of Nucleic Acid Chimeras Against Target Genes SOD1 and ANGPTL3 in Mouse Primary Hepatocytes.

This Example evaluated the $IC_{50}$ inhibitory activity of the siRNA chimeras RZ899/597-M2, RZ899/597-M4, and the RZ899058 + RZ597104 mixture against the target genes SOD1 and ANGPTL3 in primary hepatocytes from C56BL/6j mice.

(2-1) Isolation of Mouse Primary Hepatocytes: Same as step (1-1) in Example 1.

(2-2) Cell Culture and Transfection

Mouse primary hepatocytes were isolated from fresh liver tissues of C56BL/6j mice and seeded into 12-well culture plates at a density of $2 \times 10^5$ cells/well. RZ899/597-M2 and RZ899/597-M4 were serially diluted with PBS buffer to siRNA chimera working solutions at concentrations of 50 μM, 10 μM, 2 μM, 0.4 μM, 0.08 μM, 0.016 μM, and 0.0032 μM (based on siRNA chimeras); RZ899058 was serially diluted with PBS buffer to RZ899058 working solutions at concentrations of 50 μM, 10 μM, 2 μM, 0.4 μM, 0.08 μM, 0.016 μM, and 0.0032 μM; and RZ597104 was serially diluted with PBS buffer to RZ597104 working solutions at concentrations of 50 μM, 10 μM, 2 μM, 0.4 μM, 0.08 μM, 0.016 μM, and 0.0032 μM.

siRNA chimera group: 2 μL/well of siRNA chimera working solution at each concentration was added to the above 12-well culture plates, and the final transfection concentrations for each siRNA chimera (based on siRNA) were 100 nM, 20 nM, 4 nM, 0.8 nM, 0.16 nM, 0.032 nM, and 0.0064 nM, respectively. RZ899058 + RZ597104 mixture group: RZ899058 working solution and RZ597104 working solution at the same concentration were added to the same well of the 12-well culture plates, and the final transfection concentrations for the RZ899058 + RZ597104 mixture group were 00 nM, 20 nM, 4 nM, 0.8 nM, 0.16 nM, 0.032 nM, and 0.0064 nM (based on siRNA), respectively. For each final transfection concentration in each experimental group, 2 culture wells were set; and additionally, 2 μL/well of PBS buffer was added to another 2 culture wells as blank control wells. The culture plates were shaken to achieve uniform mixing. The culture plates were further incubated in a cell incubator at 37°C with 5% $CO_2$ for 24 h.

(2-3) Detection: Same as step (1-3) in Example 1.

IC50 curve fitting was performed using the four-parameter fitting model [log(inhibitor) vs. response - Variable slope (four parameters)] in GraphPad Prism 8.0. The IC50 concentration value was caculated using the following formula:

$$X=10^\wedge(LogIC50-Log((Top-Bottom)/(50-Bottom)-1)/HillSlope),$$

where each parameter in the formula can be obtained from the analysis result table in GraphPad Prism 8.0.

The detection results are shown in Tables 18 and 19, and Figs. 2 and 3.

Table 18 Inhibitory effects of target genes SOD1/ANGPTL3 in mouse primary hepatocytes

| Concentration /nM | SOD1 | | | | | |
|---|---|---|---|---|---|---|
| | RZ899058+RZ597104 | | RZ899/597-M2 | | RZ899/597-M4 | |
| | Mean inhibition rate % | STDEV | Mean inhibition rate % | STDEV | Mean inhibition rate % | STDEV |
| 0.0064 | -13.95 | 34.08 | 9.67 | 6.16 | 12.54 | 3.11 |
| 0.032 | 4.53 | 16.03 | 18.06 | 1.90 | 18.75 | 3.07 |
| 0.16 | 14.84 | 9.14 | 30.37 | 0.73 | 26.72 | 0.57 |
| 0.8 | 64.84 | 2.06 | 69.86 | 0.13 | 63.72 | 3.69 |
| 4 | 90.81 | 0.06 | 91.87 | 0.34 | 89.38 | 0.73 |
| 20 | 97.38 | 0.13 | 97.45 | 0.12 | 96.1 | 0.20 |
| 100 | 98.01 | 0.01 | 98.07 | 0.04 | 97.58 | 0.01 |

(continued)

| Concentration /nM | ANGPTL3 | | | | | |
|---|---|---|---|---|---|---|
| | RZ899058+RZ597104 | | RZ899/597-M2 | | RZ899/597-M4 | |
| | Mean inhibition rate % | STDEV | Mean inhibition rate % | STDEV | Mean inhibition rate % | STDEV |
| 0.0064 | 2.36 | 5.43 | 5.53 | 7.34 | -3.00 | 11.32 |
| 0.032 | 9.16 | 5.15 | 13.58 | 2.28 | 9.52 | 2.85 |
| 0.16 | 14.77 | 6.17 | 33.19 | 7.45 | 25.50 | 3.41 |
| 0.8 | 25.43 | 0.35 | 57.05 | 3.58 | 38.6 | 8.49 |
| 4 | 65.44 | 0.51 | 71.59 | 0.04 | 65.15 | 3.23 |
| 20 | 83.08 | 2.55 | 90.82 | 0.72 | 83.25 | 0.50 |
| 100 | 88.87 | 1.03 | 89.64 | 0.68 | 86.26 | 0.39 |

Table 19 $IC_{50}$ values for the target genes SOD1/ANGPTL3 in mouse primary hepatocytes

| Groups | IC50(nM) | |
|---|---|---|
| | SOD1 | ANGPTL3 |
| RZ899058+RZ597104 | 0.4976 | 2.1649 |
| RZ899/597-M2 | 0.3655 | 0.5364 |
| RZ899/597-M4 | 0.4762 | 1.2998 |

The results show that the IC50 values for gene inhibition of SOD1 and ANGPTL3 in the RZ899/597-M2 group were 0.3655 nM and 0.5364 nM, respectively; and the IC50 values for gene inhibition of SOD1 and ANGPTL3 in the RZ899/597-M4 group were 0.4762 nM and 1.2998 nM, respectively, both superior to those of the RZ899058 + RZ597104 mixture group, which were 0.4976 nM and 2.1649 nM.

**Example 3**

Evaluation of Inhibitory Activity of Nucleic Acid Chimeras Against Target Genes ANGPTL3 and SOD1 in Mice.

This Example evaluates the inhibitory activity of the multi-target siRNA chimeras, RZ899/597-M2 and RZ899/597-M4, and the RZ899058 + RZ597104 mixture against the target genes SOD1 and ANGPTL3 in mice using a method for evalucating *in vivo* inhibitory activity against target genes in mice.

(3-1) Animal Grouping, Administration, and Tissue Sample Collection

C57BL/6J mice aged 6-8 weeks were randomly divided into 4 groups by body weight, with 15 mice per group. Each group of mice was administered with the above siRNA chimeras via abdominal subcutaneous administration. Each mouse in the PBS control group was given a dose of 5 mL/kg (based on mouse body weight); each mouse in the RZ899/597-M2 and RZ899/597-M4 experimental groups was administered a dose of 6 mg (based on siRNA)/kg (based on mouse body weight) (corresponding to a dose of 3 mg (based on siRNA)/kg (based on mouse body weight) for each target-specific siRNA); the RZ899058 + RZ597104 mixture group was given the mixture at a dose of 6 mg (based on siRNA)/kg (based on mouse body weight) (corresponding to a dose of 3 mg (based on siRNA)/kg (based on mouse body weight) for each target-specific siRNA). Exemplarily, the day of administration was designated as the first day (D0). After administration, 5 mice in each group were sacrificed on Day 7 (D7), Day 28 (D28), and Day 56 (D56), respectively. The animals were subjected to gross dissection, and liver tissues were collected, cut into several 2 $mm^3$ pieces, and preserved in RNAlater.

(3-2) Detection

RNA Extraction: An appropriate amount of liver tissue samples was taken from RNAlater and homogenized for 60 s in a Tissuelyser II automatic tissue homogenizer. Total RNA was extracted from each liver tissue sample using an automatic nucleic acid extraction instrument and a nucleic acid extraction kit from Zhejiang Hanwei Technology Co., Ltd.,

following the method described in the instructions.

Reverse transcription reaction and real-time PCR detection: Same as step (1-3) in Example 1.

(3-3) Analysis

The detection results are shown in Table 20, and Figs. 4 and 5.

Table 20 Inhibitory effects for the target genes SOD1/ANGPTL3 in mice

| Detection time | Groups | SOD1 | | ANGPTL3 | |
|---|---|---|---|---|---|
| | | Mean inhibition rate (%) | STDEV | Mean inhibition rate (%) | STDEV |
| D7 | PBS | 0.00 | 27.35 | 0.00 | 28.42 |
| | RZ899058+RZ597104 | 96.79 | 0.69 | 77.86 | 7.89 |
| | RZ899/597-M2 | 92.46 | 2.50 | 83.66 | 2.92 |
| | RZ899/597-M4 | 93.04 | 2.60 | 86.93 | 5.60 |
| D28 | PBS | 0.00 | 10.21 | 0.00 | 4.12 |
| | RZ899058+RZ597104 | 95.91 | 1.12 | 63.66 | 11.02 |
| | RZ899/597-M2 | 89.33 | 1.73 | 71.12 | 5.81 |
| | RZ899/597-M4 | 84.94 | 3.10 | 68.43 | 8.15 |

The above results indicate that in mice, the inhibitory effects of the multi-targeting conjugates RZ899/597-M2 and RZ899/597-M4 on the mRNA expression of SOD1 and ANGPTL3 were comparable to or slightly superior to those of the RZ899058 + RZ597104 mixture group.

**Example 4**

Evaluation of Inhibitory Activity of Multi-Targeting siRNA Chimeras Against Superoxide Dismutase 1 (SOD1) and Angiopoietin-like 3 (ANGPTL3) in Mouse Primary Hepatocytes.

This Example evaluates the inhibitory activity of the multi-targeting siRNA chimeras, RZ899/597-M16, RZ899/597-M16.2, RZ899/597-M21 and RZ899/597-M21.2, and the control siRNA mixture, RZ899058 + RZ598104, against the target genes SOD1 and ANGPTL3 using an activity evaluation assay in mouse primary hepatocyte. The dual-target connection mode of RZ899/597-M16, RZ899/597-M16.2, RZ899/597-M21, and RZ899/597-M21.2 all is that the sense strand (SS) for one target is linked to the antisense strand (AS) for the other target, with the connection direction being the 5' terminus of the SS strand linked to the 3' terminus of the AS strand, and the distinctions among them lie solely in the linker and the manner of carrier conjugation.

The primer sequence information used in this experiment is identical to that provided in Table 16.

The results of Example 4 indicate that the multi-targeting conjugates tested in the study were all capable of effectively reducing the mRNA expression of the target genes SOD1 and ANGPTL3. Among them, the inhibitory activities of RZ899/597-M16.2, RZ899/597-M21, and RZ899/597-M21.2 against the target genes were comparable to those of the RZ899058 + RZ597104 mixture (Fig. 6, Table 21).

Table 21 Inhibitory effects for target genes SOD1/ANGPTL3 in mouse primary hepatocytes after administration of siRNA chimeras

| Groups | SOD1 | | ANGPTL3 | |
|---|---|---|---|---|
| | Mean inhibition rate (%) | STDEV | Mean inhibition rate (%) | STDEV |
| PBS | 0.00 | 9.30 | 0.00 | 6.10 |
| RZ899058+RZ597104 | 96.39 | 0.77 | 87.74 | 6.64 |
| RZ899/597-M16 | 90.14 | 0.97 | 71.61 | 7.69 |
| RZ899/597-M16.2 | 91.24 | 4.67 | 87.87 | 1.51 |
| RZ899/597-M21 | 93.50 | 1.78 | 81.34 | 1.07 |

(continued)

| Groups | SOD1 | | ANGPTL3 | |
|---|---|---|---|---|
| | Mean inhibition rate (%) | STDEV | Mean inhibition rate (%) | STDEV |
| RZ899/597-M21.2 | 95.06 | 0.23 | 87.46 | 1.90 |

**Example 5**

Evaluation of Inhibitory Activity of Multi-Targeting siRNA Chimeras Against SOD1 and ANGPTL3 in Mouse Primary Hepatocytes.

This Example evaluates the inhibitory activity of the dual-target siRNA chimeras, RZ899/597-M26, RZ899/597-M26.2, RZ899/597-M26.3, RZ899/597-M28 and RZ899/597-M28.2, and the control siRNA mixture, RZ899058 + RZ598104, against the target genes SOD1 and ANGPTL3 using an activity evaluation assay in mouse primary hepatocyte. The sense strand connection mode of this series of the dual-target siRNA conjugates is that the 3' terminus of the sense strand for one target is linked to the 3' terminus of the sense strand for the other target via click chemistry, with the difference of the manner of the carrier conjugation.

The isolation, culture, and transfection of mouse primary hepatocytes were performed as described above. The density of mouse primary hepatocytes was adjusted to $2 \times 10^5$ cells/mL, and the cells were seeded into the 12-well culture plates pre-coated with rat tail collagen Type I. The volume of the cell suspension added was 1000 μL/well, i.e., an amount of $2 \times 10^5$ cells/well. Each group of multi-targeting siRNA conjugates was diluted with PBS to a 5 μM working solution (based on siRNA). 2 μL/well of the multi-targeting siRNA conjugate working solution was added to the above 12-well culture plates, respectively. For the RZ899058 + RZ597104 group, 2 μL/well of RZ899058 and 2 μL/well of RZ597104 were added to the same culture well, respectively. This corresponded to a final transfection concentration of 10 nM for each multi-targeting siRNA conjugate in each well (based on siRNA), and 2 culture wells were set for each multi-targeting siRNA conjugate. Additionally, 2 μL/well of PBS was added to another 2 culture wells as blank control wells. The culture plates were shaken to achieve uniform mixing. The culture plates were placed in a cell incubator at 37°C with 5% $CO_2$ for further incubation of 24 h. RNA extraction and detection were performed as described above, and the primers were as shown in Example 4.

The results of Example 5 show that, the multi-targeting siRNA chimeras, RZ899/597-M26, RZ899/597-M26.2, RZ899/597-M26.3, RZ899/597-M28 and RZ899/597-M28.2, were all capable of effectively reducing the mRNA expression levels of the target genes SOD1 and ANGPTL3. Among them, RZ899/597-M26.3, RZ899/597-M28 and RZ899/597-M28.2 exhibited higher inhibitory activities against the two target genes compared with the control group (Fig. 7, Table 22).

Table 22 Inhibitory activity for target genes SOD1/ANGPTL3 in mouse primary hepatocytes after administration of siRNA chimeras

| Groups | SOD1 | | ANGPTL3 | |
|---|---|---|---|---|
| | Mean inhibition rate (%) | STDEV | Mean inhibition rate (%) | STDEV |
| PBS | 0.00 | 13.63 | 0.00 | 17.52 |
| RZ899058+RZ597104 | 95.28 | 1.10 | 89.84 | 1.80 |
| RZ899/597-M26 | 92.19 | 1.00 | 80.00 | 1.45 |
| RZ899/597-M26.2 | 89.32 | 1.31 | 84.31 | 0.98 |
| RZ899/597-M26.3 | 94.09 | 1.21 | 91.60 | 0.31 |
| RZ899/597-M28 | 94.01 | 0.53 | 97.43 | 0.36 |
| RZ899/597-M28.2 | 96.10 | 1.27 | 95.01 | 1.21 |

**Example 6**

Evaluation of Inhibitory Activity of Multi-Targeting siRNA Chimeras Against SOD1 and ANGPTL3 in Mice

This Example evaluates the inhibitory activity of the siRNA chimera RZ899/597-M16.3, which simultaneously targets the SOD1 and ANGPTL3 genes, and the control siRNA mixture RZ899058 + RZ598104 against the target genes SOD1 and ANGPTL3 in C57BL/6J mice. The dual-target connection mode of RZ899/597-M16.3 is that the sense strand (SS) for one target is linked to the antisense strand (AS) for the other target, with the connection direction of the 5' terminus

of the SS strand linked to the 3' terminus of the AS strand.

C57BL/6j mice aged 6-8 weeks were randomly divided into 4 groups by body weight, with 15 mice per group. Each group of mice was administered the above siRNA conjugate via abdominal subcutaneous administration. Each mouse in the PBS control group was given a dose volume of 5 mL/kg, and each mouse in the siRNA conjugate experimental group was administered a dose of 6 mg/kg (based on siRNA) with a dose volume of 5 mL/kg. The day of administration was designated as Day 0 (D0). After administration, 5 mice in each group were sacrificed on Day 7 (D7), Day 14 (D14), and Day 28 (D28), respectively. The animals were subjected to gross dissection, and liver tissues were collected, cut into several 2 $mm^3$ pieces, and preserved in RNAlater. During the experiment, an appropriate amount of liver tissue samples was taken and homogenized in a Tissuelyser II automatic tissue homogenizer for 60 seconds. RNA extraction and detection were performed as described above, and the difference in gene expression was calculated using the ΔΔCt method. Primers were as shown in Example 4.

The results of Example 6 show that, in mice, compared with the RZ899058 + RZ597104 mixture, the multi-targeting conjugate RZ899/597-M16.3 exhibited comparable inhibitory effects on the SOD1 gene on D7, D14, and D28, and exerted a higher inhibitory effect on the ANGPTL3 gene (Figs. 8-9, Table 23).

Table 23 Inhibitory effects for target genes SOD1/ANGPTL3 in mice after administration of siRNA chimeras

| Detection time | Groups | SOD1 | | ANGPTL3 | |
|---|---|---|---|---|---|
| | | Mean inhibition rate (%) | STDEV | Mean inhibition rate (%) | STDEV |
| D7 | PBS | 0.00 | 13.33 | 0.00 | 15.46 |
| | RZ899058+RZ597104 | 97.12 | 0.45 | 88.57 | 4.51 |
| | RZ899/597-M16.3 | 98.47 | 0.33 | 99.33 | 0.30 |
| D14 | PBS | 0.00 | 8.00 | 0.00 | 30.93 |
| | RZ899058+RZ597104 | 97.06 | *1.05* | 84.94 | 5.43 |
| | RZ899/597-M16.3 | 98.69 | 0.25 | 99.15 | 0.49 |
| D28 | PBS | 0.00 | 6.71 | 0.00 | 9.08 |
| | RZ899058+RZ597104 | 96.10 | 0.84 | 63.44 | 10.60 |
| | RZ899/597-M16.3 | 96.22 | 1.65 | 93.78 | 3.45 |

## Example 7

Evaluation of Inhibitory Activity of Multi-Targeting siRNA Chimeras Against SOD1 and ANGPTL3 in Mice

This Example evaluates the inhibitory activity of the siRNA chimeras RZ899/597-M26.3 and RZ899/597-M28.2, each of which simultaneously target the SOD1 and ANGPTL3 genes, against the target genes SOD1 and ANGPTL3 in C57BL/6J mice using an *in vivo* evaluation method in mice.

C57BL/6j mice aged 6-8 weeks were randomly divided into 4 groups by body weight, with 15 mice per group. Each group of mice was administered the above siRNA chimeras via abdominal subcutaneous administration. Each mouse in the PBS control group was given a dose volume of 5 mL/kg, and each mouse in the siRNA chimera experimental group was administered a dose of 6 mg/kg (based on siRNA) with a dose volume of 5 mL/kg. The day of administration was designated as Day 0 (D0). After administration, 5 mice in each group were sacrificed on Day 7 (D7), Day 14 (D14), and Day 28 (D28), respectively. The animals were subjected to gross dissection, and liver tissues were collected, cut into several 2 $mm^3$ pieces, and preserved in RNAlater. During the experiment, an appropriate amount of liver tissue samples was taken and homogenized in a Tissuelyser II automatic tissue homogenizer for 60 seconds. RNA extraction and detection were performed as described above, and the difference in gene expression was calculated using the ΔΔCt method. Primers were as shown in Example 4.

The results of Example 7 show that, in mice, the inhibitory activities of RZ899/597-M26.3 against the target genes SOD1 and ANGPTL3 were essentially comparable to those of the RZ899058 + RZ597104 mixture on D7, D14, and D28. Compared with the RZ899058 + RZ597104 mixture, RZ899/597-M28.2 exerted a higher inhibitory effect on both target genes SOD1 and ANGPTL3 (Figs. 10-11, Table 24).

Table 24 Inhibitory effects for target genes SOD1/ANGPTL3 in mice after administration of siRNA chimeras

| Detection time | Groups | SOD1 | | ANGPTL3 | |
|---|---|---|---|---|---|
| | | Mean inhibition rate (%) | STDEV | Mean inhibition rate (%) | STDEV |
| D7 | PBS | 0.00 | 13.33 | 0.00 | 15.46 |
| | RZ899058+RZ597104 | 97.12 | 0.45 | 88.57 | 4.51 |
| | RZ899/597-M26.3 | 97.55 | 0.46 | 93.03 | 4.39 |
| | RZ899/597-M28.2 | 97.61 | 0.72 | 95.45 | 2.54 |
| D14 | PBS | 0.00 | 8.00 | 0.00 | 30.93 |
| | RZ899058+RZ597104 | 97.06 | 1.05 | 84.94 | 5.43 |
| | RZ899/597-M26.3 | 97.54 | 0.74 | 93.61 | 2.63 |
| | RZ899/597-M28.2 | 98.03 | 0.66 | 96.17 | 1.80 |
| D28 | PBS | 0.00 | 6.71 | 0.00 | 9.08 |
| | RZ899058+RZ597104 | 96.10 | 0.84 | 63.44 | 10.60 |
| | RZ899/597-M26.3 | 92.58 | 4.63 | 56.17 | 17.97 |
| | RZ899/597-M28.2 | 95.17 | 3.26 | 74.48 | 17.35 |

Finally, it should be noted that the above embodiments are merely intended to illustrate the technical solutions of the present invention, but not to limit them. Although the present invention has been described in detail with reference to the foregoing embodiments, it should be understood by those of ordinary skill in the art that modifications may still be made to the technical solutions described in the foregoing embodiments, or equivalent substitutions may be made to some or all of the technical features thereof. However, such modifications or substitutions do not make the essence of the corresponding technical solutions depart from the scope of the technical solutions of the respective embodiments of the present invention.

## Claims

1. A nucleic acid chimera, comprising a nucleotide unit I having a structure represented by formula (101):

$$H-\left(X_1\right)_{j_1}-Y_1-\left[\left(X_2\right)_{j_2}-Y_2\right]_k-\left(X_3\right)_{j_3}-H$$

(101)

wherein each of $X_1$, $X_2$ and $X_3$ is independently selected from

$$*-\left(M_1\right)_m-\left(M_2\right)_n-*$$ ,

and each $M_1$ independently comprises a ligand capable of binding to a cellular receptor; and
each $M_2$ is independently selected from a modified or unmodified nucleotide; each m is independently selected from 1, 2, or 3; and each n is independently selected from an integer of 0-3;
wherein $X_1$, $X_2$, and $X_3$ may be the same or different; and
wherein each of $Y_1$ and $Y_2$ independently represents a sense strand of a double-stranded oligonucleotide, and each of the sense strands may correspond to the same target gene or a different target gene; and the corresponding relationship means that the sense strand is identical or substantially identical to a segment of consecutive nucleotides in the mRNA of the target gene;
$j_1$, $j_2$ and $j_3$ are independently selected from an integer of 0-3, and $j_2 \neq 0$; and
k is selected from an integer of 1 to 5.

2. The nucleic acid chimera according to claim 1, wherein the nucleic acid chimera further comprises a nucleotide unit II, the nucleotide unit II comprises an antisense strand that is complementary or substantially complementary to the

sense strand of the double-stranded oligonucleotide, the number of the antisense strands is equal to the number of the sense strands, and each of the antisense strands is at least partially complementary to a corresponding sense strand thereof to form a duplex region; wherein the antisense strand is complementary or at least partially complementary to mRNA of at least one target gene;

optionally, each of the antisense strand is independently complementary or at least partially complementary to mRNAs of two or at least two target genes;
optionally, the sense strand and the antisense strand of the double-stranded oligonucleotide each comprise at least partially modified nucleotides;
optionally, the sense strand and/or the antisense strand comprise at least one phosphorothioate linkage;
optionally, the sense strand and the antisense strand comprise at least 15 consecutive nucleotides, wherein the antisense strand has complementarity to the mRNA of the target gene; and
optionally, after *in vivo* administration, the chimera is optionally cleaved at $X_2$ or at a position attached to $X_2$, thereby cleaving to give independent double-stranded oligonucleotide portions.

**3.** The nucleic acid chimera according to claim 1, wherein, k is selected from 1;

optionally, $j_1$ is selected from 0, $j_2$ is selected from 3, and $j_3$ is selected from 0;
optionally, $j_1$ is selected from 0, $j_2$ is selected from 3, and $j_3$ is selected from 3;
optionally, $j_1$ is selected from 1, $j_2$ is selected from 1, and $j_3$ is selected from 1;
optionally, $j_1$ is selected from 2, $j_2$ is selected from 2, and $j_3$ is selected from 2;
optionally, $j_1$ is selected from 3, $j_2$ is selected from 3, and $j_3$ is selected from 0;
optionally, $j_1$ is selected from 3, $j_2$ is selected from 0, and $j_3$ is selected from 3;
optionally, m is selected from 1, 2 or 3, and each n is independently selected from 0 or 1; and
preferably, m is selected from 1, and n is selected from 0; or, m is selected from 1, and n is selected from 1.

**4.** The nucleic acid chimera according to any one of claims 1-3, wherein the modified nucleotide is independently selected from an abasic nucleotide, a 2'-halogenated modified nucleotide, a 2'-deoxy modified nucleotide or a 2'-O-$(CH_2)_{n2}$-$R_1$ modified nucleotide, or a nucleotide analog; and the nucleotide analog is selected from one or more of PNA, MNA, BNA, LNA, GNA, TNA, and UNA;

wherein, $n_2$ is selected from 0, 1 or 2; $R_1$ is selected from optionally substituted $C_{1-6}$ alkyl, optionally substituted $C_{1-6}$ alkoxy, or -$Si(R_{1a})_3$, each $R_{1a}$ is independently selected from optionally substituted $C_{1-6}$ alkyl or optionally substituted $C_{1-6}$ alkoxy, wherein the term "substituted" means that one or more hydrogen atoms on the alkyl or alkoxy are replaced by substituents; and optionally, the substituents are independently selected from $C_1$-$C_3$ alkyl, $C_1$-$C_3$ alkoxy, or halogen;
optionally, 2'-O-$(CH_2)_{n2}$-$R_1$ is selected from 2'-O-$CH_3$, 2'-O-$CH_2$-$CH_3$, 2'-O-TBDMS, 2'-O-TIPS, 2'-O-TOM, 2'-O-$CH_2$-O-$CH_2$-$CH_3$, 2'-O-$CH_2$-O-$CH_2$-$CF_3$ or 2'-O-$CH_2$-$CH_2$-O-$CH_3$;
optionally, the sense strand and the antisense strand of the double-stranded oligonucleotide are complementary or substantially complementary to form a duplex as shown in the following formulas:

SS: 5'- (N)a' - (X)p' - (N)b' - (X)q' - (N)c' - (X)r' - (N) d' -3'
AS: 3' - (N)a - (X)p - (N)b - (X)q - (N)c -5',

wherein SS represents the sense strand, and AS represents the antisense strand; and all nucleotides of the sense and antisense strands are modified nucleotides;
each N independently represents the following modified nucleotide: a 2'-O-methyl modified nucleotide, a 2'-fluoro modified nucleotide, or a 2'-deoxy modified nucleotide;
each X independently represents a 2'-O-methoxyethyl modified nucleotide, a 2'-O-methyl modified nucleotide, or a 2'-$O(CH_2)_{n3}OR_3$ substituted modified nucleotide; wherein $n_3$ is 1 or 2, and $R_3$ is selected from substituted or unsubstituted $C_1$-$C_6$ alkyl, or substituted or unsubstituted $C_1$-$C_6$ alkoxy; and optionally, a substituent is selected from halogen, $C_1$-$C_3$ alkyl, $C_1$-$C_3$ alkoxy, hydroxyl, or amino;
a, a', p, p', b, b', q, q', c, c', r' and d' each independently represent the number of nucleotides, wherein: a' is selected from an integer of 3-8; p' is selected from an integer of 0-3; b' is selected from an integer of 4-13; q' is selected from an integer of 0-4; c' is selected from an integer of 3-9; r' is selected from an integer of 0-3; d' is selected from an integer of 0-9; a is selected from an integer of 4-7; p is selected from an integer of 0-1; b is selected from an integer of 4-8; q is selected from an integer of 0-4; c is selected from an integer of 6-10; and, p', q', r', p and q are not simultaneously 0, and $0 \leq q' + r' \leq 4$;

preferably, a' is selected from an integer of 3-8; p' is selected from 0 or 1; b' is selected from an integer of 4-13; q' is selected from 0 or 1; c' is selected from an integer of 3-9; r' is selected from 0 or 1; d' is selected from an integer of 1-8; a is selected from an integer of 4-7; p is selected from 1; b is selected from an integer of 4-8; q is selected from 0 or 1; and, c is selected from an integer of 6-10;
preferably, the nucleotide N is selected from a 2'-O-methyl modified nucleotide or a 2'-fluoro modified nucleotide, and the nucleotide X is selected from a 2'-O-methoxyethyl modified nucleotide or a 2'-O-methyl modified nucleotide, and further preferably a 2'-O-methoxyethyl modified nucleotide;
optionally, the sense strand is 17-21 nucleotides in length, and the antisense strand is 19-23 nucleotides in length;
optionally, in the duplex, in the direction from the 5' end to the 3' end, in the sense strand, at least three nucleotides of the nucleotides at positions 7-10 of the nucleotide sequence are selected from 2'-fluoro modified nucleotides, and the nucleotides at the remaining positions are selected from 2'-O-methyl modified nucleotides or 2'-O-methoxyethyl modified nucleotides; and in the antisense strand, the nucleotides at positions 2, 6, 14, and 16 of the nucleotide sequence are selected from 2'-fluoro modified nucleotides, any one of the nucleotides at positions 9, 10, 11, and 12 is selected from a 2'-fluoro modified nucleotide, and the nucleotides at the remaining positions are selected from 2'-O-methyl modified nucleotides or 2'-O-methoxyethyl modified nucleotides;
optionally, in the duplex, in the direction from the 5' end to the 3' end, in the sense strand, at least three nucleotides of the nucleotides at positions 7-10 of the nucleotide sequence are selected from 2'-fluoro modified nucleotides, up to two of the nucleotides at positions 5, 12 and 18 are selected from 2'-O-methoxyethyl modified nucleotides, and the nucleotides at the remaining positions are selected from 2'-O-methyl modified nucleotides; and, in the antisense strand, the nucleotides at positions 2, 6, 14, and 16 of the nucleotide sequence are selected from 2'-fluoro modified nucleotides, any one of the nucleotides at positions 9-12 is selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides;
optionally, each nucleotide of the double-stranded oligonucleotide is independently selected from a modified nucleotide, wherein in the duplex, modifications of the sense and antisense strands are selected from any one of the following (1)-(8):

(1) in the direction from the 5' end to the 3' end, in the sense strand, the nucleotides at positions 7-10 of the nucleotide sequence are selected from 2'-fluoro modified nucleotides, and the nucleotides at the remaining positions are selected from 2'-O-methyl modified nucleotides; and in the antisense strand, the nucleotides at positions 2, 6, 9, 14, and 16 of the nucleotide sequence are selected from 2'-fluoro modified nucleotides, and the nucleotides at the remaining positions are selected from 2'-O-methyl modified nucleotides;
(2) in the direction from the 5' end to the 3' end, in the sense strand, the nucleotides at positions 7-10 of the nucleotide sequence are selected from 2'-fluoro modified nucleotides, and the nucleotides at the remaining positions are selected from 2'-O-methyl modified nucleotides; and in the antisense strand, the nucleotides at positions 2, 6, 12, 14 and 16 of the nucleotide sequence are selected from 2'-fluoro modified nucleotides, and the nucleotides at the remaining positions are selected from 2'-O-methyl modified nucleotides;
(3) in the direction from the 5' end to the 3' end, in the sense strand, the nucleotides at positions 7-10 of the nucleotide sequence are selected from 2'-fluoro modified nucleotides, and the nucleotides at the remaining positions are selected from 2'-O-methyl modified nucleotides; and in the antisense strand, the nucleotides at positions 2, 6, 9, 14 and 16 of the nucleotide sequence are selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides;
(4) in the direction from the 5' end to the 3' end, in the sense strand, the nucleotides at positions 7-10 of the nucleotide sequence are selected from 2'-fluoro modified nucleotides, and the nucleotides at the remaining positions are selected from 2'-O-methyl modified nucleotides; and in the antisense strand, the nucleotides at positions 2, 6, 12, 14 and 16 of the nucleotide sequence are selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides;
(5) in the direction from the 5' end to the 3' end, in the sense strand, the nucleotide at position 5, 12 or 18 of the nucleotide sequence is selected from a 2'-O-methoxyethyl modified nucleotide, the nucleotides at positions 7-10 are selected from 2'-fluoro modified nucleotides, and the nucleotides at the remaining positions are selected from 2'-O-methyl modified nucleotides; and in the antisense strand, the nucleotides at positions 2, 6, 9, 14 and 16 of the nucleotide sequence are selected from 2'-fluoro modified nucleotides, and the nucleotides at the remaining positions are selected from 2'-O-methyl modified nucleotides;
(6) in the direction from the 5' end to the 3' end, in the sense strand, the nucleotide at position 5, 12 or 18 of the nucleotide sequence is selected from a 2'-O-methoxyethyl modified nucleotide, the nucleotides at positions 7-10 are selected from 2'-fluoro modified nucleotides, and the nucleotides at the remaining positions are

selected from 2'-O-methyl modified nucleotides; and in the antisense strand, the nucleotides at positions 2, 6, 12, 14 and 16 of the nucleotide sequence are selected from 2'-fluoro modified nucleotides, and the nucleotides at the remaining positions are selected from 2'-O-methyl modified nucleotides;

(7) in the direction from the 5' end to the 3' end, in the sense strand, the nucleotide at position 5, 12 or 18 of the nucleotide sequence is selected from a 2'-O-methoxyethyl modified nucleotide, the nucleotides at positions 7-10 are selected from 2'-fluoro modified nucleotides, and the nucleotides at the remaining positions are selected from 2'-O-methyl modified nucleotides; and in the antisense strand, the nucleotides at positions 2, 6, 9, 14 and 16 of the nucleotide sequence are selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides; and

(8) in the direction from the 5' end to the 3' end, in the sense strand, the nucleotide at position 5, 12, or 18 of the nucleotide sequence is selected from a 2'-O-methoxyethyl modified nucleotide, the nucleotides at positions 7-10 are selected from 2'-fluoro modified nucleotides, and the nucleotides at the remaining positions are selected from 2'-O-methyl modified nucleotides; and in the antisense strand, the nucleotides at positions 2, 6, 12, **14** and 16 of the nucleotide sequence are selected from 2'-fluoro modified nucleotides, the nucleotide at position 15 is selected from a 2'-O-methoxyethyl modified nucleotide, and the nucleotides at the remaining positions are selected from 2'-O-methyl modified nucleotides;

optionally, in the duplex, in the direction from the 5' end to the 3' end, the sense strand comprises 1 or 2 consecutive phosphorothioate linkages between the terminal nucleotides at the 5' terminus; and/or, the antisense strand independently comprises 1 or 2 consecutive phosphorothioate linkages at the 5' terminus and the 3' terminus, respectively.

**5.** The nucleic acid chimera according to any one of claims 1-3, wherein the nucleotide unit I has a structure represented by formula (102):

$$H-\left(M_1\right)_{j_1}-Y_1-\left[\left(M_1\right)_{j_2}-Y_2\right]_k-\left(M_1\right)_{j_3}-H$$

(102) ;

wherein each substituent is as defined in any one of claims 1-3.

**6.** The nucleic acid chimera according to any one of claims 1-3, wherein the nucleotide unit I has a structure represented by formula (103):

$$H-\left(M_1-M_2\right)_{j_1}-Y_1-\left[\left(M_1-M_2\right)_{j_2}-Y_2\right]_k-\left(M_1-M_2\right)_{j_3}-H$$

(103) ;

wherein each substituent is as defined in any one of claims 1-3.

**7.** The nucleic acid chimera according to claim 1, wherein each $M_1$ is independently selected from a ligand capable of binding to the asialoglycoprotein receptor.

**8.** The nucleic acid chimera according to any one of claims 1-7, wherein each $M_1$ is independently selected from a structure represented by formula (201a) or formula (201b), or an isomer thereof, or a pharmaceutically acceptable salt thereof:

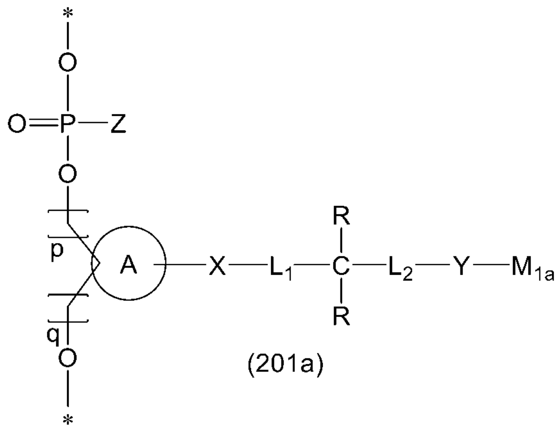

(201a)

, 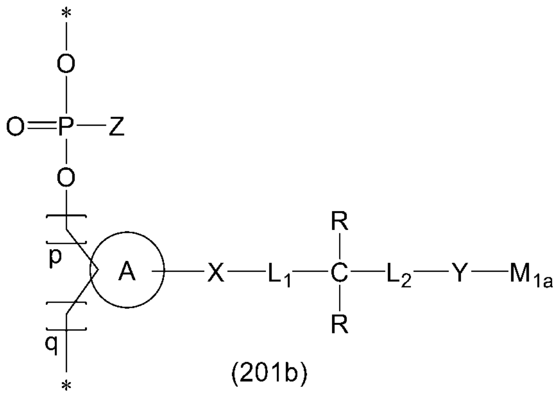

(201b)

wherein, when $M_1$ is located at a terminal position of the nucleotide unit I, $M_1$ is selected from the structure represented by formula (201a), or an isomer thereof, or a pharmaceutically acceptable salt thereof; and when $M_1$ is not located at a terminal position of the nucleotide unit I, $M_1$ is selected from the structure represented by formula (201b), or an isomer thereof, or a pharmaceutically acceptable salt thereof; and

wherein A is selected from an optionally substituted 4- to 10-membered alicyclic ring;

X is selected from NH, O or S;

$L_1$ is selected from

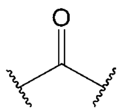, 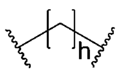, or 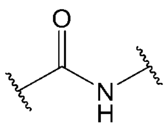;

wherein h is selected from 1, 2, 3, 4, 5, 6, 7, 8, 9, or 10;

each R is independently selected from H, optionally substituted $C_{1-6}$ alkyl, or optionally substituted $C_{1-6}$ alkoxy;

$L_2$ is selected from optionally substituted $C_2$-$C_{20}$ alkylene or

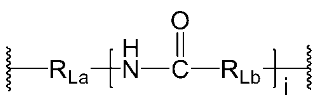;

and, $R_{La}$ and $R_{Lb}$ are independently selected from optionally substituted $C_{1-10}$ alkylene, and i is selected from 1, 2, 3, 4, or 5;

Y is selected from O or S;

$M_{1a}$ is selected from galactose or a derivative thereof;

Z is selected from hydroxyl or sulfhydryl;

p is selected from 1, 2, or 3;

q is selected from 1, 2, or 3;

optionally, A is selected from

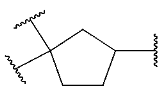, 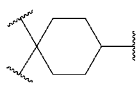, 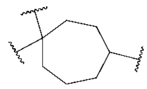, 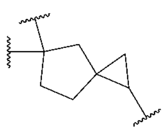,

or

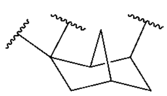;

optionally, A is selected from

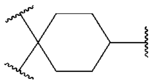;

optionally, X is selected from NH;
optionally, $L_1$ is selected from

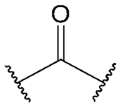;

optionally, each R is independently selected from H;
optionally, $L_2$ is selected from $C_{1-10}$ alkylene or

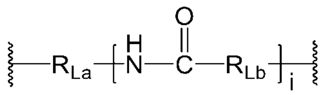;

wherein $R_{La}$ and $R_{La}$ are independently selected from $C_{1-5}$ alkylene, and i is 1, 2, or 3;
optionally, i is selected from 1;
optionally, each $L_2$ is independently selected from

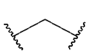, 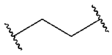, 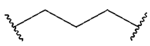, 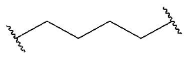,

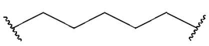, 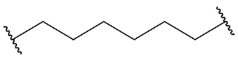, 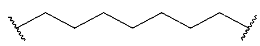, 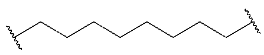,

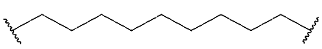, 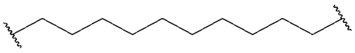, 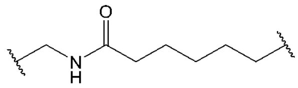,

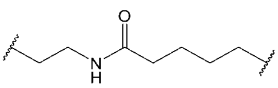, 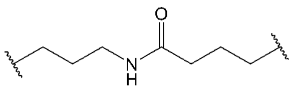, 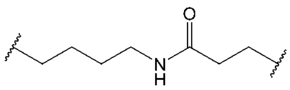, or

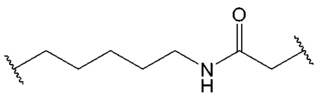;

optionally, Y is selected from O;
optionally, $M_{1a}$ is selected from a galactosamine derivative;
optionally, $M_{1a}$ is selected from

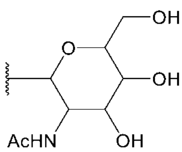;

optionally, Z is selected from hydroxyl;
optionally, p is selected from 1;

optionally, q is selected from 1.

**9.** The nucleic acid chimera according to claim 8, wherein each $M_1$ is independently selected from a structure represented by formula (202a) or formula (202b), or an isomer thereof, or a pharmaceutically acceptable salt thereof:

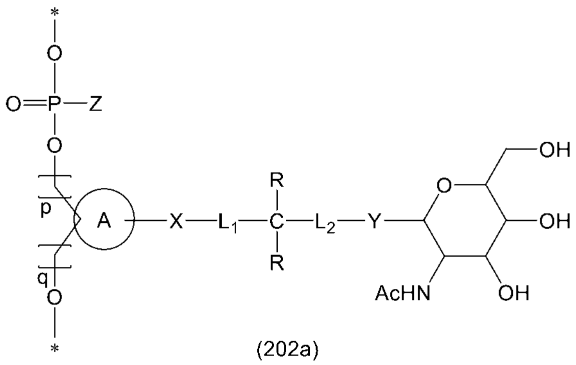

(202a) ,

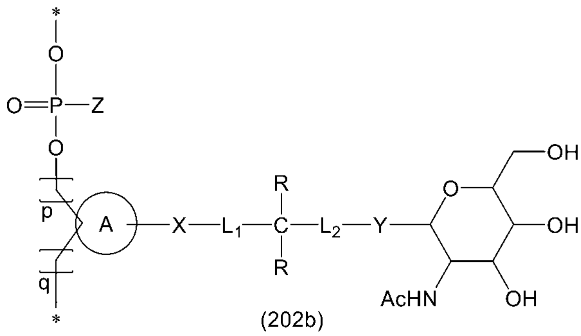

(202b) ;

wherein, when $M_1$ is located at a terminal position of the nucleotide unit I, $M_1$ is selected from the structure represented by formula (202a), or an isomer thereof, or a pharmaceutically acceptable salt thereof; and when $M_1$ is not located at a terminal position of the nucleotide unit I, $M_1$ is selected from the structure represented by formula (202b), or an isomer thereof, or a pharmaceutically acceptable salt thereof.

**10.** The nucleic acid chimera according to claim 8, wherein each $M_1$ is independently selected from a structure represented by formula (203a) or formula (203b), or an isomer thereof, or a pharmaceutically acceptable salt thereof:

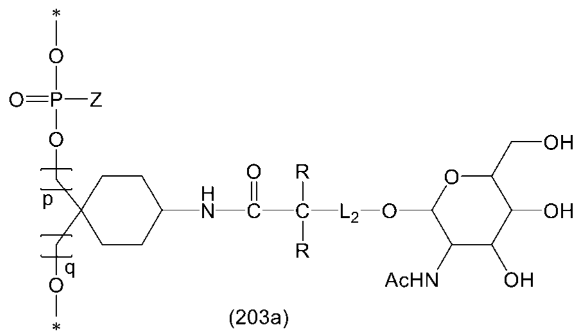

(203a) ,

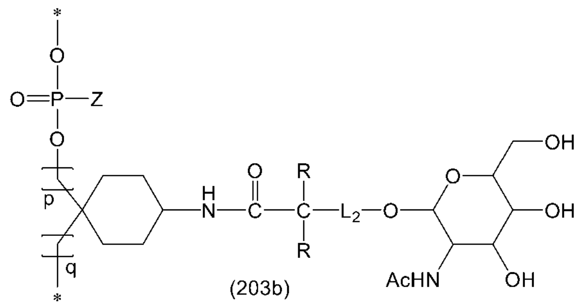

(203b) ;

wherein, when $M_1$ is located at a terminal position of the nucleotide unit I, $M_1$ is selected from the structure represented by formula (203a), or an isomer thereof, or a pharmaceutically acceptable salt thereof; and when $M_1$ is not located at a terminal position of the nucleotide unit I, $M_1$ is selected from the structure represented by formula (203b), or an isomer thereof, or a pharmaceutically acceptable salt thereof.

**11.** The nucleic acid chimera according to claim 8, wherein each $M_1$ is independently selected from any one of the following structure, or an isomer thereof, or a pharmaceutically acceptable salt thereof:

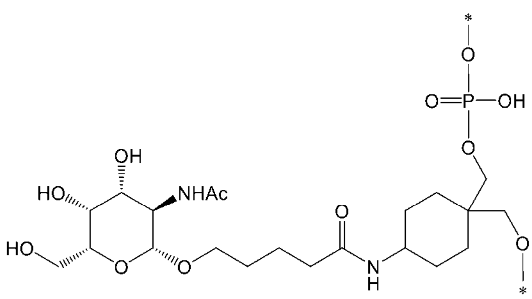

,

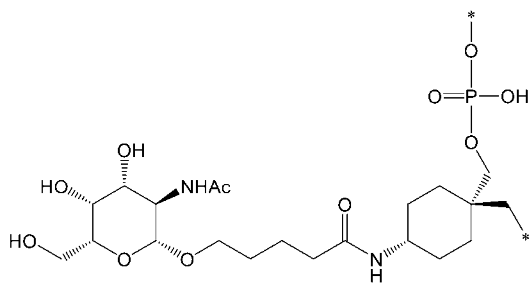

,

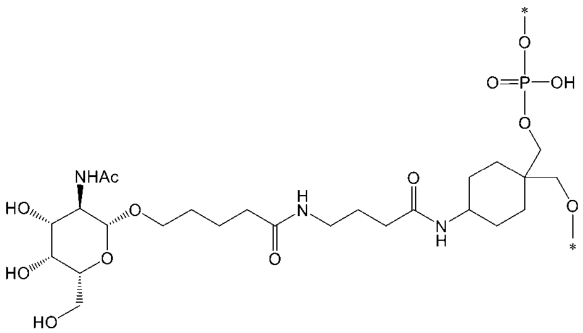,
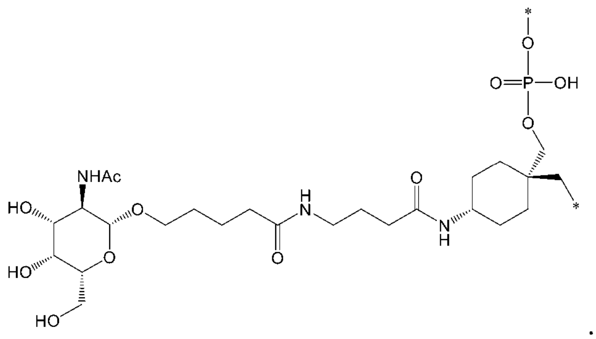.

wherein, when $M_1$ is located at a terminal position of the nucleotide unit I, $M_1$ is selected from

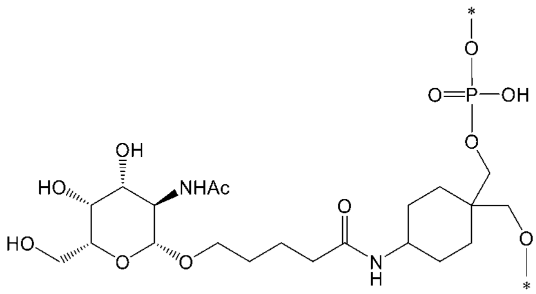

or an isomer thereof or a pharmaceutically acceptable salt thereof, or

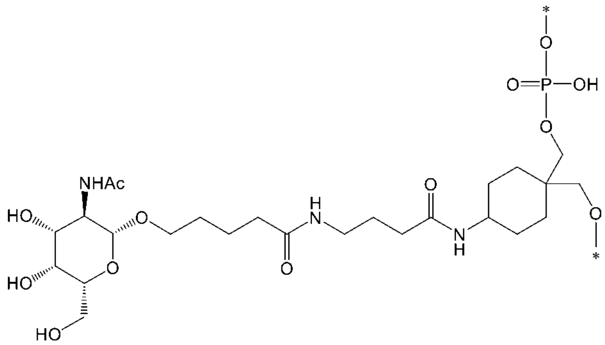

or an isomer thereof or a pharmaceutically acceptable salt thereof;
when $M_1$ is not located at a terminal position of the nucleotide unit I, $M_1$ is selected from

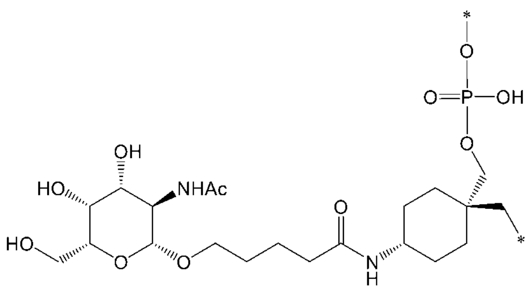

or an isomer thereof or a pharmaceutically acceptable salt thereof, or

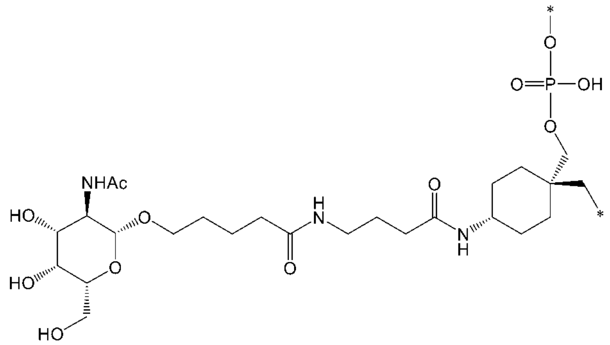

or an isomer thereof or a pharmaceutically acceptable salt thereof.

**12.** The nucleic acid chimera according to claim 1, wherein the nucleotide unit I has a structure represented by formula (104) or an isomer thereof or a pharmaceutically acceptable salt thereof:

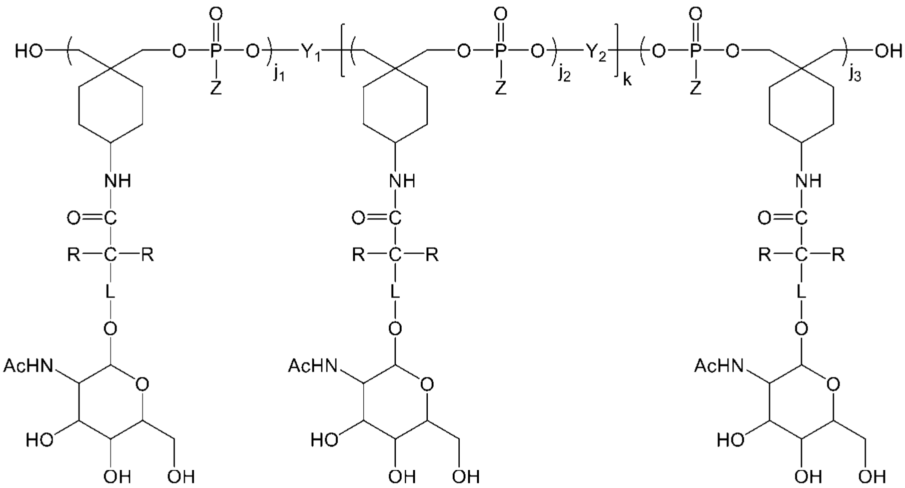

(104)

,

wherein each substituent is as defined in claims 8-11.

**13.** The nucleic acid chimera according to any one of claims 1-12, wherein the nucleic acid chimera has a structure represented by formula (105) or an isomer thereof or a pharmaceutically acceptable salt thereof:

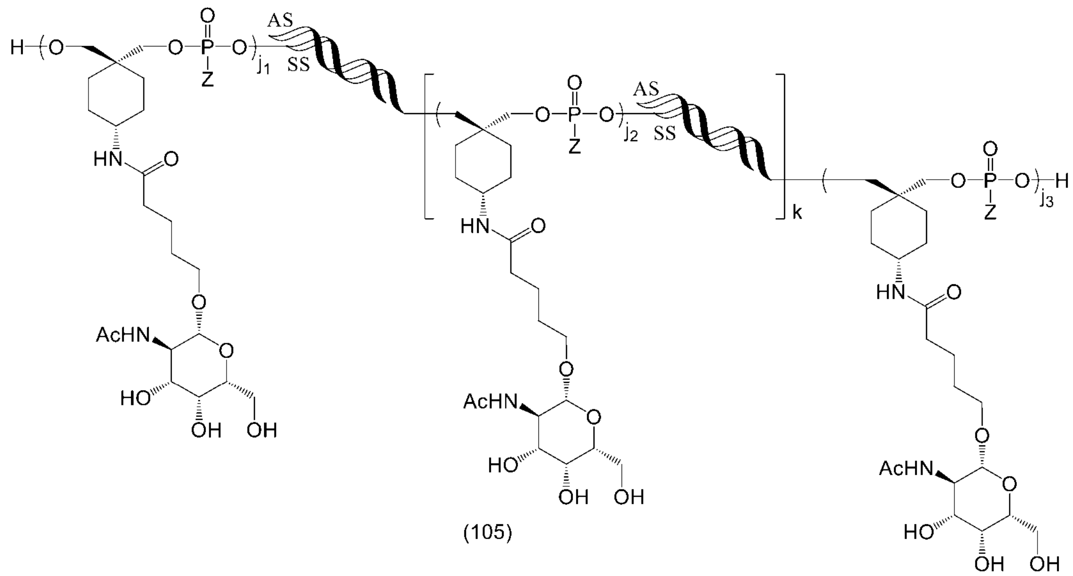

(105)

;

preferably, Z is OH;
wherein AS represents an antisense strand of a double-stranded oligonucleotide molecule; and SS represents a sense strand of the double-stranded oligonucleotide molecule.

**14.** The nucleic acid chimera according to any one of claims 1-13, wherein the nucleic acid chimera has a structure represented by formula (106) or an isomer thereof or a pharmaceutically acceptable salt thereof:

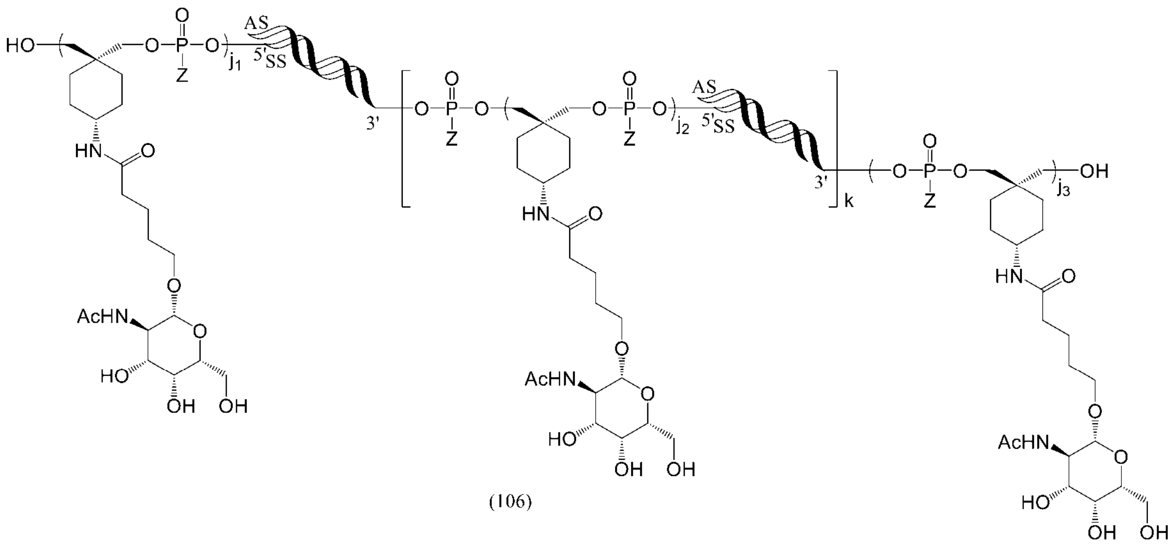

(106)

wherein AS represents an antisense strand of a double-stranded oligonucleotide molecule; and SS represents a sense strand of a double-stranded oligonucleotide;
Z is OH; and
optionally, $j_1$ is selected from 0 or 1, $j_2$ is selected from an integer of 1-3, and k is selected from 1.

**15.** A nucleic acid chimera, comprising a nucleotide unit III having a structure represented by formula (301) or an isomer thereof or a pharmaceutically acceptable salt thereof:

$$HO-(M_1)_{j_4}-Y_3-(M_1)_{j_5}-Dt-(M_1)_{j_6}-Y_4-(M_1)_{j_7}-OH$$

(301)

wherein, each $M_1$ is independently selected from a structure represented by formula (201b) or an isomer thereof or a pharmaceutically acceptable salt thereof:

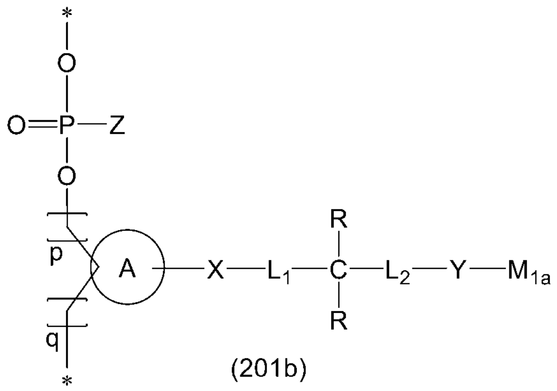

(201b)

,

wherein, A, X, $L_1$, R, $L_2$, Y, $M_{1a}$, p, and q are as defined in any one of claims 1-14; and
wherein, $Y_3$ is selected from a sense strand and $Y_4$ is selected from a sense strand; alternatively, $Y_3$ is selected from an antisense strand and $Y_4$ is selected from a sense strand;
$Y_3$ and $Y_4$ in the nucleotide unit III correspond to the same target gene or different target genes; and the corresponding relationship means that the sense strand is identical or substantially identical to a segment of consecutive nucleotides in the mRNA of the target gene, or the antisense strand is complementary or substantially complementary to a segment of consecutive nucleotides in the mRNA of the target gene;
$j_4$, $j_5$, $j_6$, and $j_7$ are each independently selected from integers of 0-3, and at least one of $j_6$ and $j_7$ is not 0; and when $Y_3$ is selected from the sense strand, at least one of $j_4$ and $j_5$ is not 0;
wherein, when $Y_3$ is selected from the sense strand and $Y_4$ is selected from the sense strand, the 5' terminus of $Y_3$ is conjugated to the 5' terminus of $Y_4$, or the 3' terminus of $Y_3$ is conjugated to the 3' terminus of $Y_4$; and, Dt has a structure represented by formula (401) or a stereoisomer thereof or a tautomer thereof:

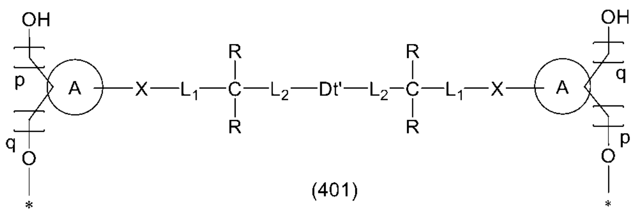

(401)

wherein, A, X, $L_1$, R, $L_2$, p, and q are as defined in any one of claims 1-14; and
Dt' is selected from

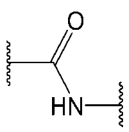, 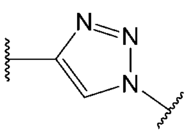, 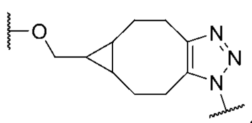, or 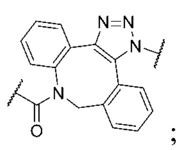;

when $Y_3$ is selected from the antisense strand and $Y_4$ is selected from the sense strand, the antisense strand $Y_3$ and the sense strand $Y_4$ in the nucleotide unit III do not form base complementary pairing; the 5' terminus of $Y_3$ is conjugated to the 3' terminus of $Y_4$, or the 3' terminus of $Y_3$ is conjugated to the 5' terminus of $Y_4$; and Dt has a structure represented by formula (501) or a stereoisomer thereof or a tautomer thereof:

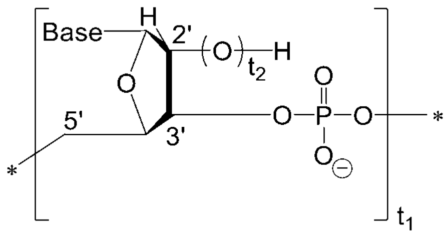

(501)

wherein, $t_1$ is selected from an integer of 0-5, $t_2$ is independently selected from 0 or 1, and Base is selected from a nucleobase A, U, G, C, or T; and when $t_1$ is 0, $j_6$ is not 0.

**16.** The nucleic acid chimera according to claim 15, wherein,

when $Y_3$ is selected from the sense strand and $Y_4$ is selected from the sense strand, Dt has a structure represented by formula (402) or a stereoisomer thereof or a tautomer thereof:

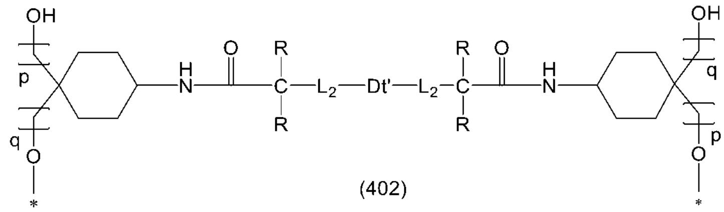

(402)

;

optionally, Dt' is selected from

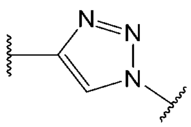

;

optionally, when $Y_3$ is selected from the sense strand and $Y_4$ is selected from the sense strand, Dt has a structure represnted by formula (403) or a stereoisomer thereof or a tautomer thereof:

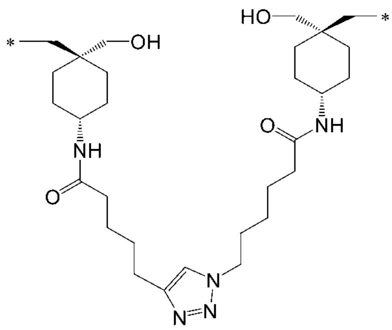

(403)

;

optionally, when $Y_3$ is selected from the sense strand and $Y_4$ is selected from the sense strand, the nucleotide unit III has a structure represented by formula (301a) or an isomer thereof or a pharmaceutically acceptable salt thereof:

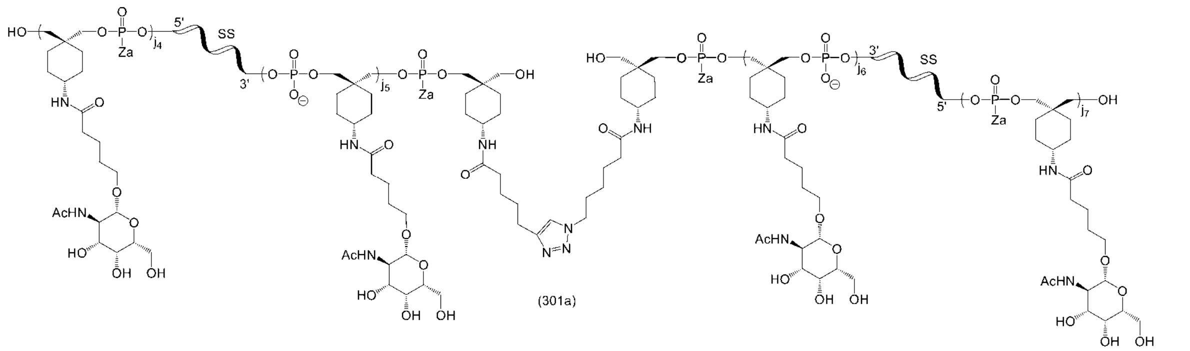

(301a)

wherein, each Za is independently selected from hydroxyl or sulfhydryl;

optionally, when $Y_3$ is selected from the sense strand and $Y_4$ is selected from the sense strand, the nucleic acid chimera further comprises two antisense strands, a nucleotide sequence of one antisense strand is at least partially reverse-complementary to the nucleotide sequence of the sense strand $Y_3$ in the nucleotide unit III, and a nucleotide sequence of the other antisense strand is at least partially reverse-complementary to the nucleotide sequence of the sense strand $Y_4$ in the nucleotide unit III.

**17.** The nucleic acid chimera according to claim 15, wherein, when $Y_3$ is selected from the antisense strand and $Y_4$ is selected from the sense strand, the nucleotide unit III has a structure represented by formula (301b) or an isomer thereof or a pharmaceutically acceptable salt thereof:

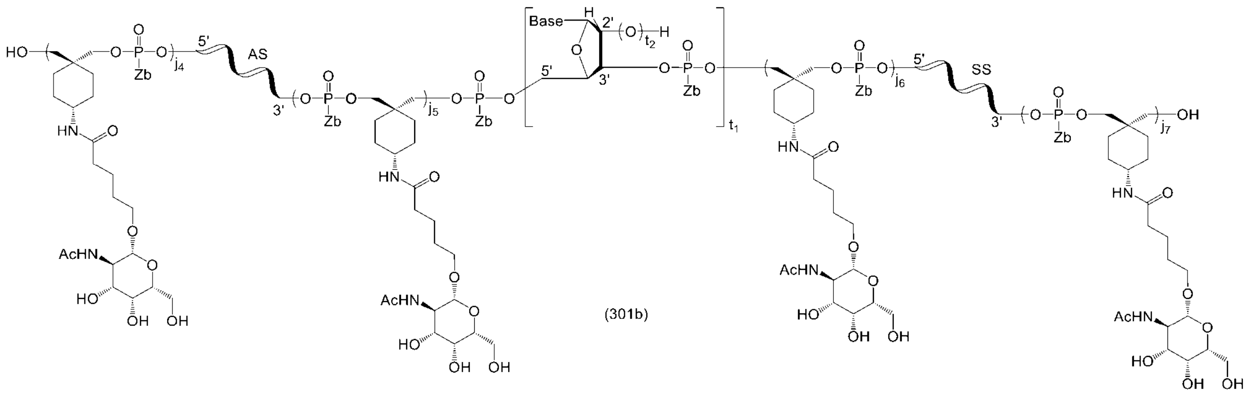

(301b)

wherein each Zb is independently selected from hydroxyl or sulfhydryl;

the antisense strand $Y_3$ and the sense strand $Y_4$ in the nucleotide unit III correspond to the same target gene or different target genes, and the antisense strand $Y_3$ and the sense strand $Y_4$ in the nucleotide unit III do not form base complementary pairing;

optionally, when $Y_3$ is selected from the antisense strand and $Y_4$ is selected from the sense strand, the nucleotide unit III has a structure reporesented by formula (302b) or an isomer thereof or a pharmaceutically acceptable salt thereof:

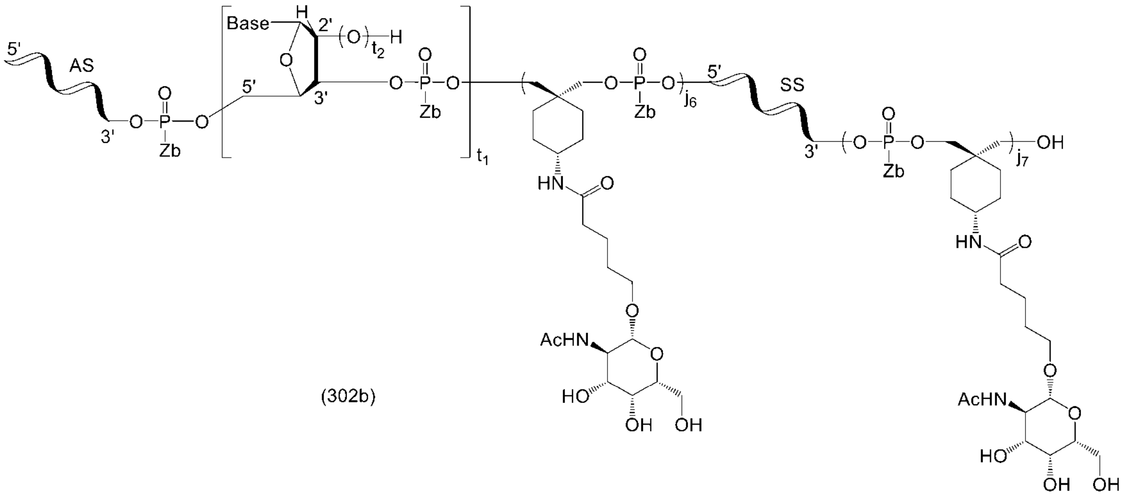

(302b)

wherein, each Zb is independently selected from hydroxyl or sulfhydryl; and

the antisense strand $Y_3$ and the sense strand $Y_4$ in the nucleotide unit III correspond to the same target gene or different target genes, and the antisense strand $Y_3$ and the sense strand $Y_4$ in the nucleotide unit III do not form base complementary pairing;

optionally, when $Y_3$ is selected from the antisense strand and $Y_4$ is selected from the sense strand, the nucleic acid chimera comprises two nucleotide units III, wherein the antisense strand $Y_3$ and the sense strand $Y_4$ in the same nucleotide unit III correspond to the same target gene or different target genes, and the antisense strand $Y_3$ and the sense strand $Y_4$ in the same nucleotide unit III do not form base complementary pairing;

the nucleotide sequence of the antisense strand $Y_3$ in a first nucleotide unit III is at least partially reverse-complementary to the nucleotide sequence of the sense strand $Y_4$ in a second nucleotide unit III; and

the nucleotide sequence of the sense strand $Y_4$ in the first nucleotide unit III is at least partially reverse-complementary to the nucleotide sequence of the antisense strand $Y_3$ in the second nucleotide unit III;

optionally, when $Y_3$ is selected from the antisense strand and $Y_4$ is selected from the sense strand, the nucleic acid chimera further comprises one sense strand and one antisense strand, wherein a nucleotide sequence of the sense strand is at least partially reverse-complementary to the nucleotide sequence of the antisense strand $Y_3$ in the nucleotide unit III; and a nucleotide sequence of the antisense strand is at least partially reverse-complementary to the nucleotide sequence of the sense strand $Y_4$ in the nucleotide unit III.

**18.** A composition, comprising the nucleic acid chimera according to any one of claims 1-17 and a physiologically acceptable excipient.

**19.** The nucleic acid chimera according to any one of claims 1-17 for use in treating a disease or condition caused by dysregulation of expression of at least one gene.

**20.** Use of the nucleic acid chimera according to any one of claims 1-17 in the preparation of a medicament for treating a disease or condition caused by dysregulation of expression of at least one gene.

**21.** A method of treating a disease or condition, comprising administering the nucleic acid chimera according to any one of claims 1-17 to an individual in need thereof.

**22.** The method according to claim 21, wherein the nucleic acid chimera is administered to the individual subcutaneously or intravenously.

**23.** The method according to claim 21 or 22, wherein after *in vivo* administration, the nucleic acid chimera is cleaved to give at least two independent double-stranded oligonucleotide molecules, each targeting a portion of mRNA transcribed from one or more target genes, and the target genes may be the same or different.

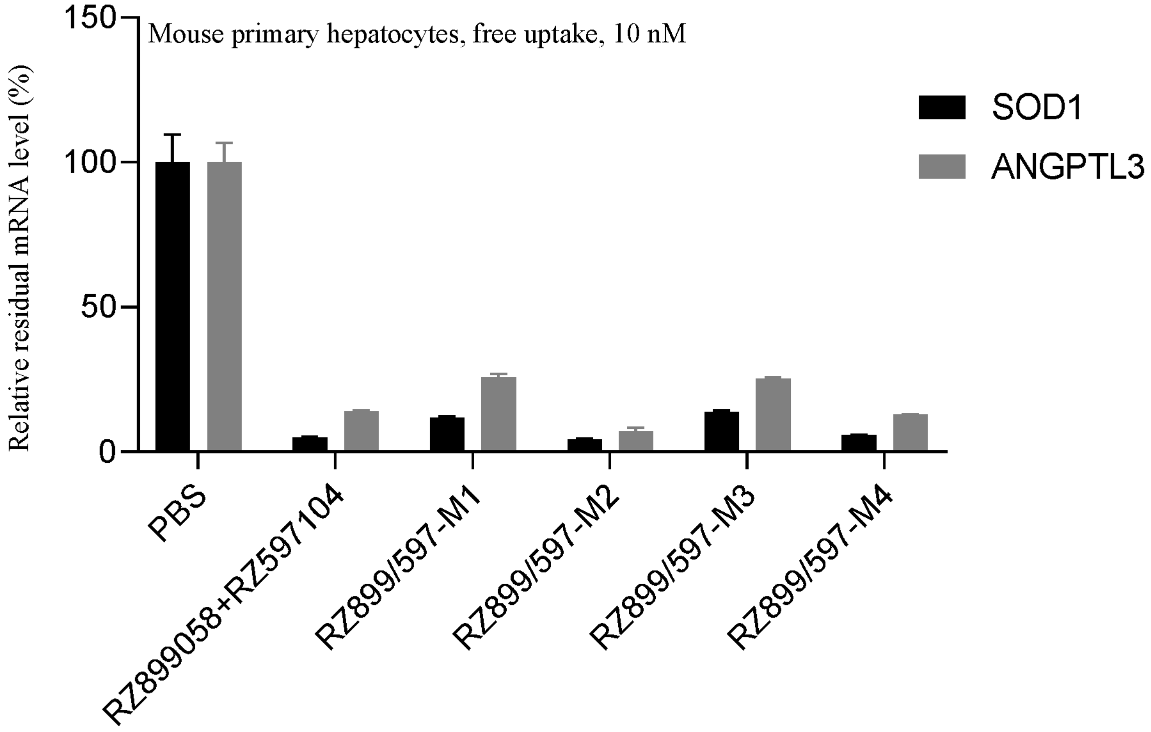
150
100
50
0
Mouse primary hepatocytes, free uptake, 10 nM
Relative residual mRNA level (%)
SOD1
ANGPTL3
PBS
RZ899058+RZ597104
RZ899/597-M1
RZ899/597-M2
RZ899/597-M3
RZ899/597-M4

Fig. 1

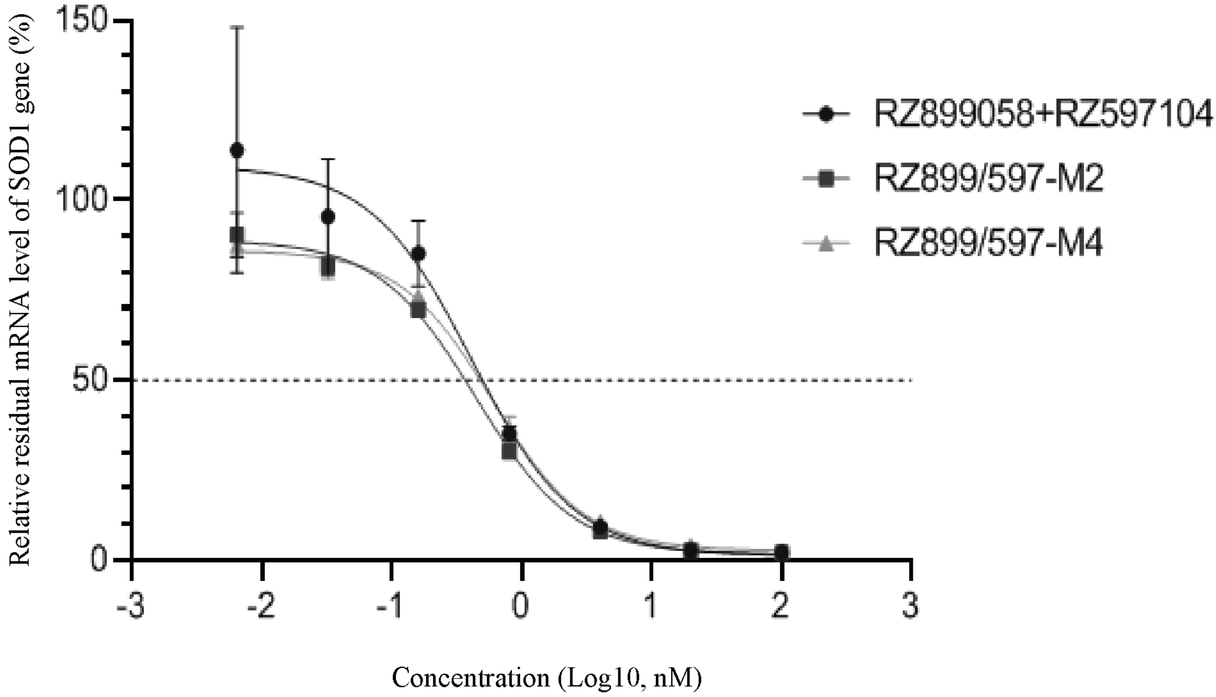
150
100
50
0
-3
-2
-1
0
1
2
3
Relative residual mRNA level of SOD1 gene (%)
RZ899058+RZ597104
RZ899/597-M2
RZ899/597-M4
Concentration (Log10, nM)

Fig. 2

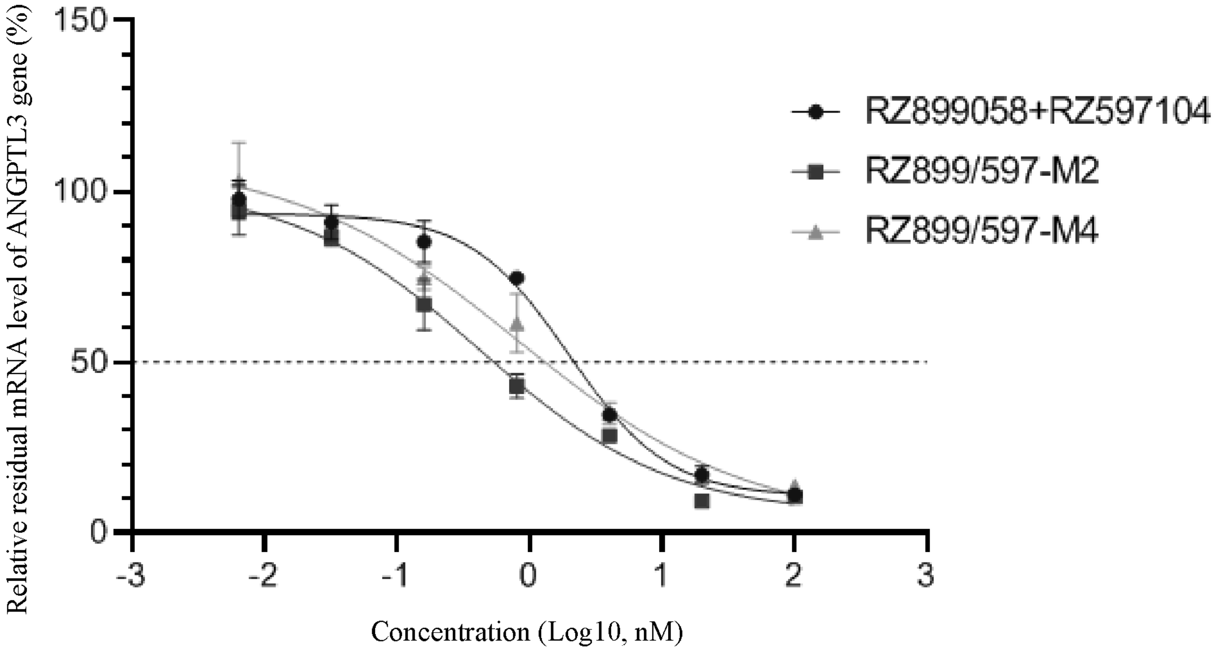

Relative residual mRNA level of ANGPTL3 gene (%)
150
100
50
0
-3
-2
-1
0
1
2
3
Concentration (Log10, nM)
RZ899058+RZ597104
RZ899/597-M2
RZ899/597-M4


Fig. 3

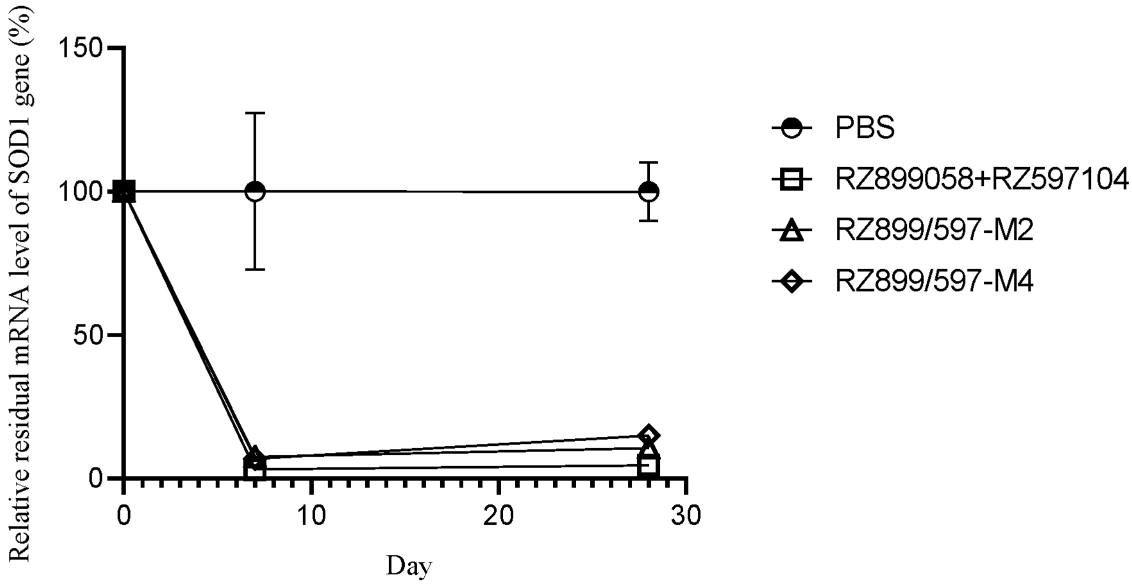

Relative residual mRNA level of SOD1 gene (%)
150
100
50
0
0
10
20
30
Day
PBS
RZ899058+RZ597104
RZ899/597-M2
RZ899/597-M4


Fig. 4

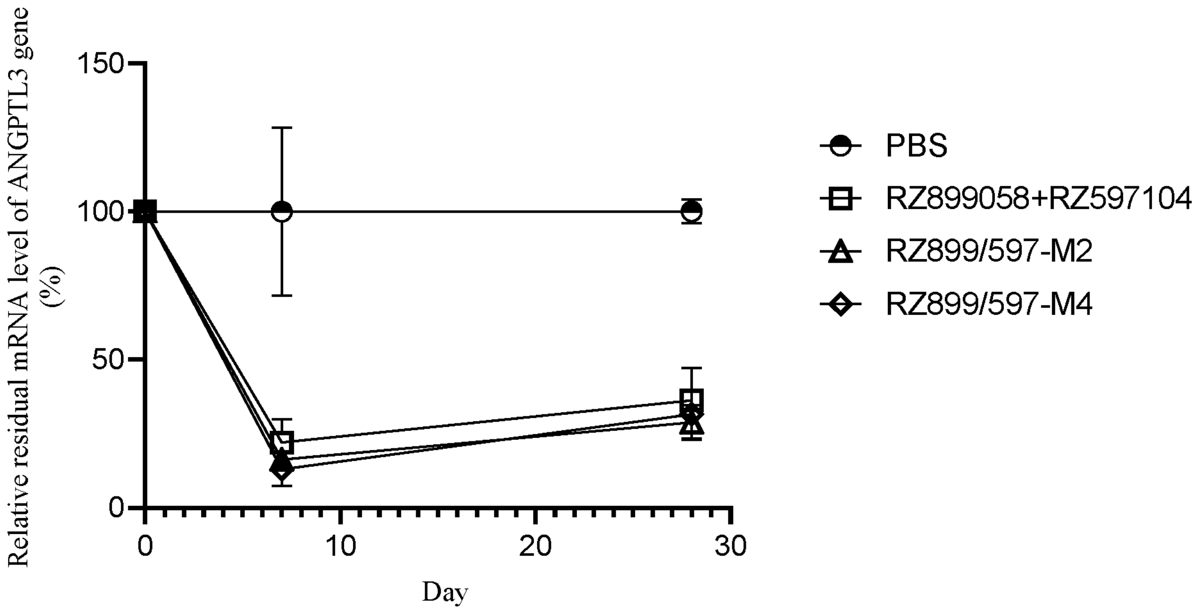

Relative residual mRNA level of ANGPTL3 gene (%)
150
100
50
0
0
10
20
30
Day
PBS
RZ899058+RZ597104
RZ899/597-M2
RZ899/597-M4


Fig. 5

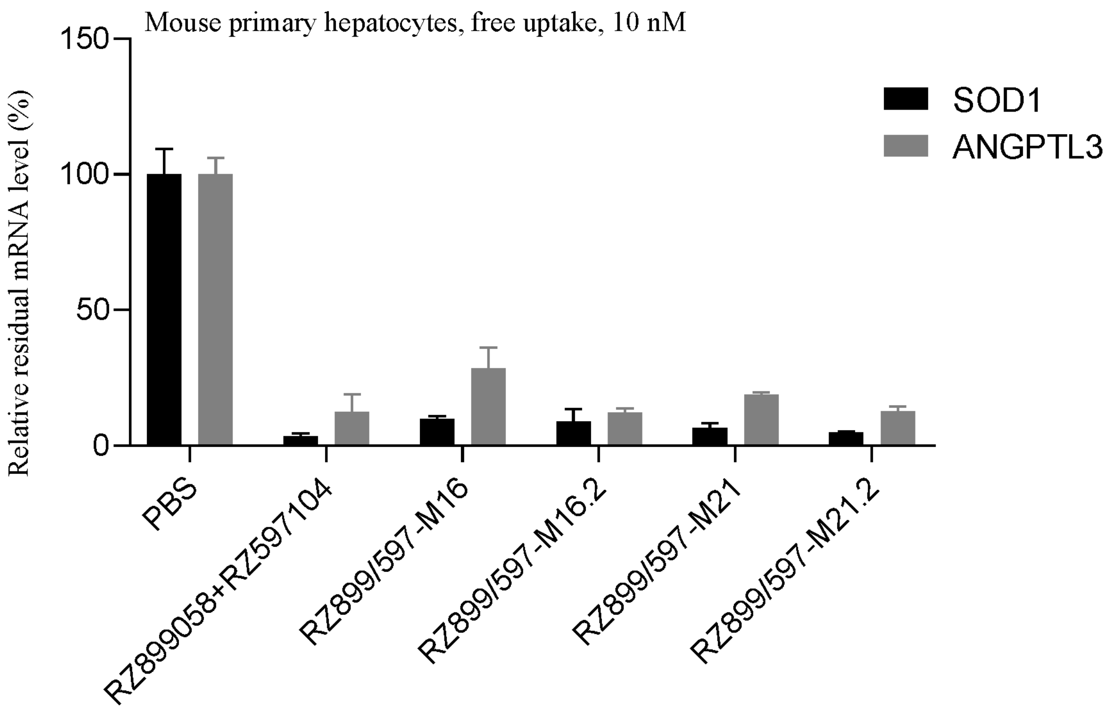

Mouse primary hepatocytes, free uptake, 10 nM
Relative residual mRNA level (%)
150
100
50
0
SOD1
ANGPTL3
PBS
RZ899058+RZ597104
RZ899/597-M16
RZ899/597-M16.2
RZ899/597-M21
RZ899/597-M21.2


Fig. 6

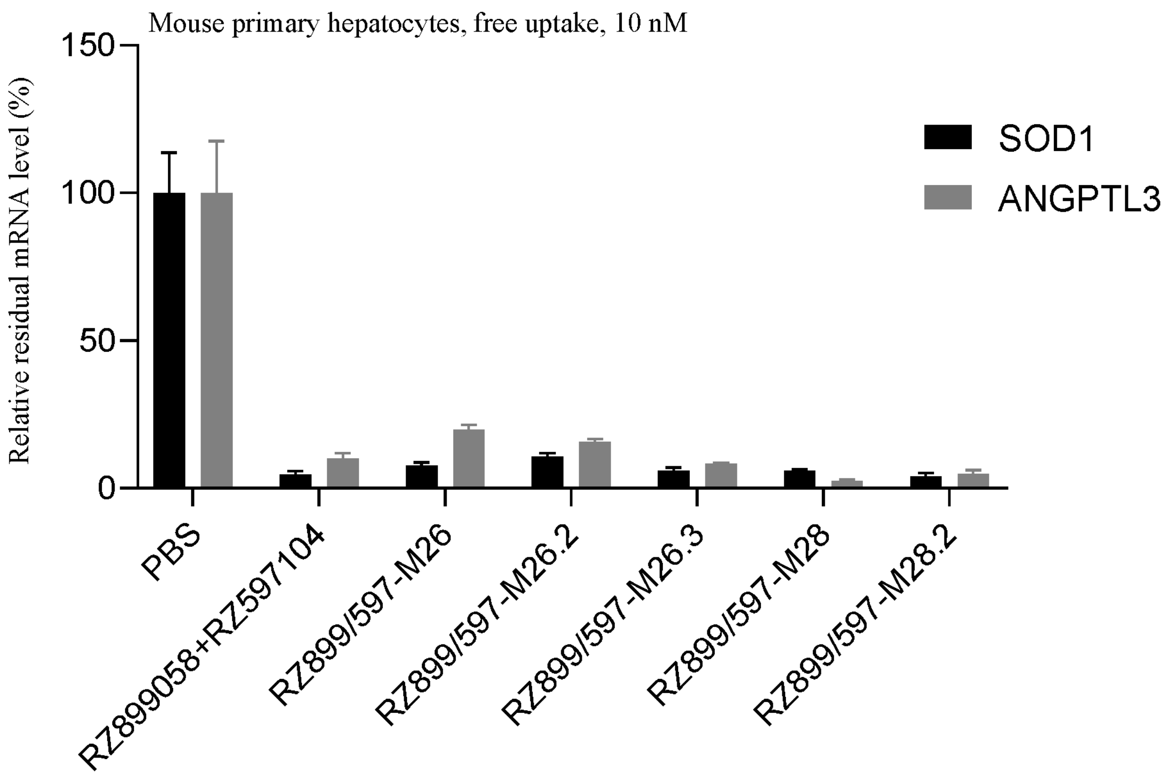
Mouse primary hepatocytes, free uptake, 10 nM
Relative residual mRNA level (%)
150
100
50
0
SOD1
ANGPTL3
PBS
RZ899058+RZ597104
RZ899/597-M26
RZ899/597-M26.2
RZ899/597-M26.3
RZ899/597-M28
RZ899/597-M28.2

Fig. 7

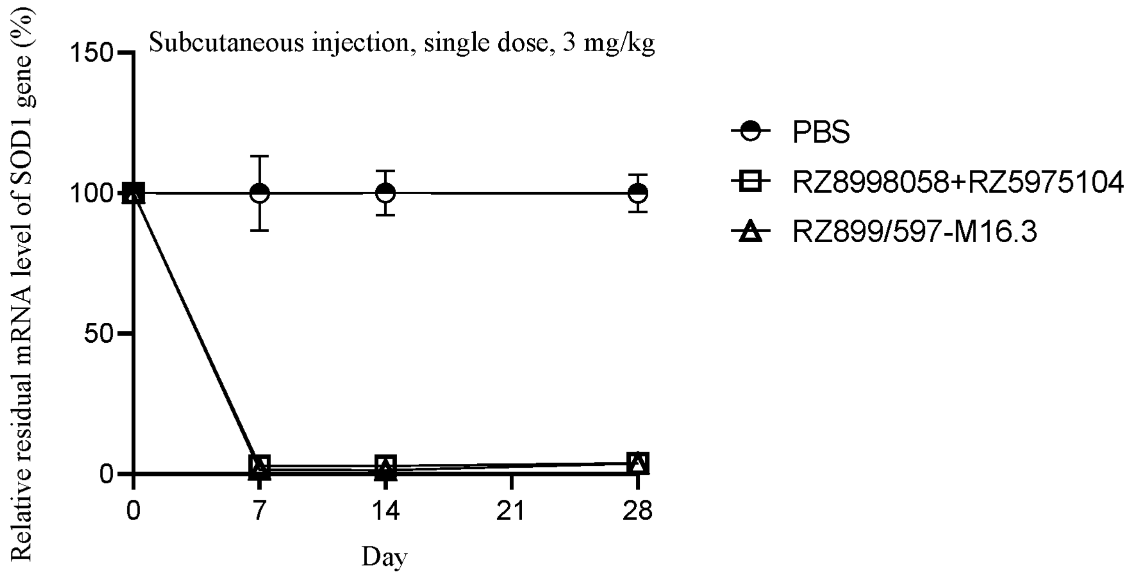
Subcutaneous injection, single dose, 3 mg/kg
Relative residual mRNA level of SOD1 gene (%)
150
100
50
0
0
7
14
21
28
Day
PBS
RZ8998058+RZ5975104
RZ899/597-M16.3

Fig. 8

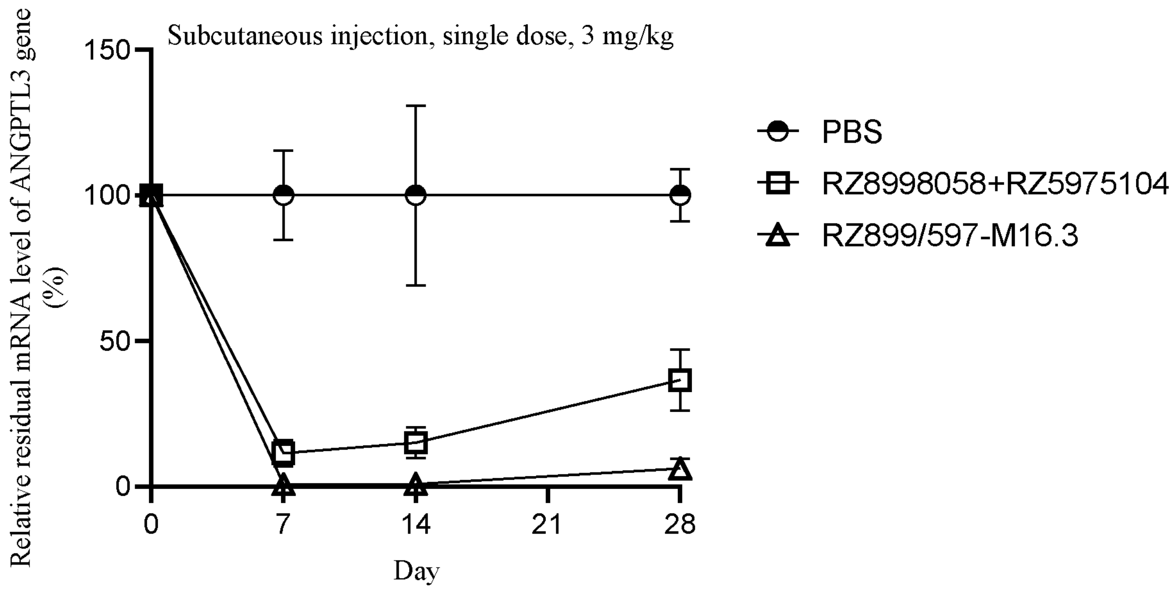
Subcutaneous injection, single dose, 3 mg/kg
Relative residual mRNA level of ANGPTL3 gene (%)
150
100
50
0
0
7
14
21
28
Day
PBS
RZ8998058+RZ5975104
RZ899/597-M16.3

Fig. 9

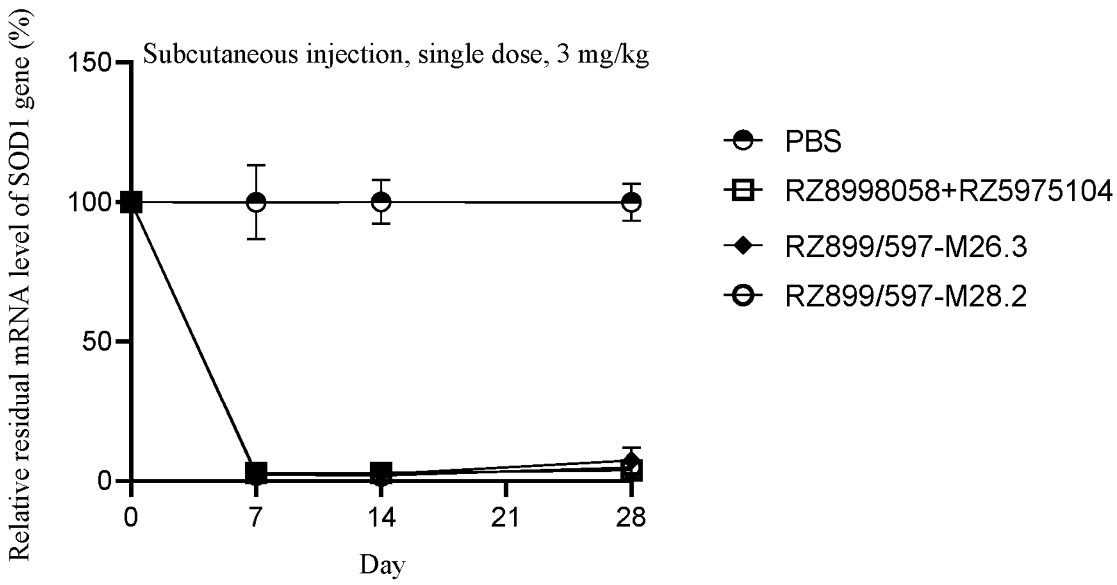
Subcutaneous injection, single dose, 3 mg/kg
Relative residual mRNA level of SOD1 gene (%)
150
100
50
0
0
7
14
21
28
Day
PBS
RZ8998058+RZ5975104
RZ899/597-M26.3
RZ899/597-M28.2

Fig. 10

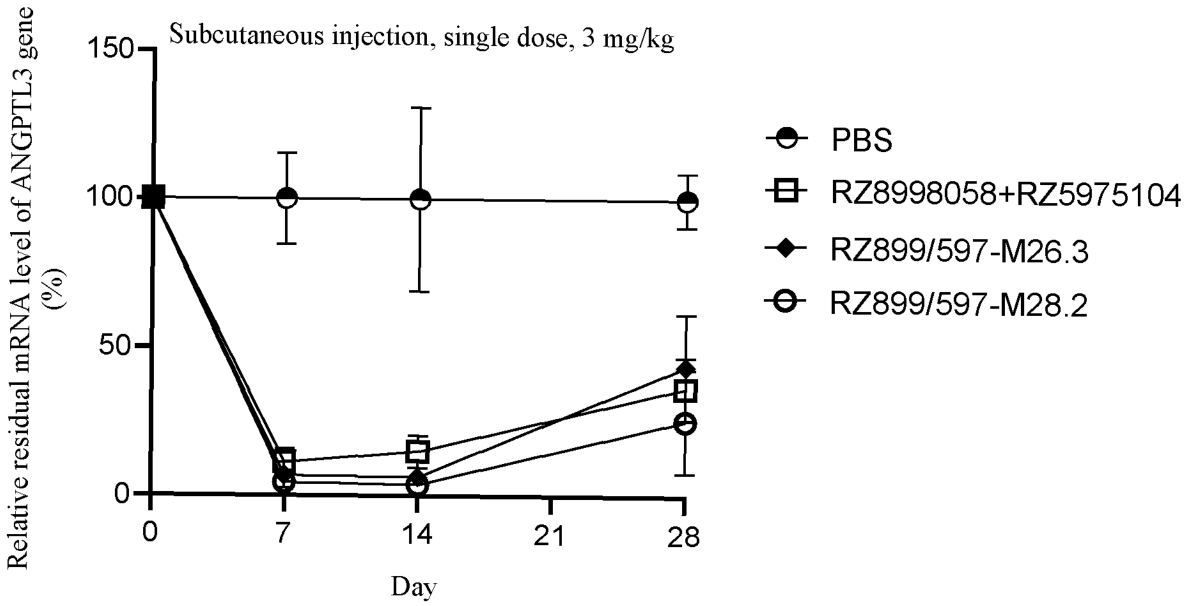
Subcutaneous injection, single dose, 3 mg/kg
Relative residual mRNA level of ANGPTL3 gene (%)
150
100
50
0
0
7
14
21
28
Day
PBS
RZ8998058+RZ5975104
RZ899/597-M26.3
RZ899/597-M28.2

Fig. 11

**INTERNATIONAL SEARCH REPORT**

International application No.
**PCT/CN2024/118774**

**A. CLASSIFICATION OF SUBJECT MATTER**

C12N 15/113(2010.01)i; A61K 31/713(2006.01)i; A61P 1/16(2006.01)i; C07H 21/02(2006.01)i; A61K 47/54(2017.01)i

According to International Patent Classification (IPC) or to both national classification and IPC

**B. FIELDS SEARCHED**

Minimum documentation searched (classification system followed by classification symbols)

IPC: C12N A61K A61P C07H

Documentation searched other than minimum documentation to the extent that such documents are included in the fields searched

Electronic data base consulted during the international search (name of data base and, where practicable, search terms used)

CNABS; CNTXT; DWPI; VEN; ENTXTC; ENTXT; WPABS; CNKI; VCN; CJFD; 万方, WANFANG; ISI Web of Science; 读秀学术, Duxiu Scholar; Springer; NCBI; ELiviser; 中国专利生物序列检索系统, China Patent Biological Sequence Search System; STNext: 炫景瑞医药, 炫景生物, 核苷酸, 寡核苷酸, 小核酸, 嵌合体, 正义链, 反义链, N-乙酰半乳糖胺, 人血管生成素样蛋白3, 多, 三, 双, 两, 四, 靶点, 插入, 配体, 连接子, rigerna therapeutics, nucleotides, Oligonucleotides, ASO, small nucleic acid, siRNA, chimeras, sense strand, antisense strand, N-acetylgalactosamine, GalNAc, ANGPTL3, SOD1, multiple, Tri, double, two, Tetra, target, ligand, linker

**C. DOCUMENTS CONSIDERED TO BE RELEVANT**

| Category* | Citation of document, with indication, where appropriate, of the relevant passages | Relevant to claim No. |
|---|---|---|
| X | WO 2023283403 A2 (ALNYLAM PHARMACEUTICALS, INC.) 12 January 2023 (2023-01-12)<br>description, paragraphs [0021], [0023], [0033], [0034], [0054], [0077], [0088], [0091], [0112] and [1082] | 1-7, 18-23 |
| X | Lidya Salim et al. "Synthesis of Folate-labeled siRNAs from a Folate Derivative Phosphoramidite"<br>*Organic & Biomolecular Chemistry*, Vol. 21, No. (16), 28 April 2023 (2023-04-28), pages 3365-3372<br>page 3367, right-hand column, paragraph 3, and Scheme 3 | 1-3, 5, 6, 18-23 |
| A | WO 2023283403 A2 (ALNYLAM PHARMACEUTICALS, INC.) 12 January 2023 (2023-01-12)<br>description, paragraphs [0021], [0023], [0033], [0034], [0054], [0077], [0088], [0091], [0112] and [1082] | 8-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 December 2024** | **18 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/118774**

**C. DOCUMENTS CONSIDERED TO BE RELEVANT**

| Category* | Citation of document, with indication, where appropriate, of the relevant passages | Relevant to claim No. |
|---|---|---|
| A | CN 116655715 A (BEIJING XUANJINGRUI PHARMACEUTICAL TECHNOLOGY CO., LTD. et al.) 29 August 2023 (2023-08-29)<br>claim 15 | 1-23 |
| A | US 2021155926 A1 (SILENCE THERAPEUTICS GMBH) 27 May 2021 (2021-05-27)<br>description, paragraphs [0137]-[0162] | 1-23 |
| A | Tsuyoshi Yamamoto et al. "Serial Incorporation of a Monovalent GalNAc Phosphoramidite Unit into Hepatocyte-Targeting Antisense Oligonucleotides"<br>*Bioorganic & Medicinal Chemistry,* 27 November 2015 (2015-11-27), pp. 26-32<br>page 27, figure 1 | 1-23 |

Form PCT/ISA/210 (second sheet) (July 2022)

**INTERNATIONAL SEARCH REPORT**

International application No.
**PCT/CN2024/118774**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/118774**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **21-23**
because they relate to subject matter not required to be searched by this Authority, namely:

The subject matter of claims 21-23 relates to a method for treating diseases or conditions. However, the above-described method has a final purpose of treatment of a disease in a living animal body, and thus falls within the category of methods for diagnosis and treatment of diseases (PCT Rule 39.1(iv)).

With regard to claims 21-23, the reasonably expected amendments are as follows:

Claim 21: the use of the nucleic acid chimera according to any one of claims 1-17 in the preparation of a drug for treating diseases or conditions caused by dysregulated expression of at least one gene, the use comprising administering to an individual in need of treatment the nucleic acid chimera according to any one of claims 1-17.

Claim 22: the use according to claim 21, wherein the nucleic acid chimera is administered to the individual subcutaneously or intravenously.

Claim 23: the use according to claim 21 or 22, wherein after in-vivo administration, the nucleic acid chimera is decomposed to obtain at least two independent double-stranded oligonucleotide molecules, which respectively target portions of mRNA transcribed from one or more target genes, and the target genes may be the same or different.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/118774**

| Patent document cited in search report | Publication date (day/month/year) | Patent family member(s) | Publication date (day/month/year) |
|---|---|---|---|
| WO 2023283403 A2 | 12 January 2023 | EP 4367237 A2 | 15 May 2024 |
| | | JP 2024527584 A | 25 July 2024 |
| | | WO 2023283403 A3 | 13 April 2023 |
| CN 116655715 A | 29 August 2023 | None | |
| US 2021155926 A1 | 27 May 2021 | WO 2019193189 A1 | 10 October 2019 |
| | | EP 3775209 A1 | 17 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

• CN 202311265471

### Non-patent literature cited in the description

• *CHEMICAL ABSTRACTS*, 79598-53-1
• *CHEMICAL ABSTRACTS*, 25952-53-8
• *CHEMICAL ABSTRACTS*, 2592-95-2
• *CHEMICAL ABSTRACTS*, 30964-00-2